# EUROPÄISCHE PATENTSCHRIFT

(11) **EP 1 563 095 B1**
(45) Veröffentlichungstag und Bekanntmachung des Hinweises auf die Patenterteilung: **22.12.2010**
(21) Anmeldenummer: 03779977.2
(22) Anmeldetag: 18.11.2003
(51) Int. Cl.: C12Q 1/68

(54) **MICROARRAY-BASIERTES VERFAHREN ZUR AMPLIFIKATION UND DETEKTION VON NUKLEINSÄUREN IN EINEM KONTINUIERLICHEN PROZESS**
MICROARRAY-BASED METHOD FOR AMPLIFYING AND DETECTING NUCLEIC ACIDS DURING A CONTINUOUS PROCESS
PROCEDE BASE SUR UN MICRORESEAU POUR L'AMPLIFICATION ET LA MISE EN EVIDENCE D'ACIDES NUCLEIQUES LORS D'UN PROCEDE EN CONTINU

(30) Priorität: 19.11.2002 DE 10253966
(43) Veröffentlichungstag der Anmeldung: 17.08.2005
(73) Patentinhaber: Clondiag GmbH, 07749 Jena (DE)
(72) Erfinder: ERMANTRAUT, Eugen, 07745 Jena (DE); BICKEL, Ralf, 07546 Gera (DE); ELLINGER, Thomas, 07743 Jena (DE); WAGENHAUS, Annette, 07749 Jena (DE)
(74) Vertreter: Neuefeind, Regina
(86) Internationale Anmeldenummer: PCT/EP2003/012905
(87) Internationale Veröffentlichungsnummer: WO 2004/046378

(56) Entgegenhaltungen:
- WO-A-00/60919
- DE-A- 10 149 684
- US-A- 5 627 054
- US-A- 5 891 625
- O'MEARA DEIRDRE ET AL: "Capture of single-stranded DNA assisted by oligonucleotide modules" ANALYTICAL BIOCHEMISTRY, Bd. 255, Nr. 2, 15. Januar 1998 (1998-01-15), Seiten 195-203, XP002288286 ISSN: 0003-2697
- MALDONADO-RODRIGUEZ R ET AL: "HYBRIDIZATION OF GLASS-TETHERED OLIGONUCLEOTIDE PROBES TO TARGET STRANDS PREANNEALTED WITH LABELED AUXILIARY OLIGONUCLEOTIDES" MOLECULAR BIOTECHNOLOGY, TOTOWA, NJ, US, Bd. 11, 1999, Seiten 1-12, XP002928550 ISSN: 1073-6085

## Beschreibung

Gegenstand der vorliegenden Erfindung sind Verfahren, die im Rahmen einer Microarray-basierten Auswertung eine effiziente Amplifikation von Nukleinsäuren durch PCR und deren Nachweis durch Hybridisierung in einem kontinuierlichen Prozess ermöglichen. Gegenstand der Erfindung sind dabei insbesondere Verfahren, bei denen eine PCR durchgeführt wird, der zu Anfang ein Kompetitor zugesetzt wird. Ein weiterer Gegenstand der Erfindung sind Verfahren, bei denen die Hybridisierung in Gegenwart eines Moleküls durchgeführt wird, das in der Nähe der hybridisierenden Sequenzen an eines der hybridisierenden Nukleinsäuremoleküle bindet.

Biomedizinische Tests basieren häufig auf dem Nachweis Wechselwirkung zwischen einem Molekül, das in bekannter Menge und Position vorhanden ist (der molekularen Sonde) und einem nachzuweisenden, unbekanntem Molekül bzw. nachzuweisenden, unbekannten Molekülen (den molekularen Ziel- oder Targetmolekülen). Bei modernen Tests sind die Sonden in Form einer Substanzbibliothek auf Trägern, den sogenannten Microarrays oder Chips abgelegt, so dass eine Probe parallel an mehreren Sonden gleichzeitig analysiert werden kann (Lockhart et al. (2000) Nature, 405, 827-836). Für die Herstellung der Microarrays werden die Sonden dabei üblicherweise in vorgegebener Art und Weise auf einer geeigneten, beispielsweise in WO 00/12575 beschriebenen Matrix immobilisiert (siehe z.B. US 5 412 087, WO 98/36827) bzw. synthetisch erzeugt (siehe z.B. US 5 143 854).

Der Nachweis einer Wechselwirkung zwischen der Sonde und dem Targetmolekül erfolgt dabei folgendermaßen:

Die Sonde bzw. die Sonden werden in vorgegebener Art und Weise an einer bestimmten Matrix in Form eines Microarrays fixiert. Die Targets werden dann in einer Lösung mit den Sonden in Kontakt gebracht und unter definierten Bedingungen inkubiert. Infolge der Inkubation findet zwischen der Sonde und dem Target eine spezifische Wechselwirkung statt. Die dabei auftretende Bindung ist deutlich stabiler als die Bindung von Targetmolekülen an Sonden, die für das Targetmolekül nicht spezifisch sind. Zum Entfernen von Targetmolekülen, die nicht spezifisch gebunden worden sind, wird das System mit entsprechenden Lösungen gewaschen oder erwärmt.

Der Nachweis der spezifischen Wechselwirkung zwischen einem Target und seiner Sonde kann dann durch eine Vielzahl von Verfahren erfolgen, die in der Regel von der Art des Markers abhängt, der vor, während oder nach der Wechselwirkung der Targetmoleküle mit den Sonden in die Targetmoleküle eingebracht worden ist Typischerweise handelt es sich bei solchen Markern um fluoreszierende Gruppen, so dass spezifische Target-Sonden-Wechselwirkungen mit hoher Orts-Auflösung und im Vergleich zu anderen herkömmlichen Nachweismethoden (v.a. massensensitive Methoden) mit geringem Aufwand fluoreszenz-optisch ausgelesen werden können (Marshall et al. (1998) Nature Biotechnology, 16, 27-31;Ramsay (1998) Nature Biotechnology, 16 40-44).

Abhängig von der auf dem Microarray immobilisierten Substanzbibliothek und der chemischen Natur der Targetmoleküle können anhand dieses Testprinzips Wechselwirkungen zwischen Nukleinsäuren und Nukleinsäuren, zwischen Proteinen und Proteinen sowie zwischen Nukleinsäuren und Proteinen untersucht werden (zur Übersicht siehe Lottspeich et al. (1998) Bioanalytik, Spektrum Akademischer Verlag, Heidelberg Berlin Oxfold).

Als Substanzbibliotheken, die auf Microarrays oder Chips immobilisiert werden können, kommen dabei Antikörper-Bibliotheken, Rezeptor-Bibliotheken, Peptid-Bibliotheken und Nukleinsäure-Bibliotheken in Frage. Die Nukleinsäure-Bibliotheken nehmen die mit Abstand wichtigste Rolle ein.

Es handelt sich dabei um Microanrays, auf denen Desoxyribonukleinsäure- (DNA) Moleküle, Ribonukleinsäure- (RNA) Moleküle oder Moleküle von Nukleinsäureanaloga (z.B. PNA) immobilisiert sind. Voraussetzung für die Bindung eines mit einer Fluoreszenzgruppe markierten Targetmoleküls (DNA-Molekül oder RNA-Molekül) an eine Nukleinsäuresonde des Microarrays ist, dass sowohl Targetmolekül als auch Sondenmolekül in Form einer einzelsträngigen Nukleinsäure vorliegen.

Nur zwischen solchen Molekülen kann eine effiziente und spezifische Hybridisierung stattfinden. Einzelsträngige Nukleinsäureziel- und Nukleinsäuresondenmoleküle erhält man in der Regel durch Hitzedenaturierung und optimal zu wählende Parameter (Temperatur, Ionenstärke, Konzentration helixdestabilisierender Moleküle), was gewährleistet, dass nur Sonden mit nahezu perfekt komplementären (einander entsprechenden) Sequenzen mit der Zielsequenz gepaart bleiben (Leitch et al. (1994) In vitro Hybridisierung, Spektrum Akademischer Verlag, Heidelberg Berlin Oxford).

Ein typisches Beispiel für die Verwendung von Microarrays in biologischen Testverfahren ist der Nachweis von Mikroorganismen in Proben in der biomedizinischen Diagnostik. Dabei macht man sich die Tatsache zunutze, dass die Gene für ribosomale RNA (rRNA) ubiquitär verbreitet sind und über Sequenzabschnitte verfügen, die für die jeweilige Spezies charakteristisch sind. Diese Spezies-charakteristischen Sequenzen werden in Form von einzelsträngigen DNA-Oligonukleotidsonden auf ein Microarray aufgebracht. Die zu untersuchenden Target-DNA-Moleküle werden zunächst aus der zu untersuchenden Probe isoliert und mit fluoreszierenden Markern versehen. Anschließend werden die markierten Target-DNA-Moleküle in einer Lösung mit den auf dem Microarray aufgebrachten Sonden inkubiert, unspezifisch auftretende Wechselwirkungen werden durch entsprechende Waschschritte entfernt und spezifische Wechselwirkungen durch fluoreszenzoptische Auswertung nachgewiesen. Auf diese Art und Weise ist es möglich mit einem einzigen Test in einer Probe gleichzeitig z. B. mehrere Mikroorganismen nachzuweisen. Die Anzahl der nachweisbaren Mikroorganismen hängt bei diesem Testverfahren theoretisch nur von der Anzahl der spezifischen Sonden ab, die auf dem Microarray aufgebracht worden sind.

Ein weiteres Beispiel für eine Anwendung in einem medizinischen Testverfahren ist die Erstellung eines Single Nucleotide Polymorphismus ( kurz "SNP") - Profils als Ansatzpunkt für eine individualisierte Therapie.

Die Gesamtheit der genetischen Information eines Lebewesens ist bei allen Lebewesen, mit Ausnahme von eineiigen Mehrlingen und Klonen, individuell. Der Grad der Unterschiedlichkeit wird dabei um so größer, je kleiner der biologische Verwandtschaftsgrad zwischen den Individuen ist. Die so genannten Single Nucleotide Polymorphismen (SNP's) stellen dabei die häufigste Variation im menschlichen Genom dar.

Anhand des jeweiligen SNP-Profils eines Menschen soll es nun möglich werden zu beurteilen, wer bei einer medikamentösen Therapie auf die jeweiligen Medikamente anspricht oder bei wem mit unerwünschten Nebenwirkungen zu rechnen ist. Zahlen aus der Onkologie zeigen, dass oft nur 20 - 30% der Patienten auf spezielle Wirkstoffe ansprechen. Die restlichen 70% können diese häufig aus offenbar genetischen Gründen nicht verwerten. Auf DNA-Arrays basierende Testsysteme stellen hierbei als Entscheidungshilfe eine hervorragende Methode dar, den Patienten schnell und zuverlässig und durch wenige Handgriffe auf wenige, ausschließlich für eine Krankheit relevante SNP's zu testen. Dadurch können Therapien für Patienten individuell variiert werden.

Bei vielen Tests in der biomedizinischen Diagnostik tritt das Problem auf, dass vor dem eigentlichen Testverfahren die Targetmoleküle zunächst in ausreichender Form vorhanden sein müssen und damit häufig aus der Probe zunächst vervielfältigt werden müssen. Die Vervielfältigung von DNA-Molekülen geschieht durch die Polymerase-Kettenreaktion (PCR). Für die Vervielfältigung von RNA müssen die RNA-Moleküle durch reverse Transkription in entsprechend komplementäre DNA (cDNA) umgewandelt werden. Diese cDNA kann dann ebenfalls durch PCR vervielfältigt (amplifiziert) werden. Bei der PCR handelt es sich um eine Labor-Standard-Methode (Sambrook et al. (2001) Molecular Cloning: A laboratory manual, 3rd edition, Cold Spring Harbor, N.Y., Cold Spring Harbor Laboratory Press).

Die Vervielfältigung von DNA durch PCR ist verhältnismäßig schnell, ermöglicht durch miniaturisierte Verfahren einen hohen Probendurchsatz in geringen Ansatzvolumina und ist durch Automatisierung arbeitseffizient.

Eine Charakterisierung von Nukleinsäuren durch eine alleinige Vervielfältigung ist jedoch nicht möglich. Vielmehr ist es notwendig, nach der Amplifikation Analysemethoden wie Nukleinsäuresequenzbestimmungen, Hybridisierung und/oder elektrophoretische Trenn- und Isolationsverfahren zur Charakterisierung der PCR-Produkte einzusetzen.

Generell sollten Vorrichtungen und Verfahren zur Amplifikation von Nukleinsäuren und deren Nachweis so konzipiert sein, dass möglichst wenige Eingriffe seitens eines Experimentators notwendig sind. Die Vorteile von Verfahren, die eine Vervielfältigung von Nukleinsäuren und deren Nachweis ermöglichen und in deren Verlauf ein Experimentator nur minimal eingreifen muss, liegen auf der Hand. Zum einen werden Kontaminationen vermieden. Zum anderen ist die Reproduzierbarkeit solcher Verfahren, da sie einer Automatisierung zugänglich sind, wesentlich erhöht. Dies ist auch im Hinblick auf Zulassung von diagnostischen Verfahren extrem wichtig.

Es gibt gegenwärtig eine Vielzahl von Verfahren zur Amplifikation von Nukleinsäuren und deren Nachweis, bei denen zunächst das Target-Material durch PCR-Amplifikation vervielfältigt wird und die Identität bzw. der genetische Zustand der Zielsequenzen anschließend durch Hybridisierung gegen einen Sondenarray bestimmt wird. Die Amplifikation der nachzuweisenden Nukleinsäure- bzw. Target-Moleküle ist in der Regel notwendig, um ausreichende Mengen für einen qualitativen und quantitativen Nachweis im Rahmen der Hybridisierung zur Verfügung zu haben.

Sowohl die PCR-Amplifikation von Nukleinsäuren als auch der Nachweis der selben durch Hybridisierung sind einer Reihe von grundlegenden Problemen unterworfen. Dies gilt in gleicher Weise für Verfahren, die eine PCR-Amplifikation von Nukleinsäuren und deren Nachweis durch Hybridisierung kombinieren.

Eines der Probleme, das bei Verfahren, die PCR und Hybridisierung kombinieren, auftritt, ist in der Doppelsträngigkeit der Target-Moleküle begründet. Klassische PCR-Amplifikationsreaktionen erzeugen ausgehend von einem doppelsträngigen Template-Molekül üblicherweise doppelsträngige DNA-Moleküle. Diese können nur nach vorhergehender Denaturierung mit den Sonden des Sondenarrays hybridisieren. Während der Hybridisierungsreaktion konkurriert die sehr schnelle Doppelstrangbildung in der Lösung mit der Hybridisierung gegen die immobilisierten Sonden des Sondenarrays. Die Intensität der Hybridisierungssignale und damit die quantitative und qualitative Auswertung der Verfahrensergebnisse werden durch diese Kompetitionsreaktion stark begrenzt.

Hinzu kommen die Probleme, die in der Hybridisierungs-Reaktion an sich bzw. den zur Hybridisierung gebrachten Sonden und Target begründet sind. PCR-Produkte, die als Targets für Array-Hybridisierungs-Reaktionen Verwendung finden, weisen in der Regel eine Länge von mindestens circa 60 Basenpaaren auf. Dies entspricht der Summe der Längen der zur PCR-Reaktion verwendeten forward- und reverse-primer sowie der Region, die durch die PCR amplifiziert wird und Komplementarität zur Sonde auf dem Array aufweist. Einzelsträngige Moleküle dieser Länge liegen in Lösung häufig nicht unstrukturiert, d.h. linear gestreckt vor, sondern weisen mehr oder wenig stabile Sekundärstrukturen, wie z.B. Haarnadeln oder andere helikale Strukturen, auf. Wenn diese Sekundärstrukturen den Bereich des Targets betreffen, der Komplementarität zur Sonde aufweist, verhindert die Ausbildung der genannten Sekundärstrukturen eine effiziente Hybridisierung des Targets an die Sonde. Die Ausbildung von Sekundärstrukturen kann somit ebenfalls eine effiziente Hybridisierung inhibieren und eine quantitative und qualitative Auswertung der Verfahrensergebnisse erschweren, wenn nicht gar verhindern.

Im Stand der Technik wird versucht, den beschriebenen Problemen durch eine spezielle Prozessführung bzw. spezielle Protokollschritte zu begegnen. Dazu gehören u.a. die Wahl bestimmter PCR-Bedingungen, die Aufarbeitung und spezielle Behandlung der PCR-Amplifikationsprodukte sowie die Variation und Wechsel der Reaktionsparameter von PCR zur Hybridisierung.

Dem Fachmann stehen dabei mehrere bekannte Angriffspunkte zur Verfügung. Zum Beispiel kann die Effizienz der Hybridisierung von doppelsträngigen Target-Molekülen an Sondenarrays durch einen Pufferwechsel verbessert werden. Klassische Hybridisierungspuffer weisen eine hohe Ionenstärke auf und sind dahingehend optimiert, die Konkurrenz der beiden oben genannten Effekte, d.h. die Kompetition der Hybridisierung der einzelnen Stränge des Targets mit der Hybridisierung an die Sonde des Arrays und die Ausbildung von Sekundärstrukturen zu minimieren. Im Gegensatz dazu weisen PCR-Puffer in der Regel eine geringe Ionenkonzentration auf und sind für Array-Hybridisierungen an Sondenarrays entsprechend nicht optimiert. Deshalb werden Hybridisierungen von doppelsträngigen PCR-Fragmenten an Sondenarrays nicht direkt im PCR-Puffer, sondern erst nach Austausch gegen einen Hybridisierungspuffer durchgeführt. Der Pufferwechsel erfolgt dabei häufig nach der Alkoholfällung des PCR-Produkts bzw. nach Reinigung über aus dem Stand der Technik bekannte Affinitätssäulen (z.B. Qiaquick PCR-Reinigungskit der Firma Qiagen, Hilden, Deutschland). Ein typisches Protokoll für ein Verfahren, bei dem PCR-Produkte durch Hybridisierung an Sondenarrays nachgewiesen werden, kann dem GeneChip P450 Kit von Affymetrix, Inc. (Santa Clara, CA, USA) entnommen werden. Der Nachteil des genannten Verfahrens liegt darin, dass der Pufferwechsel einen zusätzlichen Arbeitsschritt bedeutet und damit, wie oben ausgeführt, die Gefahr einer zusätzlichen Kontamination im Laufe des Arbeitsprozesses gegeben ist.

Eine andere Möglichkeit, einer ineffizienten Hybridisierung aufgrund der Kompetition bei der Hybridisierung oder einer Sekundärstrukturausbildung im Target zu begegnen, liegt in der effizienten Markierung des Targets. Der negative Einfluss der Doppelstrangbildung des Targets in der Lösung und die Ausbildung von Sekundärstrukturen auf die Intensität des Hybridisierungssignals kann teilweise durch eine effiziente Markierung des PCR-Produkts, z.B. durch Einsatz markierter Desoxynukleotidtriphosphate, kompensiert werden.

Neben den im Vergleich mit einer Endmarkierung durch Einsatz markierter Primer höheren Kosten, hat diese Verfahrensweise den zusätzlichen Nachteil, dass eine Abtrennung der markierten Bausteine vom PCR-Produkt vor der Hybridisierung notwendig ist, um das unspezifische Hintergrundsignal bei der anschließenden Hybridisierung in vertretbaren Grenzen zu halten. Dieser zusätzliche Schritt verkompliziert das Nachweisverfahren und stellt eine zusätzliche Kontaminationsquelle dar. Außerdem verhindert dieser zusätzliche Arbeitsschritt die Entwicklung eines kontinuierlichen Analyseprozesses, der PCR-Amplifikation und Array-Hybridisierung kombiniert und in den der Experimentator nicht eingreifen muss.

Ein weiterer Ansatz, die genannten Probleme, die zu einer ineffizienten Hybridisierung aufgrund der Ausbildung von Sekundärstrukturen fuhren, zu beheben, besteht in der Fragmentierung des PCR-Amplifikationsprodukts. Durch enzymatische oder chemische Verfahren kann das PCR-Produkt in kurze DNA-Abschnitte, die üblicherweise 20-40 Basen umfassen, fragmentiert werden, so dass die Ausbildung stabiler Sekundärstrukturen verhindert wird. Da die Oberflächen von Sondenarrays gewöhnlich eine bessere Zugänglichkeit für diese kurzen Fragmente aufweisen, wird zudem durch die Fragmentierung des PCR-Produkts die Kompetition der Doppelstrangbildung des Targets in der Lösung mit der Hybridisierung gegen die immobilisierten Sonden auf dem Sondenarray minimiert.

Allerdings hat diese Verfahrensweise mehrere Nachteile. Zum einen stellt die Fragmentierung der DNA einen zusätzlichen Protokollschritt dar, der mindestens eines zusätzlichen Pufferwechsels bedarf. Die damit verbundenen Nachteile sind bereits oben dargelegt worden. Darüber hinaus erfolgt die Fragmentierung der DNA in der Regel nicht Sequenz-spezifisch, so dass ein Teil der DNA-Moleküle in dem Bereich fragmentiert wird, der Komplementarität zur Sonde aufweist. Derart fragmentierte Targets binden naturgemäß schlechter oder gar nicht an die immobilisierten Sonden. Um zu vermeiden, dass die Sonden fragmentiert werden, müssen die zur Fragmentierung verwendeten Enzyme oder chemischen Substanzen vorher inaktiviert werden. Dies kann ebenfalls zusätzliche Arbeitsschritte bedeuten. Außerdem schließt die Fragmentierung eine kostengünstige und einfach durchzuführende Endmarkierung der PCR-Produkte aus, da nach der Fragmentierung interne Fragmente nur bei interner Markierung nachweisbar sind.

Aufgrund der oben dargestellten Nachteile, wie sie bei der Hybridisierung von Targets, die durch klassische PCR-Protokolle amplifiziert worden sind, an immobilisierte Sonden eines Sondenarrays auftreten, sind im Stand der Technik Versuche unternommen worden, die PCR-Bedingungen dahingehend zu modifizieren, dass im Rahmen der PCR nur einer der beiden Stränge des zur PCR verwandten doppelsträngigen DNA-Templates amplifiziert wird.

Durch die Entwicklung von PCR-Protokollen, die die Produktion eines Einzelstrangüberschusses ermöglichen, sollte prinzipiell die Konkurrenz der Doppelstrangbildung der Targetmoleküle gegenüber der Hybridisierung mit den Sondenmolekülen bei der Hybridisierung vermieden werden.

Im Falle der PCR kann einzelsträngige DNA entweder nach der Reaktion aus dem doppelsträngigen PCR-Produkt erzeugt werden oder es werden PCR-Bedingungen gewählt, bei denen einer der Stränge im Verlauf der PCR im Überschuss gebildet wird.

Für die Erzeugung einzelsträngige DNA-Target-Moleküle aus doppelsträngigen PCR-Produkten kann einer der beiden Stränge selektiv enzymatisch abgebaut werden, während der andere gegen den nukleolytischen Abbau resistent ist und dadurch erhalten bleibt.

Wenn während der PCR z.B. einer der verwendeten Primer am 5'-Ende mit mehreren Phosphothioat-Bausteine modifiziert ist, kann das erhaltene PCR-Produkt mit T7 gene 6 Exonuklease behandelt werden. Dabei entsteht einzelsträngige DNA, da nur der nicht-modifizierte Strang exonukleolytisch abgebaut werden kann, während der Phosphothiat-modifizierte Strang gegen den exonukleolytischen Abbau resistent ist (Nikiforov et al. (1994) PCR Methods Appl., Apr 3 (5), 285-291).

Bei einem alternativen Verfahren wird einer der beiden zur PCR verwendeten Primer am 5'-Ende mit einer Phosphatgruppe modifiziert. Bei der anschließenden Behandlung des PCR-Fragments mit Lambda Exonuklease wird nur der phosphorylierte Strang exonukleolytisch abgebaut. Der andere Strang bleibt als Einzelstrang erhalten (Michel et al. (1997) Histochem. J., 29(9), 685-683; Kujau et al. (1997) Mol. Biotechnol. 3, 333-335; Null et al. (2000) Analyst, 125 (4), 619-626).

Alternativ können doppelsträngige PCR-Produkte durch Abtrennen eines Stranges in einzelsträngige DNA überführt werden und als Target-Moleküle für die Hybridisierung verwendet werden. Zum Beispiel kann einer der zur PCR verwendeten Primer mit Biotin markiert werden. Wenn der amplifizierte DNA-Doppelstrang anschließend an einen Streptavidin-beschichteten festen Träger gebunden wird, kann nach Denaturierung der Streptavidin-beschichtete Träger von der Lösung z.B. durch Zentrifugation abgetrennt werden, so dass einer der Stränge in Lösung zurückbleibt (Bowman et al. (1993) Methods Enzymol., 224, 339-406).

Die genannten Verfahren haben allesamt den Nachteil, dass die Abtrennung eines der beiden im Rahmen der PCR amplifizierten DNA-Stränge einen zusätzlichen Arbeitsschritt darstellt und entsprechend die oben aufgeführten Nachteile mit sich bringt. Darüber hinaus sind die zur Herstellung der Primer verwendeten Phosphothioate bzw. zur Markierung der Primer verwendeten Biotin-Marker mit erhöhten Kosten verbunden.

Verfahren, bei denen bereits während der PCR-Reaktion ein Einzelstrangüberschuss entsteht, sind allgemein als lineare oder asymmetrische PCR bekannt.

Bei der klassischen linearen PCR-Reaktion wird ein doppelsträngiges Template in einer PCR-Reaktion amplifiziert, der nur ein einzelner Primer zugegeben wurde. Entsprechend wird ausgehend von diesem Primer nur einer der beiden Stränge produziert, der dann im Überschuss vorliegt (Kaltenboeck et al. (1992) Biotechniques, 12(2), 164-166). Die lineare PCR hat allerdings den Nachteil, dass die durch sie amplifizierten Target-Mengen in der Regel nicht für eine effizienten quantitativen und qualitativen Nachweis im Rahmen einer Hybridisierung eines Sondenarrays ausreichen.

Von symmetrischer PCR wird üblicherweise gesprochen, wenn beide Primer in gleichen molaren Mengen vorliegen. Die Amplifikationsrate folgt dann einer exponentiellen Kinetik der Form 2ⁿ (n = Anzahl der PCR-Zyklen). Die Kombination aus symmetrischer PCR-Reaktion mit bezüglich des Templates limitierender Primermenge und anschließender Zugabe eines einzelnen Primers im Überschuss gefolgt von einer linearen Amplifikation wird üblicherweise als zweistufige lineare oder asymmetrische PCR beschrieben. Der Nachteil dieses Verfahrens ist darin zu sehen, dass erst nach der Durchführung der symmetrischen PCR der Primer zur Durchführung der linearen PCR zugegeben wird, was einen zusätzlichen Eingriff ins experimentelle System darstellt.

Ein anderes Verfahren der zweistufigen, asymmetrischen PCR ist in US 5,891,625 beschrieben. Nach einer symmetrischen PCR-Amplifikation werden Peptid-Nukleinsäuren (Peptide Nucleic Acids, PNA) der Reaktion zugegeben, die Komplementarität zu einem der beiden Primer aufweisen. Durch Hybridisierung des entsprechenden Primers mit dem Antisense-PNA steht für die weitere PCR-Amplifikation nur noch der andere Primer zur Verfügung, so dass nur noch ein Strang linear amplifiziert wird. Nachteilig an diesem Verfahren ist wiederum, dass die Antisense-PNA erst nach Durchführung einer symmetrischen PCR der Reaktion beigesetzt wird, was einen zusätzlichen Manipulationsschritt darstellt.

US 5,627,054 beschreibt ein weiteres Verfahren der zweistufigen asymmetrischen PCR, bei dem nach Durchführung einer symmetrischen PCR der Reaktion ein Kompetitor zugesetzt wird, der die Bindungsstellen eines der Primer auf dem Template blockiert. Bei dem Kompetitor handelt es sich um eine Nukleinsäure, die aufgrund ihrer Sequenz oder einer chemischen Modifikation am 3'-Ende nicht von der zur PCR verwendeten Polymerase verlängert werden kann. Üblicherweise wird der Kompetitor im molaren Überschuss zum Primer zugesetzt. Auch hier wird der Kompetitor erst nach Durchführen einer symmetrischen PCR der Reaktion zugeführt. Dieser zusätzliche Arbeitsschritt wird in Kauf genommen, da beim Auslassen der symmetrischen PCR die Menge an produziertem Einzelstrang nicht ausreichend ist, um diese effizient in einer quantitativen und qualitativen Hybridisierung auf einem Sondenarray nachzuweisen.

Wie bereits dargestellt, haben die beschriebenen Verfahren den gemeinsamen Nachteil, dass für die Herstellung einzelsträngiger DNA mindestens ein zusätzlicher Reaktionsschritt notwendig ist, der das Eingreifen des Experimentators erfordert. Damit sind diese Verfahren für die Anwendung in geschlossenen Systemen bzw. auf mäandrierenden Kanälen basierenden Flussthermocyclern (Köhler et al. (1998) Micro Channel Reactors for Fast Thermocycling, Proc. 2nd International Conference on Microreaction Technology, New Orleans, 241-247), bei denen eine Zugabe oder ein Abtrennen von Komponenten während der Reaktion nicht möglich ist, nicht geeignet.

Alternative Verfahren gestatten die asymmetrische PCR-Amplifikation in einem Schritt. Zum Beispiel kann durch Zugabe der Primer im asymmetrischen Verhältnis (d.h. einer der Primer ist im molaren Unterschuss vorhanden und wird im Verlauf der Reaktion aufgebraucht) ein Strang im Überschuss produziert werden. Der Nachteil dieses Verfahrens besteht aber darin, dass erst nach Erreichen einer bestimmten Konzentration des PCR-Produkts ein Einzelstrangüberschuss erreicht wird. Das Erreichen dieser Konzentration hängt jedoch wiederum in starkem Maße von der Anfangskonzentration des Templates ab, so dass die kritische Konzentration bei Proben mit geringer Template-Menge u.U. gar nicht erreicht wird. Das Amplifikationsprodukt würde dann nur in doppelsträngiger Form vorliegen und das Signal bei einer Hybridisierungs-basierten Analyse wegen der gering konzentrierten Proben überproportional geschwächt.

Die Ausbildung von Sekundärstrukturen im Target hat, wie bereits oben dargestellt wurde, einen wesentlichen Einfluss auf die Stärke des Hybridisierungssignals (Southern et al. (1994) Nucleic Acids Res., 22, 1368-1373; Sohail et al. (1999) RNA, 5, 646-655).

Entsprechend sind unterschiedliche Versuche unternommen worden, durch Verringerung der Sekundärstruktur der Target-Moleküle, die Signal-Intensitäten in Hybridisierungs-Nachweisverfahren zu erhöhen.

Zum Beispiel wurde während der chemischen Synthese eines DNA-Moleküls N4-Ethyl-substituierte Cytosin-Reste verwendet, die die Ausbildung von Sekundärstrukturen stark reduzieren (Nguyen et al. (2000) Nucleic Acids Res., 28, 3904-3909). Allerdings kann diese Methode dann nicht eingesetzt werden, wenn die nachzuweisenden Target-Moleküle nicht chemisch, sondern enzymatisch synthetisiert werden.

Eine weitere Möglichkeit der Schwächung von Signalintensitäten in Hybridisierungs-Nachweisverfahren durch Ausbildung von Sekundärstrukturen zu begegnen, besteht in der Verwendung von den bereits erwähnten PNAs als Sonden. Aufgrund der physiko-chemischen Eigenschaften der PNAs kann die Hybridisierung dann bei vergleichsweise geringen Ionenstärken durchgeführt werden, so dass die Neigung von Target-Molekülen zur Ausbildung von Sekundärstrukturen minimiert wird. Allerdings werden PNA-Arrays momentan nicht kommerziell angeboten, so dass die genannte Methode zwar denkbar, für Standardanwendungen aber nicht praktikabel ist.

Die Hybridisierung von Sonden mit Target-Molekülen kann auch stabilisiert werden und die Signalintensitäten entsprechend erhöht werden, wenn vor der Hybridisierung auf dem Array die Target-Moleküle mit Oligonukleotidsonden hybridisiert werden, die in direkter Nachbarschaft zu der Sequenz liegen, die mit der Sonde des Sondenarrays hybridisiert. Bei der anschließenden Hybridisierung des "vorhybridisierten" Target-Moleküls mit der Sonde des Sondenarrays wird in diesem Fall eine kontinuierliche Helix über den Bereich der Sonde und des zuvor hybridisierten Oligonukleotids aufgebaut, so dass das Gesamthybrid über sogenannte "stacking interactions" zusätzlich stabilisiert wird (O'Meara et al. (1998) Anal. Biochem., 225(2), 195-203; Maldonado-Rodriguez et al. (1999) Mol. Biotechnol., 11(1), 1-12). Möglicherweise spielt bei der Hybridisierungs-Stabilisierung und entsprechenden Signalintensitätserhöhung auch die Destabilisierung von Sekundärstrukturen im Target-Molekül eine Rolle.

Nachteilig jedoch ist, dass die Intensität des Hybridisierungssignals drastisch sinkt, wenn die durch "stacking interactions" bewirkte Stabilisierung durch Einfügen einer Lücke von einer oder mehrerer Basen zwischen den Bindungsstellen des vorhybridisierten Oligonukleotids und der Sonde des Sondenarrays entfällt. Insofern muss ein großer Aufwand betrieben werden, um die Oligonukleotide, die vorhybridisiert werden, exakt auszuwählen. Ein weiterer Nachteil ist, dass die Zugabe der Oligonukleotide schon zu Beginn der PCR-Amplifikation nicht möglich ist, da diese sonst als PCR-Primer verwendet würden und zu zusätzlichen PCR-Fragmenten führen würden. Darüber hinaus verlieren die Oligonukleotide durch die enzymatische Verlängerung in die Targetregion hinein ihre Funktion als Sekundärstruktur-brecher. Da eine anfängliche Zugabe der Oligonukleotide zur PCR nicht möglich ist, kann kein kontinuierlicher Prozess aus PCR und Hybridisierung aufgebaut werden. Vielmehr sind zusätzliche Prozessschritte notwendig, was die bereits mehrfach dargestellten Nachteile mit sich bringt.

Aus den oben dargestellten Verfahren geht hervor, dass gegenwärtig kein Verfahren existiert, welches die Array-basierte Durchführung von Analysen, die auf einer Amplifikation des Targetmaterials durch PCR und anschließender Analyse des Amplifikationsprodukts durch Hybridisierung gegen Sondenarrays beruhen, bei guter Sensitivität und Spezifität in einem kontinuierlichen Prozess ermöglicht.

Ein solches Verfahren sollte es gestatten, zu Beginn der Analyse den für den gesamten Verlauf notwendigen Reaktionansatz einschließlich Array zu kombinieren, PCR und Hybridisierung als kontinuierlichen Prozess durchzuführen und anschließend das Hybridisierungsmuster auf dem Sondenarray auszulesen. Ein solches einfaches, sensitives und spezifisches Verfahren wäre nicht nur wegen der Einsparung an Kosten und Zeit äußerst wünschenswert. Es ist darüber hinaus eine Voraussetzung dafür, dass sensitive und spezifische kombinierte Amplifikations- und Hybridisierungsreaktionen im Arrayformat in geschlossenen Reaktionskammern ausgeführt werden können. Damit würde ein solches Verfahren einen wichtigen Beitrag auf dem Weg zur Etablierung von "point of care"-Diagnostikverfahren darstellen.

Ebenso existiert gegenwärtig kein Array-basiertes Verfahren, das eine parallele Echtzeit-Quantifizierung von Targets durch PCR-Amplifikation und Hybridisierung gestattet. Ein solches Verfahren könnte beispielsweise durch sich abwechselnde PCR-Amplifkations- und Hybridisierungsschritte realisiert werden, wobei nach jedem Amplifkationsschritt eine Hybridisierung gegen ein Array erfolgt und die PCR-Reaktion anhand der Hybridisierungs-signale quantifiziert wird. Ein solches Verfahren würde gegenüber dem Stand der Technik erfordern, dass einerseits PCR und Hybridisierung in einem kontinuierlichen Prozess durchgeführt werden und dass andererseits eine sehr hohe Sensitivität bei der Hybridisierungs-basierten Detektion über den gesamten Verlauf der PCR-Amplifkation erreicht werden kann.

WO 00/60919 A offenbart ein Verfahren zur "Strand-Displacement-Amplification" (SDA) von Ziel-Nukleinsäuren unter Verwendung von an elektronisch adressierbaren Bindungsstellen eines Mikroarrays gekoppelten Primerpaaren. US 5,891,625 A offenbart eine asymmetrische Polymerase-Kettenreaktion unter Verwendung von Nukleinsäureanaloga, die an eine Primer-Bindungsstelle einer spezifischen Primersequenz hybridisieren können, wobei das gebildete Hybrid unter PCR-Reaktionsbedingungen stabil ist und somit eine Hybridisierung dieses Primer an die Template-Nukleinsäure unterbindet. D. O'Meara et al. (Analytical Biochemistry, 255 (2), 1998, 195-203) offenbaren Verfahren zur Hybridisierung einzelsträngiger DNA an Sondenmoleküle, worin die Interaktion zwischen einem einzelsträngigen PCR-Produkt und den auf einem Sensorchip immobilisierten Oligonukleotiden mittels Streptavidin-Biotin-Chemie analysiert wird. Hierzu wird zunächst die Ziel-DNA mittels einer herkömmlichen symmetrischen PCR amplifiziert und anschließend der Hybridisierungslösung Kompetitor-Oligonukleotide zugefügt, die zu einem Einzelstrang der Ziel-DNA komplementär sind. Ein ähnliches Verfahren beschreiben R. Maldonado-Rodriguez et al. (Molecular Biotechnology, 11, 1999, 1-12), wonach die zu analysierende Ziel-DNA mittels einer herkömmlichen symmetrischen PCR bereitgestellt wird und anschließend die einzelsträngigen Fragmente mittels Chromatographie aufgetrennt werden.

Es ist daher eine Aufgabe der vorliegenden Erfindung, Microarray-basierte Verfahren zur Verfügung zu stellen, die eine PCR-Amplifikation des Targetsmaterials und Nachweis desselben durch Hybridisierung gegen Sondenarrays bei guter Sensitivität und Spezifität in einem kontinuierlichen Prozess erlauben. Insbesondere ist es eine Aufgabe der vorliegenden Erfindung, Verfahren für

Microarray-basierte Auswertungen zur Verfügung zu stellen, bei denen die PCR-Amplifikation des Targetmaterials und der Nachweis des amplifizierten Materials durch Hybridisierung gegen einen Sondenarray ohne zwischengeschaltete experimentelle Schritte oder Aufarbeitungsreaktionen durchgeführt werden können. Insbesondere ist es eine Aufgabe der vorliegenden Erfindung, Verfahren für Microarray-basierte Auswertungen zur Verfügung zu stellen, bei denen das Targetmaterial in einem kontinuierlichen PCR-Amplifikationsprozess in einem für die Hybridisierung ausreichenden Einzelstrangüberschuss produziert wird.

Es ist eine weitere Aufgabe der Erfindung, ein Verfahren zur parallelen Hybridisierungs-basierten Echtzeit-Quantifizierung von PCR-Targets bereitzustellen, das auf der Erhöhung der Sensitivität der Hybridisierungsreaktion und der Durchführung der PCR-Amplifkations- und Hybridisierungsreaktion in einem kontinuierlichen, zyklischen Prozess beruht.

Es ist eine ebenfalls eine Aufgabe der Erfindung, ein Verfahren zur Verfügung zu stellen, in dem in einer asymmetrischen PCR eine Menge an Einzelstrangüberschuss amplifiziert wird, die für einen effizienten Nachweis in einem Hybridisierungsverfahren ausreichend ist.

Daneben ist es eine Aufgabe der vorliegenden Erfindung, Verfahren zur Microarray-basierten Auswertung zur Verfügung zu stellen, bei denen die Ausbildung von Sekundärstrukturen des Targetmaterials verhindert wird.

Diese und andere Aufgaben der vorliegenden Erfindung, wie sie sich aus der Beschreibung ergeben, werden durch den Gegenstand des unabhängigen Anspruchs gelöst. Bevorzugte Ausführungsformen der Erfindung werden durch die Unteransprüche definiert.

Gegenstand der vorliegenden Erfindung ist ein Verfahren zur effizienten Amplifikation von Nukleinsäuren und deren Nachweis in einem kontinuierlichen Prozess, das dadurch gekennzeichnet ist, dass die nachzuweisende Nukleinsäure zunächst durch eine PCR im Einzelstrangüberschuss amplifiziert wird, wobei der Reaktion zu Anfang mindestens ein Kompetitor ausgewählt aus Nukleinsäuren und/oder Nukleinsäureanaloga in mindestens äquimolaren Mengen zu einem in der PCR verwendete Primer zugesetzt wird, der die Bildung eines der beiden durch die PCR-amplifizierten Template-Stränge inhibiert, und die amplifizierte Nukleinsäure durch Hybridisierung mit einer komplementären Sonde nachgewiesen wird.

Im Rahmen der vorliegenden Erfindung ist überraschenderweise festgestellt worden, dass durch eine PCR-Reaktion, der zu Anfang ein Kompetitor ausgewählt aus Nukleinsäuren und/oder Nukleinsäureanaloga in mindestens äquimolaren Mengen zu einem in der PCR verwendete Primer zugesetzt wurde, der die Amplifizierung eines der beiden Template-Stränge inhibiert, ausreichend einzelsträngige Nukleinsäuremoleküle im Überschuss amplifiziert werden, um diese in einem zweiten Schritt durch Hybridisierung mit einer komplementären Sonde nachzuweisen.

Bei den erfindungsgemäß eingesetzten Kompetitormengen musste aufgrund theoretischer Überlegungen davon ausgegangen werden, dass die Amplifikation eines Template-Strangs im Wesentlichen unterbunden wird, so dass es zu einer nahezu linearen Amplifikation kommt. Auf diese Weise wäre zwar ein Einzelstrangüberschuss in der PCR zu erzielen, dieser aber aufgrund geringer Mengen für eine effiziente Hybridisierung nicht ausreichend.

Überraschenderweise wurde jedoch festgestellt, dass eine aufgrund des Kompetitors zwar gedämpfte exponentielle Amplifikation der Template-Stränge stattfindet, die aber eine ausreichend große Menge an Einzelsträngen erzielt, um eine effiziente Hybridisierung zu gewährleisten. Insbesondere wird der Verlust an Menge durch den Einzelstrangüberschuss bei Hybridisierungs-basierten Assays mehr als aufgehoben.

Bisher war davon ausgegangen worden, dass die alleinige Durchführung einer asymmetrischen PCR-Reaktion nicht genügend einzelsträngige Nukleinsäuremoleküle amplifiziert, um diese in anschließenden Nachweisreaktionen wie z.B. einer Hybridisierung mit einer entsprechenden Sonde zu detektieren. Daher war zur Amplifikation von einzelsträngigen Nukleinsäuremolekülen bisher im Stand der Technik immer eine zweistufige asymmetrische oder lineare PCR durchgeführt worden, d.h. der asymmetrischen oder linearen PCR-Amplifikation war eine klassische symmetrische PCR-Amplifikation vorgeschaltet, was jedoch ein mehrfaches experimentelles Eingreifen, wie z.B. die Zugabe neuer Primer, im Laufe der Prozessführung bedeutete.

Dem gegenüber zeigt die vorliegende Erfindung überraschenderweise, dass durch Zugabe von Kompetitoren ausgewählt aus Nukleinsäuren und/oder Nukleinsäureanaloga in mindestens äquimolaren Mengen zu einem in der PCR verwendete Primer zu dem PCR-Reaktionsansatz beim Start der Amplifikationsreaktion gleichzeitig eine effiziente Amplifikation der Zielsequenz und ein derart hoher Einzelstrangüberschuss erreicht werden kann, dass ein effizienter, quantitativer und qualitativer Nachweis der amplifizierten Einzelstränge durch Hybridisierung mit entsprechenden Sonden möglich ist.

Gegenstand der Erfindung ist daher ein Verfahren zur effizienten Amplifikation von Nukleinsäuren, bei dem die, gegebenenfalls anschließend nachzuweisende, Nukleinsäure durch eine PCR amplifiziert wird, wobei der Reaktion zu Anfang mindestens ein Kompetitor ausgewählt aus Nukleinsäuren und/oder Nukleinsäureanaloga in mindestens äquimolaren Mengen zu einem in der PCR verwendete Primer zugesetzt wird, der die Bildung eines der beiden durch die PCR amplifizierten bzw. amplifizierbaren Template-Stränge inhibiert.

In einer bevorzugten Ausführungsform der Erfindung werden die amplifizierten Nukleinsäuren durch anschließende Hybridisierung mit einer komplementären Sonde nachgewiesen.

Gegenstand der vorliegenden Erfindung ist ferner ein Verfahren zur effizienten Amplifikation von Nukleinsäuremolekülen und deren Nachweis durch Hybridisierung in einem kontinuierlichen Prozess, wobei
a) die nachzuweisende Nukleinsäure zurächst durch eine PCR-Reaktion amplifiziert wird und der Reaktion zu Anfang mindestens ein Kompetitor ausgewählt aus Nukleinsäuren und/oder Nukleinsäureanaloga in mindestens äquimolaren Mengen zu einem in der PCR verwendete Primer zugesetzt wird, der die Bildung eines der beiden durch die PCR-amplifizierten Template-Stränge inhibiert, und
b) das amplifizierte Nukleinsäuremoleküle durch Hybridisierung mit einer komplementären Sonde nachgewiesen wird.

Gegenstand der vorliegenden Erfindung ist insbesondere ein Verfahren zur effizienten Amplifikation von Nukleinsäuremolekülen und deren Nachweis durch Hybridisierung in einem kontinuierlichen Prozess, das dadurch gekennzeichnet ist, dass der PCR ein DNA-Molekül zugesetzt wird, das mit einem der zur PCR-Amplifikation des Templates verwendeten Primer um die Bindung an das Template konkurriert, und nicht enzymatisch verlängert werden kann. Die durch die PCR amplifizierten einzelsträngigen Nukleinsäuremoleküle werden dann durch Hybridisierung mit einer komplementären Sonde nachgewiesen.

Ein weiterer Gegenstand der Erfindung ist ein Verfahren zur Amplifikation von Nukleinsäuremolekülen und deren Nachweis durch Hybridisierung in einem kontinuierlichen Prozess, das dadurch gekennzeichnet ist, dass eine nachzuweisende Nukleinsäure zunächst im Einzelstrangüberschuss durch eine PCR amplifiziert wird und durch eine anschließende Hybridisierung mit einer komplementären Sonde nachgewiesen wird, wobei der PCR-Reaktion zu Anfang ein Kompetitor ausgewählt aus Nukleinsäuren und/oder Nukleinsäureanaloga in mindestens äquimolaren Mengen zu einem in der PCR verwendete Primer zugesetzt wird, bei dem es sich um ein DNA-Molekül oder ein Molekül eines NukleinsäureAnalogons handelt, das an einen der beiden Stränge des Templates hybridisieren kann, aber nicht an den Bereich, der durch die Sonden-Hybridisierung nachgewiesen wird, und das enzymatisch nicht verlängerbar ist.

Ein weiterer Gegenstand der Erfindung ist die Anwendung der erfindungsgemäßen Verfahren für Microarray-basierte Experimente und Diagnostikverfahren.

In einem Aspekt der vorliegenden Erfindung wird bei dem Verfahren zur effizienten Amplifikation mindestens einer Template-Nukleinsäure die Nukleinsäure durch eine PCR amplifiziert, wobei der Reaktion zu Anfang mindestens ein Kompetitor ausgewählt aus Nukleinsäuren und/oder Nukleinsäureanaloga in mindestens äquimolaren Mengen zu einem in der PCR verwendete Primer zugesetzt wird, der die Bildung eines der beiden durch die PCR amplifizierten Template-Stränge inhibiert.

Der Kompetitor wird ausgewählt aus Nukleinsäuren und/oder Nukleinsäureanaloga. Die Kompetitor-Nukleinsäure wird ausgewählt aus DNA- und/oder RNA-Molekülen. Das Kompetitor-Nukleinsäureanalogon wird vorzugsweise ausgewählt aus PNA, LNA, TNA und/oder einem Nukleinsäureanalogon mit einer Phosphothioat-Bindung.

Vorzugsweise ist der Kompetitor eine Nukleinsäure und/oder ein Nukleinsäureanalogon, wobei der Kompetitor enzymatisch nicht verlängerbar ist. Insbesondere ist das 3'-Ende der Nukleinsäure und/oder des Nukleinsäureanalogons enzymatisch nicht verlängerbar, besonders bevorzugt dadurch, dass es keine freie 3'-OH-Gruppe aufweist und beispielsweise anstelle der 3'-OH-Gruppe an seinem 3'-Ende eine Amino-Gruppe, eine Phosphat-Gruppe, einen Biotin-Rest, ein Fluorophor bzw. einen Fluoreszenzfarbstoff oder ein Wasserstoffatom aufweist. Geeignete Fluoreszenzfarbstoffe sind beispielsweise solche, die herkömmlicherweise zur Markierung von Primern oder Monomeren eingesetzt werden.

Ferner ist es bevorzugt, dass eine Kompetitor-Nukleinsäure bzw. ein Kompetitor-Nukleinsäure-Analogon eingesetzt wird, die bzw. das am 3'-Ende mindestens ein, zwei, drei, vier oder fünf Nukleotide bzw. Nukleotidanaloga aufweist, die nicht zu den entsprechenden Positionen in dem jeweiligen Template-Strang komplementär sind.

Vorzugsweise inhibiert der Kompetitor die Bildung eines der beiden durch die PCR amplifizierten Template-Stränge durch Bindung an einen in der PCR verwendeten Primer und/oder durch Bindung an einen der Template-Stränge.

In einer Ausführungsform ist es bevorzugt, dass der Kompetitor an einen in der PCR verwendeten Primer bindet. Dadurch wird die Hybridisierung des Primers mit einem Template-Strang inhibiert.

Vorzugsweise ist der Kompetitor eine Nukleinsäure und/oder ein Nukleinsäureanalogon mit einer zumindest teilweise zu einem der in der PCR verwendeten Primer komplementären Sequenz .

Ferner ist es bevorzugt, dass der Kompetitor mit dem Primer kovalent verknüpft wird. Die kovalente Verknüpfung zwischen Kompetitor und Primer dient vorzugsweise nicht als Template-Strang für Polymerasen.

Vorzugsweise ist die Stabilität des Komplexes von Primer mit Kompetitor geringer als die Stabilität des Komplexes des Primers mit seiner spezifischen Primer-Bindungsstelle auf dem Template-Strang. Besonders bevorzugt wird die Amplifikation bezüglich des Temperaturregimes als zweiphasiger Prozess durchgeführt, wobei in einer ersten Phase eine Annealing-Temperatur gewählt wird, bei der der Primer an seine spezifische Primer-Bindungsstelle auf dem Template-Strang, aber nicht an den Kompetitor, bindet, und in einer zweiten Phase eine Annealing-Temperatur gewählt wird, bei der auch eine Bindung zwischen Primer und Kompetitor erfolgt.

In einer weiteren Ausführungsform ist es bevorzugt, dass der Kompetitor einen der Template-Stränge bindet. Dadurch wird dessen Amplifikation inhibiert.

Insbesondere konkurriert der Kompetitor mit einem der zur PCR verwendeten Primer um die Bindung an einen der Template-Stränge. Dabei ist es ferner bevorzugt, dass die Stabilität des Komplexes des Kompetitors mit der spezifischen Primer-Bindungsstelle des Primers auf dem Template geringer ist als die Stabilität des Komplexes des Primers mit seiner spezifischen Primer-Bindungsstelle auf dem Template-Strang. Besonders bevorzugt wird die Amplifikation bezüglich des Temperaturregimes als zweiphasiger Prozess durchgeführt, wobei in einer ersten Phase eine Annealing-Temperatur gewählt wird, bei der der Primer, nicht aber der Kompetitor an die spezifische Bindungsstelle des Primers auf dem Template-Strang bindet, und in einer zweiten Phase eine Annealing-Temperatur gewählt wird, bei der auch eine Bindung des Kompetitors an die spezifische Bindungsstelle des Primers auf dem Template erfolgt.

Vorzugsweise wirkt der Kompetitor gleichzeitig als Sekundärstrukturbrecher.

Ferner ist es bevorzugt, dass mehrere Kompetitoren eingesetzt werden.

Vorzugsweise wirkt mindestens einer der Kompetitoren ausschließlich als Sekundärstrukturbrecher.

Vorzugsweise ist der Komplex des ausschließlich als Sekundärstrukturbrecher wirkenden Kompetitors mit dem Template-Strang weniger stabil als der Komplex mindestens eines inhibierend wirkenden Kompetitors mit dem Template-Strang. Besonders bevorzugt wird die Amplifikation bezüglich des Temperaturregimes als zweiphasiger Prozess durchgeführt, wobei in einer ersten Phase eine Annealing-Temperatur gewählt wird, bei der der inhibierend wirkende Kompetitor, nicht aber der ausschließlich als Sekundärstrukturbrecher wirkende Kompetitor an einen Template-Strang bindet, und in einer zweiten Phase eine Temperatur gewählt wird, bei der auch der ausschließlich als Sekundärstrukturbrecher wirkende Kompetitor an den Template-Strang bindet.

Ferner ist es bevorzugt, dass der Kompetitor und/oder Sekundärstrukturbrecher eine Nukleinsäure und/oder ein Nukleinsäureanalogon ist, das mindestens 10, bevorzugt mindestens 15, besonders bevorzugt mindestens 17 und am meisten bevorzugt mindestens 18 Nukleotide umfasst. Ebenso kann es bevorzugt sein, dass der Kompetitor und/oder Sekundärstrukturbrecher eine Nukleinsäure und/oder ein Nukleinsäureanalogon ist, das mindestens 10, bevorzugt mindestens 15, besonders bevorzugt mindestens 20, ebenfalls besonders bevorzugt mindestens 25, insbesondere bevorzugt mindestens 30 Nukleotide umfasst.

Vorzugsweise liegt ferner der Kompetitor und/oder Sekundärstrukturbrecher zu Beginn der Reaktion mindestens in äquimolaren Mengen zum Target und/oder zu einem der PCR-Primer, bevorzugt im molaren Überschuss zum Target und/oder zu einem der PCR-Primer vor. Besonders bevorzugt liegt das Verhältnis zwischen Primer bzw. Target und Kompetitor bzw. Sekundärstrukturbrecher bezogen auf die molaren Mengen zu Beginn der Reaktion zwischen 0,01 und 0,99, bevorzugt zwischen 0,05 und 0,8 und insbesondere bevorzugt zwischen 0,1 und 0,5.

Als Template-Nukleinsäure wird vorzugsweise DNA eingesetzt. Ebenso kann als Template-Nukleinsäure RNA eingesetzt werden und diese vor der PCR-Amplifikation mit einer reversen Transkriptase in DNA umgeschrieben werden.

Ferner handelt es sich in einer Ausführungsform bei der PCR um eine Multiplex-PCR.

Ein weiterer Aspekt der vorliegenden Erfindung betrifft ein Verfahren zum Nachweis von mindestens einer Nukleinsäure, worin die Nukleinsäure durch ein wie vorstehend beschriebenes erfindungsgemäßes Amplikations-Verfahren amplifiziert wird und anschließend durch Hybridisierung mit einer komplementären Sonde nachgewiesen wird.

Vorzugsweise erfolgen die Amplifikation der Nukleinsäuren und deren Nachweis durch Hybridisierung gegen eine Sonde in einem kontinuierlichen Prozess.

Ferner ist es bevorzugt, dass der Kompetitor den zur Sonde komplementären Template-Strang bindet. Besonders bevorzugt ist, dass der Kompetitor den zur Sonde komplementären Template-Strang in einem Bereich bindet, der nicht durch die Sonde adressiert wird.

Ferner ist es bevorzugt, dass der Kompetitor in unmittelbarer Nachbarschaft des Sequenzbereiches, der durch die Sonde adressiert wird, bindet. Besonders bevorzugt ist der Kompetitor ein DNA-Oligonukleotid das mit dem Template-Strang, der durch die Hybridisierung nachgewiesen wird, in unmittelbarer Nachbarschaft des Sequenzbereichs, der durch die Sonde adressiert wird, hybridisiert.

Des Weiteren kann es bevorzugt sein, dass der Kompetitor in der Nähe des Sequenzbereiches, der durch die Sonde adressiert wird, bindet. Besonders bevorzugt ist auch hier, dass der Kompetitor ein DNA-Oligonukleotid ist, das mit dem Template-Strang, der durch die Hybridisierung nachgewiesen wird, in der Nähe des Sequenzbereichs, der durch die Sonde adressiert wird, hybridisiert.

Ferner ist es bevorzugt, dass der PCR zu Beginn ein Kompetitor, der mit einem der PCR-Primer um die Bindung an das Template konkurriert, und mindestens ein Kompetitor, der in Nähe oder unmittelbarer Nachbarschaft der Sequenz, die durch die Sonde nachgewiesen wird, an das Target bindet und somit als Sekundarstrukturbrecher wirkt, zugesetzt wird.

Ferner kann es bevorzugt sein, dass mindestens einer der ausschließlich als Sekundärstrukturbrecher wirkenden Kompetitoren dem Reaktionsgemisch erst nach Abschluss der PCR zugegeben wird.

Ferner können PCR und Hybridisierung vorzugsweise im gleichen Puffersystem durchgerührt werden.

Des Weiteren werden PCR und/oder Hybridisierung vorzugsweise in einer geschlossenen Reaktionskammer durchgeführt. Eine derartige geschlossene Reaktionskammer ist beispielsweise in den internationalen Patenanmeldungen WO 01/02094, WO 03/031063 und WO 03/059516 beschrieben.

Als "Target" werden erfindungsgemäß nachzuweisende Nukleinsäuremoleküle bezeichnet. Im Rahmen der vorliegenden Erfindung sind mit Targets daher solche Nukleinsäuremoleküle gemeint, die durch eine Hybridisierung gegen auf einem Sondenarray angeordnete Sonden nachgewiesen werden. Im Rahmen der vorliegenden Erfindung handelt es sich dabei um DNA-Moleküle, die durch PCR-Amplifikation aus einem Template hergestellt wurden. Diese Target-Moleküle umfassen in der Regel Sequenzen von 40 bis 10.000 Basen Länge, bevorzugt von 60 bis 2000 Basen Länge, ebenfalls bevorzugt von 60 bis 1000 Basen Länge, insbesondere bevorzugt von 60 bis 500 Basen Länge und am meisten bevorzugt von 60 bis 150 Basen Länge. Ihre Sequenz beinhaltet die Sequenzen der Primer, sowie die durch die Primer definierten Sequenzbereiche des Templates. Bei den Target-Molekülen kann es sich allgemein um einzel- oder doppelsträngige Nukleinsäuremoleküle handeln, von denen ein oder beide Stränge radioaktiv oder nicht-radioaktiv markiert sind, so dass sie in einem der im Stand der Technik üblichen Nachweisverfahren nachgewiesen werden können.

Als "Target-Sequenz" wird erfindungsgemäß der Sequenzbereich des Targets bezeichnet, der durch Hybridisierung mit der Sonde nachgewiesen wird. Erfindungsgemäß wird auch davon gesprochen, dass dieser Bereich durch die Sonde adressiert wird.

Als "Sonde" werden erfindungsgemäß Nukleinsäuremoleküle definierter und bekannter Sequenz bezeichnet, die benutzt werden, um in Hybridisierungsverfahren Target-Moleküle nachzuweisen. Typischerweise handelt es sich erfindungsgemäß bei Sonden um einzelsträngige Nukleinsäuremoleküle oder Moleküle von Nukleinsäure-analoga, bevorzugt einzelsträngige DNA-Moleküle oder RNA-Moleküle, die mindestens über einen Sequenzbereich verfügen, der zu einem Sequenzbereich der Target-Moleküle komplementär ist. Je nach Nachweisverfahren und Anwendung können die Sonden auf einem festen Trägersubstrat, z.B. in Form eines Microarrays immobilisiert sein. Darüber hinaus können sie je nach Nachweisverfahren radioaktiv oder nicht-radioaktiv markiert sein, so dass sie über die im Stand der Technik übliche Nachweisreaktion nachgewiesen werden können.

Als "Array" wird üblicherweise die Anordnung von Molekülen, z.B. Sonden, auf einem Träger bezeichnet. Die Anordnung der Moleküle bzw. Sonden auf dem Träger kann dabei durch kovalente oder nicht kovalente Wechselwirkungen erzeugt werden. Eine Position innerhalb der Anordnung, d.h. des Arrays, wird üblicherweise als Spot bezeichnet.

Herkömmliche Microarrays im Rahmen der vorliegenden Erfindung umfassen etwa 2 bis etwa 80.000, vorzugsweise etwa 10 bis etwa 65.000, besonders bevorzugt etwa 20 bis etwa 1.024 unterschiedliche Spezies von Sondenmolekülen auf einer Flache von mehreren mm² bis mehreren cm², vorzugsweise etwa 1 mm² bis 10 cm², besonders bevorzugt 2 mm² bis 2 cm² und am meisten bevorzugt etwa 4 mm² bis 1 cm². Beispielsweise weist ein herkömmliches Microarray von 4 bis 65.536 unterschiedliche Spezies von Sondenmolekülen auf einer Fläche von 2 mm x 2 mm auf.

Bei dem Träger, der üblicherweise auch als Substrat oder Matrix bezeichnet wird, kann es sich z.B. um Objektträger oder Wafer handeln. Die Gesamtheit aus in Array-Anordnung abgelegten Molekülen und Träger wird häufig auch als "Chip", "Microarray", "DNA-chip", Sondenarray" etc. bezeichnet.

Als "Primer" wird üblicherweise ein kurzes DNA- oder RNA-Oligonukleotid (circa 12 bis 30 Basen) bezeichnet, das komplementär zu einem Abschnitt eines größeren DNA- oder RNA-Moleküls ist und über eine freie 3-OH-Gruppe an seinem 3'-Ende verfügt. Aufgrund dieser freien OH-Gruppe kann der Primer als Substrat für beliebige DNA- oder RNA-Polymerasen dienen, die in 5'-3'-Richtung Nukleotide an den Primer synthetisieren. Die Sequenz der neu synthetisierten Nukleotide ist dabei durch die Sequenz des mit dem Primer hybridisierten Templates vorgegeben, die jenseits der freien 3'-OH-Gruppe des Primers liegt. Primer üblicher Länge umfassen zwischen 12 bis 50 Nukleotide, bevorzugt zwischen 15 und 30 Nukleotide.

Als "Template" oder "Template-Strang" werden üblicherweise ein doppelsträngiges Nukleinsäuremolekül oder ein Nukleinsäurestrang bezeichnet, die als Vorlage zur Synthese von komplementären Nukleinsäuresträngen dienen.

Als "Hybridisierung" wird die Bildung von doppelsträngigen Nukleinsäuremolekülen oder Duplexmolekülen aus komplementären einzelsträngigen Nukleinsäuremolekülen bezeichnet. Im Rahmen einer Hybridisierung können z.B. DNA-DNA-Duplexes, DNA-RNA- oder RNA-RNA-Duplexes gebildet werden. Durch eine Hybridisierung können auch Duplexes mit Nukleinsäureanaloga gebildet werden, wie z.B. DNA-PNA-Duplexes, RNA-PNA-Duplexes, DNA-LNA-Duplexes und RNA-LNA-Duplexes. Hybridisierungsexperimente werden üblicherweise benutzt, um die Sequenzkomplementarität und damit die Identität zwischen zwei verschiedenen Nukleinsäuremolekülen nachzuweisen.

Dazu kann z.B. ein einzelsträngiges Nukleinsäuremolekül, die Sonde, auf einem Träger immobilisiert werden und mit einer Vielzahl von anderen einzelsträngigen Nukleinsäuremolekülen, den Targets, inkubiert werden. Liegt zwischen der Sonde und einem der einzelsträngigen Nukleinsäuremoleküle Sequenzkomplementarität vor, bindet das Target-Molekül an die Sonde. Wenn die Target-Moleküle markiert sind, kann das Hybridisierungsereignis nach Entfernen der nicht gebundenen Target-Moleküle durch Waschen unter Anwendung der entsprechenden Nachweisreaktion nachgewiesen werden.

Dem Fachmann ist bekannt, dass die Bildung stabiler doppelsträngiger Moleküle von der Stringenz der Reaktionsbedingungen abhängt.

Als stringente Reaktionsbedingungen werden solche Bedingungen bezeichnet, die die Bildung eines doppelsträngigen Nukleinsäuremoleküls aus einzelsträngigen Molekülen nur dann erlauben, wenn eine hundertprozentige oder eine hohe Komplementarität zwischen den Einzelsträngen vorliegen.

Eine hohe Komplementarität zwischen Einzelsträngen ist erfindungsgemäß gegeben, wenn die Nukleinsäuresequenzen der einzelsträngigen Moleküle zu mindestens 60%, bevorzugt zu mindestens 70%, ebenfalls bevorzugt zu mindestens 80%, besonders bevorzugt zu mindestens 90%, insbesondere bevorzugt zu mindestens 95% und am meisten bevorzugt zu mindestens 98% komplementär sind.

Dem Fachmann ist bekannt, dass die Stringenz der Hybridisierung von den Temperaturen während der Hybridisierung und der Waschschritte, sowie den Salzkonzentrationen und pH-Werten der Hybridisierungs- und Waschpuffer abhängen.

Eine erhöhte Stringenz kann dadurch erreicht werden, dass zunehmend verdünntere Salzlösungen im Rahmen der Hybridisierung verwendet werden. Höhere Stringenz der Hybridisierung kann auch dadurch erreicht werden, dass dem Hybridisierungs-oder Waschpuffer destabilisierende Agenzien wie z.B. Formamid zugesetzt werden. Typische stringente Bedingungen sind z.B. in Sambrook et al. (*vide supra*) gegeben. Es kann sich dabei z.B. um 6 x SSPE, 0,1% SDS bei 50 bis 65° C handeln.

Als niedrigstringente Bedingungen werden solche Reaktionsbedingungen bezeichnet, bei denen im Rahmen einer Hybridisierungsreaktion die Bildung von doppelsträngigen Nukleinsäuremolekülen möglich ist, die nur über eine eingeschränkte Komplementarität verfügen. Erfindungsgemäß verfügen Nukleinsäuremoleküle über eine eingeschränkte Komplementarität, wenn sie hinsichtlich ihrer Sequenz zu 20%, bevorzugt zu höchstens 30%, besonders bevorzugt zu höchstens 40%, insbesondere bevorzugt zu höchstens 50% und am meisten bevorzugt zu höchstens 60% komplementär sind.

Als komplementär werden zwei Nukleinsäuresequenzen bezeichnet, wenn die Sequenzen aufgrund ihrer Basen-Abfolge Wasserstoffbrückenbildungen zwischen den Nukleotiden der einzelnen Nukleinsäuremoleküle ausbilden können und sich entsprechend doppelsträngige Nukleinsäuremoleküle bilden. Wenn sich zwischen jedem der gegenüberliegenden Nukleotidpaare von zwei einzelsträngigen Nukleinsäuremolekülen Wasserstoffbrücken ausbilden können, spricht man von 100% Komplementarität.

Bei dem erfindungsgemäßen Verfahren kann als Kompetitor in der PCR jedes Molekül eingesetzt werden, das eine bevorzugte Amplifikation nur eines der beiden in der PCR-Reaktion vorhandenen Template-Stränge bewirkt. Bei Kompetitoren kann es sich daher erfindungsgemäß um Nukleinsäuren oder deren Analoga handeln.

Besonders bevorzugt werden erfindungsgemäß als Sekundärstrukturbrecher Nukleinsäuremoleküle und Nukleinsäure-Analoga-Moleküle eingesetzt.

Erfindungsgemäß wird durch anfängliche Zugabe des Kompetitors zur PCR während der Amplifikation die Bildung eines der beiden Template-Stränge im wesentlichen inhibiert. "Im wesentlichen inhibiert" bedeutet erfindungsgemäß, dass im Rahmen der PCR ein ausreichender Einzelstrangüberschuss und eine ausreichende Menge des anderen Template-Strangs hergestellt werden, um einen effizienten Nachweis des amplifizierten Strangs durch die Hybridisierung zu gewährleisten. Die Amplifikation folgt damit nicht einer exponentiellen Kinetik der Form 2ⁿ (mit n = Anzahl der Zyklen), sondern einer gedämpften Amplifikationskinetik der Form <2ⁿ.

Erfindungsgemäß beträgt der durch das erfindungsgemäße PCR-Verfahren erzielte Einzelstrangüberschuss des amplifizierten Strangs gegenüber dem nichtamplifizierten Strang den Faktor 1,1 bis 1.000, bevorzugt den Faktor 1,1 bis 300, ebenfalls bevorzugt den Faktor 1,1 bis 100, besonders bevorzugt den Faktor 1,5 bis 100, ebenfalls besonders bevorzugt den Faktor 1,5 bis 50, insbesondere bevorzugt den Faktor 1,5 bis 20 und am meisten bevorzugt den Faktor 2 bis 10.

Typischerweise wird die Funktion eines Kompetitors darin bestehen, dass er selektiv an einen der beiden Template-Stränge bindet und damit die Amplifikation des entsprechenden komplementären Stranges behindert.

Nukleinsäuren oder Nukleinsäure-Analoga werden als Kompetitoren im erfindungsgemäßen Verfahren eingesetzt. Üblicherweise werden die Nukleinsäuren bzw. Nukleinsäure-Analoga dadurch als Kompetitor der PCR wirken, dass sie entweder mit einem der zur PCR verwendeten Primer um die Primer-Bindungsstelle konkurrieren oder, aufgrund einer Sequenz-Komplementarität mit einem Bereich eines nachzuweisenden Template-Strangs hybridisieren können. Bei diesem Bereich handelt es sich erfindungsgemäß nicht um die Sequenz, die durch die Sonde nachgewiesen wird. Solche Nukleinsäure-Kompetitoren sind erfindungsgemäß enzymatisch nicht verlängerbar.

Bei den Nukleinsäure-Analoga kann es sich z.B. um sogenannte Peptid-Nukleinsäuren (peptide nucleic acids, PNA) handeln. Bei Nukleinsäure-Analoga kann es sich aber auch um Nukleinsäuremoleküle handeln, bei denen die Nukleotide über eine Phosphothioat-Bindung anstelle einer Phosphat-Bindung miteinander verknüpft sind. Ebenso kann es sich um Nukleinsäure-Analoga handeln, bei denen die natürlich vorkommenden Zuckerbausteine Ribose bzw. Desoxyribose gegen alternative Zucker wie z.B. Arabinose oder Trehalose ("trehalose nucleic acid", TNA) etc. ausgetauscht wurden. Weiterhin kann es sich bei dem Nukleinsäurederivat um "locked nucleic acid" (LNA) handeln. Weitere übliche Nukleinsäure-Analoga sind dem Fachmann bekannt

Bevorzugt werden als Kompetitoren DNA- oder RNA-Moleküle, insbesondere bevorzugt DNA- oder RNA-Oligonukleotide bzw. deren Analoga eingesetzt.

Abhängig von der Sequenz der als Kompetitoren eingesetzten Nukleinsäuremoleküle bzw. Nukleinsäure-Analoga beruht die Inhibierung der Amplifikation eines der beiden Template-Stränge im Rahmen der PCR-Reaktion auf unterschiedlichen Mechanismen. Dies wird im Folgenden beispielhaft anhand eines DNA-Moleküls diskutiert.

Wenn als Kompetitor z.B. ein DNA-Molekül verwendet wird, kann dies eine Sequenz aufweisen, die mit der Sequenz eines der zu PCR verwendeten Primer zumindest teilweise derart identisch ist, dass eine spezifische Hybridisierung des DNA-Kompetitor-Moleküls mit dem entsprechenden Template-Strang unter stringenten Bedingungen möglich ist. Da das zur Kompetition verwendete DNA-Molekül erfindungsgemäß in diesem Fall nicht durch eine DNA-Polymerase verlängerbar ist, kompetiert das DNA-Molekül mit dem jeweiligen Primer während der PCR-Reaktion um die Bindung an das Template. Je nach Mengenverhältnis des DNA-Kompetitor-Moleküls zum Primer kann auf diese Weise die Amplifikation des durch den Primer definierten Template-Strangs derart inhibiert werden, dass die Herstellung dieses Template-Strangs deutlich reduziert ist. Die PCR verläuft dabei nach einer exponentiellen Kinetik, die höher ist, als bei den verwendeten Kompetitor-Mengen zu erwarten wäre. Auf diese Weise entsteht ein Einzelstrang-überschuss in einer Menge, die ausreichend für einen effizienten Nachweis der amplifizierten Target-Moleküle durch Hybridisierung ist.

Erfindungsgemäß dürfen bei dieser bevorzugten Ausführungsform die zur Kompetition verwendeten Nukleinsäuremoleküle bzw. Nukleinsäureanaloga enzymatisch nicht verlängerbar sein.

"Enzymatisch nicht verlängerbar" bedeutet, dass die zur Amplifikation verwendete DNA- oder RNA-Polymerase den Nukleinsäure-Kompetitor nicht als Primer verwenden kann, d.h. nicht in der Lage ist, 3' von der durch den Kompetitor definierten Sequenz den jeweiligen Gegenstrang zum Template zu synthetisieren.

Alternativ zu der dargestellten Möglichkeit kann das DNA-Kompetitor-Molekül auch über eine Sequenz verfügen, die zu einem Bereich des nachzuweisenden Template-Strangs komplementär ist, der nicht durch eine der Primer-Sequenzen adressiert wird, und die enzymatisch nicht verlängerbar ist. Im Rahmen der PCR wird das DNA-Kompetitor-Molekül dann an diesem Template-Strang hybridisieren und die Amplifikation dieses Stranges entsprechend blockieren.

Dem Fachmann ist bekannt, dass die Sequenzen von DNA-Kompetitor-Molekülen oder allgemein Nukleinsäure-Kompetitor-Molekülen entsprechend gewählt werden können. Wenn die Nukleinsäure-Kompetitor-Moleküle eine Sequenz aufweisen, die nicht mit der Sequenz eines der zur PCR verwendeten Primer im Wesentlichen identisch, sondern zu einem anderen Bereich des nachzuweisenden Template-Strangs komplementär ist, ist erfindungsgemäß diese Sequenz so zu wählen, dass sie nicht in den Bereich der Template-Sequenz fällt, der im Rahmen der Hybridisierung mit einer Sonde nachgewiesen wird. Dies ist deswegen notwendig, da zwischen der PCR und der Hybridisierungsreaktion bei dem erfindungsgemäßen Verfahren keine Aufarbeitungsreaktion stattfinden muss. Würde als Kompetitor ein Nukleinsäuremolekül verwendet, das in den nachzuweisenden Bereich fällt, würde dies mit dem einzelsträngigen Target-Molekül um die Bindung an die Sonde kompetieren.

Bevorzugt hybridisieren solche Kompetitoren in der Nähe der Template-Sequenz, die durch die Sonde nachgewiesen wird. Die Positionsangabe "in der Nähe" ist dabei erfindungsgemäß so zu verstehen, wie sie für Sekundärstrukturbrecher angegeben ist (siehe unten). Allerdings können die erfindungsgemäßen Kompetitoren auch in unmittelbarer Nachbarschaft der nachzuweisenden Sequenz hybridisieren, d.h. exakt ein Nukleotid von der nachzuweisenden Target-Sequenz entfernt.

Wenn als kompetierende Moleküle enzymatisch nicht verlängerbare Nukleinsäuren oder Nukleinsäure-Analoga verwendet werden, sind diese hinsichtlich ihrer Sequenz oder Struktur so zu wählen, dass sie nicht enzymatisch durch DNA- oder RNA-Polymerasen verlängert werden können. Bevorzugt ist das 3'-Ende eines Nukleinsäure-Kompetitors so ausgelegt, dass es keine Komplementarität zum Template aufweist und/oder anstelle der 3-OH-Gruppe am 3'-Ende einen anderen Substituenten trägt.

Weist das 3'-Ende des Nukleinsäure-Kompetitors keine Komplementarität zum Template auf, unabhängig davon, ob der Nukleinsäure-Kompetitor an eine der Primer-Bindungsstellen des Templates oder an eine der durch die PCR zu amplifizierenden Sequenzen des Templates bindet, kann der Nukleinsäure-Kompetitor wegen der fehlenden Basen-Komplementarität am 3'-Ende nicht durch die gängigen DNA-Polymerasen verlängert werden. Diese Art der Nicht-Verlängerbarkeit von Nukleinsäure-Kompetitoren durch DNA-Polymerasen ist dem Fachmann bekannt. Bevorzugt weist der Nukleinsäure-Kompetitor an seinem 3'-Ende bezüglich der letzten 4 Basen, besonders bevorzugt bezüglich der letzten 3 Basen, insbesondere bevorzugt bezüglich der letzten 2 Basen und am meisten bevorzugt bezüglich der letzten Base keine Komplementarität zu seiner Zielsequenz auf. Solche Kompetitoren können an den genannten Positionen auch nicht-natürliche Basen aufweisen, die keine Hybridisierung erlauben.

Erfindungsgemäß können Nukleinsäure-Kompetitoren, die enzymatisch nicht verlängerbar sind, auch eine 100%-ige Komplementarität zu ihrer Zielsequenz aufweisen, wenn sie in ihrem Rückgrat oder an ihrem 3'-Ende derart modifiziert sind, dass sie enzymatisch nicht verlängerbar sind.

Weist der Nukleinsäure-Kompetitor an seinem 3'-Ende eine andere Gruppe als die OH-Gruppe auf, handelt es sich bei diesen Substituenten bevorzugt um eine Phosphat-Gruppe, um ein Wasserstoff-Atom (Dideoxynukleotid), eine Biotingruppe oder eine Aminogruppe. Diese Gruppen können durch die gängigen Polymerasen nicht verlängert werden.

Besonders bevorzugt wird bei einem erfindungsgemäßen Verfahren als Kompetitor ein DNA-Molekül verwendet, das mit einem der beiden zur PCR verwendeten Primer um die Bindung an das Template kompetiert und welches am 3'-Ende während der chemischen Synthese mit einem Aminolink versehen wurde. Solche Kompetitoren können 100%-ige Komplementarität zu ihrer Zielsequenz haben.

Erfindungsgemäße Nukleinsäure-Analoga-Kompetitoren wie z.B. PNAs müssen dagegen nicht über eine blockierte 3'-OH-Gruppe oder eine nicht-komplementäre Base an ihrem 3'-Ende verfügen, da sie aufgrund des durch die Peptid-Bindung veränderten Rückgrats nicht durch die DNA-Polymerasen erkannt und somit auch nicht verlängert werden. Entsprechende andere Modifikationen der Phosphatgruppe, die durch die DNA-Polymerasen nicht erkannt werden, sind dem Fachmann bekannt. Dazu gehören u.a. Nukleinsäuren mit Rückgrat-modifikationen wie z.B. 2'-5' Amid-Bindungen (Chan et al. (1999) J. Chem. Soc., Perkin Trans. 1, 315-320), Sulfid-Bindungen (Kawai et al. (1993) Nucleic Acids Res., 1 (6), 1473-1479), LNA (Sorensen et al. (2002) J. Am. Chem. Soc., 124 (10), 2164-2176) und TNA (Schoning et al. (2000) Science, 290 (5495), 1347-1351).

Erfindungsgemäß können auch mehrere Kompetitoren, die an unterschiedliche Bereiche des Templates (z.B. u.a. die Primer-Bindungsstelle) hybridisieren, gleichzeitig in einer PCR eingesetzt werden. Wenn die Kompetitoren über Sekundärstrukturbrechereigenschaften verfügen, kann dadurch die Effizienz der Hybridisierung zusätzlich gesteigert werden.

In einer alternativen Ausführungsform kann das DNA-Kompetitor-Molekül über eine zu einem der Primer komplementäre Sequenz verfügen. Solche z.B. Antisense-DNA-Kompetitor-Moleküle können dann je nach Mengenverhältnis zwischen Antisense-DNA-Kompetitor-Molekül und Primer dazu verwendet werden, den Primer in der PCR-Reaktion zu titrieren, so dass dieser nicht mehr mit dem jeweiligen Template-Strang hybridisiert und entsprechend nur der durch den anderen Primer definierte Template-Strang amplifiziert wird. Dem Fachmann ist bewusst, dass bei dieser Ausführungsform der Erfindung der Nukleinsäure-Kompetitor enzymatisch verlängerbar sein kann, aber nicht muss.

Wenn erfindungsgemäß von Nukleinsäure-Kompetitoren gesprochen wird, schließt dies Nukleinsäure-Analoga-Kompetitoren mit ein, wenn sich nicht aus dem Kontext etwas anderes ergibt. Der Nukleinsäure-Kompetitor kann an den entsprechenden Strang des Templates reversibel oder irreversibel binden. Die Bindung kann durch kovalente bzw. nicht kovalente Wechselwirkungen erfolgen.

Bevorzugt erfolgt die Bindung des Nukleinsäure-Kompetitors über nicht-kovalente Wechselwirkungen und ist reversibel. Insbesondere bevorzugt erfolgt die Bindung an das Template durch Ausbildung von Watson-Crick-Basenpaarungen.

Die Sequenzen der Nukleinsäure-Kompetitoren richten sich in der Regel nach der Sequenz des Template-Strangs, der nachgewiesen werden soll. Bei antisense-Primern dagegen nach den zu titrierenden Primer-Sequenzen, die aber wiederum durch die Template-Sequenzen definiert sind.

Bei der PCR-Amplifikation von Nukleinsäuren handelt es sich um eine Labor-Standardmethode, mit deren vielfältigen Variations- und Ausgestaltungsmöglichkeiten der Fachmann vertraut ist. Prinzipiell ist eine PCR dadurch charakterisiert, dass das doppelsträngige Nukleinsäure-Template, üblicherweise ein doppelsträngiges DNA-Molekül, zuerst einer Hitze-Denaturierung für 5 Minuten bei 95° C unterworfen wird, wodurch die beiden Stränge voneinander getrennt werden. Nach einer Abkühlung auf die sogenannte "annealing"-Temperatur (definiert durch den Primer mit der niedrigeren Schmelztemperatur) lagern sich die in der Reaktionslösung vorhandenen "forward"- und "reverse"-Primer an die zu ihrer Sequenz komplementären Stellen in den jeweiligen Template-Strängen an. Die "annealing"-Temperatur der Primer richtet sich dabei nach der Länge und Basenzusammensetzung der Primer. Sie kann aufgrund theoretischer Überlegungen kalkuliert werden. Angaben zur Kalkulation von "annealing"-Temperaturen finden sich z.B. in Sambrook et al. (*vide supra*).

Nach dem Annealen der Primer, das typischerweise in einem Temperaturbereich von 40-75° C, bevorzugt von 45-72° C und insbesondere bevorzugt von 50-72° C erfolgt, folgt ein Elongationsschritt, bei dem durch die Aktivität der in der Reaktionslösung vorhandenen DNA-Polymerase Desoxyribonukleotide mit dem 3'-Ende der Primer verknüpft werden. Die Identität der eingefügten dNTPs richtet sich dabei nach der Sequenz des mit dem Primer hybridisierten Template-Strangs. Da in der Regel thermostabile DNA-Polymerasen eingesetzt werden, läuft der Elongationsschritt üblicherweise zwischen 68-72° C ab.

Bei der symmetrischen PCR wird durch eine Wiederholung dieses beschriebenen Zyklus aus Denaturierung, Annealing der Primer und Elongation der Primer eine exponentielle Vermehrung des durch die Primersequenzen definierten Nukleinsäureabschnitts des Targets erreicht.

Hinsichtlich der Pufferbedingungen bei der PCR, der verwendbaren DNA-Polymerasen, der Herstellung von doppelsträngigen DNA-Templates, des Designs von Primern, der Wahl der Annealing-Temperatur und Variationen der klassischen PCR steht dem Fachmann zahlreiche Literatur zur Verfügung. Somit wird unter einer PCR im Rahmen der vorliegenden Erfindung nicht nur eine klassische PCR, sondern auch eine PCR-Variation wie RT-PCR, inverse PCR, quantitative PCR, Multiplex-PCR, Hot Start-PCR, Touchdown-PCR und dergleichen verstanden (siehe auch Sambrook et al., Molecular Cloning - A Laboratory Manual, Band 2, 3. Auflage, Cold Spring Harbor Laboratory Press, Cold Spring Harbor, New York, 2001).

Dem Fachmann ist geläufig, dass als Template auch z.B. einzelsträngige RNA, wie z.B. mRNA, eingesetzt werden kann. Diese wird in der Regel vorher durch eine Reverse Transkription in eine doppelsträngige cDNA überführt.

In einer bevorzugten Ausführungsform des Verfahrens wird als Polymerase eine thermostabile DNA-abhängige DNA-Polymerase verwendet. In einer besonders bevorzugten Ausführungsform wird eine thermostabile DNA-abhängige DNA-Polymerase ausgewählt aus der Gruppe bestehend aus Taq-DNA-Polymerase (Eppendorf, Hamburg, Deutschland sowie Qiagen, Hilden, Deutschland), Pfu-DNA-Polymerase (Stratagene, La Jolla, USA), Tth-DNA-Polymerase (Biozym Epicenter Technol., Madison, USA), Vent-DNA-Polymerase, DeepVent-DNA-Polymerase (New England Biolabs, Beverly, USA), Expand-DNA-Polymerase (Roche, Mannheim, Deutschland) verwendet.

Die Verwendung von Polymerasen, die aus natürlich vorkommenden Polymerasen durch gezielte oder evolutive Veränderung optimiert worden sind, ist ebenfalls bevorzugt Bei der Durchführung des erfindungs gemäßen Verfahrens ist insbesondere die Verwendung der Taq-Polymerase der Firma Eppendorf (Deutschland) bzw. des Advantage-cDNA-Polymerase-Mix von Clontech (Palo Alto, CA, USA) bevorzugt.

Als Reaktionspuffer werden im Rahmen des erfindungsgemäßen Verfahrens vor allem Reaktionspuffer für thermostabile DNA-abhängige DNA-Polymerasen verwendet, wie sie kommerziell angeboten werden. Dabei findet insbesondere der mit der jeweils verwendeten Polymerase gelieferte Reaktionspuffer Verwendung. Besonders bevorzugt ist die Verwendung des cDNA-PCR-Reaction Buffer der Firma Clontech (Palo Alto, CA, USA).

In besonders bevorzugten Ausführungsformen des erfindungsgemäßen Verfahrens kann die Ionenstärke des PCR-Reaktionspuffers durch Zugabe von Salzen erhöht werden. Dies kann durch die Zugabe von einwertigen Ionen wie K⁺, Na⁺ oder anderen Ionen erfolgen. Besonders bevorzugt wird der cDNA-PCR-Reaction-Buffer der Firma Clontech in Verbindung mit einer erhöhten Kaliumacetat-Konzentration verwendet. Üblicherweise liegt die Konzentration der einwertigen Metallionen zwischen 0 mM und 2M, bevorzugt zwischen 20 mM und 200 mM und besonders bevorzugt zwischen 40 mM und 120 mM.

Die erhöhte Ionenstärke beeinflusst die PCR-Reaktion selbst nicht, ist aber vorteilhaft für die effiziente Hybridisierung der PCR-Fragmente an die Sonden.

Bei einer bevorzugten Ausführungsform haben der um eine Bindungsstelle konkurrierende Primer und Kompetitor bevorzugt die gleichen Bindungseigenschaften (Tm-Wert, annealing Temperatur oder Schmelztemperatur). Alternativ können sich Kompetitor und Primer auch in ihren Bindungseigenschaften unterscheiden. In einem bevorzugten Fall hat der Kompetitor eine geringere Schmelztemperatur als der konkurrierende Primer. Dies ist besonders bevorzugt, wenn die PCR in einem kontinuierlichen aber bezüglich des Temperaturregimes und der Amplifikationskinetik zweistufigen Prozess durchgeführt werden soll.

In diesem Fall wird in der ersten Phase der Amplifikation im Annealing-Schritt der PCR eine Temperatur gewählt, bei der der Primer, aber nicht der Kompetitor an den Templatestrang bindet. Unter diesen Bedingungen erfolgt die Amplifikation symmetrisch. Es entsteht kein Einzelstrangüberschuss. In der zweiten Amplifikationsphase wird ein Einzelstrangüberschuss erzeugt, indem der Hybridisierungsschritt des PCR-Zyklus bei einer, vorzugsweise erniedrigten, Temperatur stattfindet, bei der sowohl Primer als auch Kompetitor binden. Dieses zweistufige Temperatur-regime ist analog auch bei der Verwendung von Kompetitoren, die an Primer binden, sowie beim Einsatz von Strukturbrechern bevorzugt (siehe oben).

In einer speziellen Ausführungsform kann Primer- und Kompetitorkomponente auf ein und demselben Molekül (Primer-Kompetitor) lokalisiert sein. Der Primer-Kompetitor hat in dieser Ausführungsform bevorzugt eine Haarnadelstruktur, bei der der 3'-Bereich der Primersequenz entspricht, während der 5'-Bereich eine zum Primer zumindest teilweise komplementäre Sequenz aufweist. Bei dieser Ausführungsform konkurriert die Hybridisierung von Kompetitormodul und Primer mit der Hybridisierung des Primers an das Template. Es handelt sich somit um den bereits erwähnten antisense-Kompetitor-Ansatz. In einer bevorzugten Ausführungsform sind Primermodul und Kompetitormodul des Primer-Kompetitors durch eine Region miteinander verknüpft, die nicht als Template für Polymerasen dienen kann. Besonders bevorzugt handelt es sich dabei um basenfreie Abschnitte, oder Abschnitte mit nicht-natürlichen Basen, die mit den natürlichen Basen keine Basenpaarung eingehen können. Ebenso besonders bevorzugt ist in diesem Zusammenhang das Ersetzen der Basen durch andere Bausteine bzw. die Verbindung von Primer- und Kompetitormodul durch Spacerstrukturen, die keine DNA darstellen, beispielsweise Polyethylenglycol-Derivate, Peptide, Terpene u.a.. Durch diese Bereiche wird verhindert, dass die Polymerase das 3'-Ende des zum Primer-Kompetitor komplementären Templatestranges nach Hybridisierung des Primer-Kompetitors bindet und die Kompetitorregion kopiert. Dies würde dazu führen, dass sich am Ende des verlängerten Templates eine Haarnadelstruktur ausbilden könnte, die dann wiederum durch die Polymerase in Abhängigkeit des gleichen Stranges verlängert werden könnte ("self priming").

In einer besonders bevorzugten Ausführungsform des antisense-Kompetitor-Ansatzes ist die Stabilität (Tm-Wert) des Hybrids von Primer- und Kompetitorkomponente geringer als die Stabilität des Hybrids aus Primer und Template. In diesem Fall wird die PCR in einem kontinuierlichen aber bezüglich des Temperaturregimes und der Amplifikationskinetik zweistufigen Prozess wie oben beschrieben durchgeführt.

In diesem Fall wird in der ersten, PCR-Phase die Ausbildung des Primer-Kompetitor-Hybrids verhindert, indem eine Annealing-Temperatur gewählt wird, die über dem Tm-Wert des Primer-Kompetitor-Hybrids liegt. In der zweiten Phase wird die Hybridisierungstemperatur abgesenkt, so dass durch Ausbildung des Primer-Kompetitor-Hybrids die Hybridisierung von Primer und Template teilweise oder vollständig verhindert wird.

Besonders bevorzugt wird die Amplifikation als ein einphasiger Prozess durchgeführt. Primer und Kompetitor stellen in diesem Prozess besonders bevorzugt diskrete Moleküle dar, wobei der Kompetitor enzymatisch nicht verlängerbar ist und mit dem Primer um die Bindung an das Template kompetiert.

Das Verhältnis von Primer zu Kompetitor bezogen auf die molaren Mengen liegt am Beginn der Reaktion zwischen 0,01 und 0,99, bevorzugt zwischen 0,05 und 0,8 und besonders bevorzugt zwischen 0,1 und 0,5.

Der Kompetitor liegt erfindungsgemäß somit mindestens in äquimolaren Mengen und bevorzugt im 2- ,5-, 10-, 20-, 50-, 100-, 1000-fachen molaren Überschuss zu einem der PCR-Primer zu Anfang der Reaktion vor.

Durch das erfindungsgemäße Verfahren kann das Hybridisierungssignal bis um den Faktor 2 bis 10.000, bevorzugt um den Faktor 2 bis 1.000, ebenfalls bevorzugt um den Faktor 2 bis 500, besonders bevorzugt um den Faktor 2 bis 200, ebenfalls besonders bevorzugt um den Faktor 2 bis 100, insbesondere bevorzugt um den Faktor 2 bis 60 und am meisten bevorzugt um den Faktor 2 bis 30 im Vergleich zu Verfahren, bei denen das Target in einer symmetrischen PCR amplifiziert wird, gesteigert werden.

Wie bereits erwähnt, handelt es sich bei einer besonders bevorzugten Ausführungsform der Erfindung um ein Verfahren, bei dem zunächst eine PCR durchgeführt wird, der zu Anfang ein Nukleinsäure-Kompetitor zugefügt wurde, der mit einem der beiden zur PCR verwendeten Primer um die Bindung an den entsprechenden Target-Strang konkurriert und nicht enzymatisch verlängerbar ist. Dadurch entsteht im Rahmen der PCR ein Einzelstrangüberschuss des Target-Moleküls, das dann in der anschließenden Hybridisierung gegen eine entsprechende Sonde nachgewiesen werden kann. Ein solches Verfahren kann z.B. bei einer Microarray-basierten Auswertung durchgeführt werden und weist vielfältige Vorteile auf.

Das erfindungsgemäß besonders bevorzugte Verfahren läuft dabei nach folgender Ausführungsform ab: Zunächst wird ein Reaktionsgemisch angesetzt, bestehend aus einer Template-DNA und Reagenzien, die die PCR-Amplifikation eines Abschnitts der Template-DNA gestatten. Bei diesen Reagenzien handelt es sich typischerweise um einen Reaktionspuffer, eine thermostabile DNA-abhängige DNA-Polymerase, Desoxynukleotidtriphosphate (dNTP) sowie gegebenenfalls weitere Komponenten. Ebenfalls der Reaktion zugesetzt werden ein oder mehrere Primerpaare, die Komplementarität zu den die zu amplifizierende Region auf dem Template einschließenden Bereichen auf jedem Strang besitzen, sowie ein Nukleinsäure-Kompetitor, der mit einem Primer des Primerpaars um die Bindungsstelle auf dem Template konkurriert und nicht als Primer dienen kann, da er selbst nicht enzymatisch verlängerbar ist. Erfindungsgemäß können auch mehrere Primerpaare und Kompetitoren gleichzeitig eingesetzt werden, so dass mehrere Template-Bereiche gleichzeitig im Einzelstrangüberschuss durch eine PCR amplifiziert werden. Erfindungsgemäß können auch mehrere Templates eingesetzt werden.

Das Reaktionsgemisch wird in geeigneter Form mit einem Microarray in Kontakt gebracht, der Sonden trägt, die zumindest zum Teil Komplementarität zu dem Strang des erwarteten Amplifikationsprodukts aufweisen. Das mit dem Sondenarray versehene Reaktionsgemisch wird zunächst unter Bedingungen inkubiert, die eine PCR-Amplifikation gestatten. Daraufhin erfolgt die Inkubation unter Bedingungen, bei denen eine Hybridisierung des Amplifikationsprodukts mit den Sonden des Microarrays stattfinden kann. Da die zur Amplifikation verwendeten Primer vorher entsprechend markiert worden sind und die Identität und Lokalisation der Sonden auf dem Array bekannt sind, kann das Hybridisierungsmuster auf dem Sondenarray mit einer geeigneten Detektionseinrichtung ausgelesen werden und Informationen darüber geben, welche Target-Moleküle in einer Probe enthalten sind. Gegegebenfalls werden der Microarray und das Reaktionsgemisch vor der Auswertung getrennt und der Array in einem geeigneten Puffer gespült.

Die Hybridisierung erfolgt erfindungsgemäß bevorzugt in dem gleichen Puffer, in dem die PCR-Reaktion durchgeführt wurde. Dabei handelt es sich üblicherweise um einen Standard-PCR-Puffer, bevorzugt wird der PCR-Puffer durch Zugabe von Ionen bezüglich seiner Ionenstärke erhöht (siehe oben). Besonders bevorzugt findet die Hybridisierung im cDNA-Reaktionspuffer der Firma Clontech (Palo Alto, CA, USA) statt, dessen Ionenstärke, bevorzugt durch Zugabe von Kaliumacetat, auf eine Endkonzentration von 15 bis 100 mM, besonders bevorzugt von 50 bis 90 mM erhöht wurde.

Die optimale Hybridisierungstemperatur hängt u. a. von der Zusammensetzung des verwendeten Puffers und der Länge der hybridisierenden Bereiche ab und liegt zwischen 0°C und 65°C. Bei Verwendung des besonders bevorzugten Puffersystems und einer Länge der hybridisierenden Bereiche zwischen 18 und 25 Basen liegt die bevorzugte Hybridisierungs-temperatur zwischen 20°C und 55°C, besonders bevorzugt zwischen 30°C und 45°C und am meisten bevorzugt zwischen 35°C und 45°C.

Die weiter unten dargestellten Experimente zeigen, dass diese bevorzugte Ausführung des erfindungsgemäßen Verfahrens mehrere Vorteile mit sich bringt. Als Primer wurden in den weiter unter dargestellten Experimenten DNA-Moleküle mit einer definierten Sequenz und einem zur Target-Sequenz des Templates komplementären, durch DNA-Polymerasen verlängerbaren 3'-Ende verwendet, während es sich bei dem Kompetitor um ein DNA-Molekül gleicher Länge und Sequenz bezüglich eines Primers handelte, wobei das 3'-Ende durch eine Aminomodifikation geblockt wurde. Das Verhältnis von Primer und Kompetitor wurde bei gleicher Endkonzentration des Primer-Kompetitor-Gemisches zwischen 0% und 100% Kompetitor-Anteil variiert.

In dieser bevorzugten Ausführungsform konkurrieren Primer und Kompetitor während der Reaktion um die gleiche Bindungsstelle auf der Zielsequenz. In Abhängigkeit von dem gewählten Primer-Kompetitor-Verhältnis wird während des Annealingschrittes der PCR ein bestimmter Anteil eines Templatestranges vom Kompetitor gebunden. Dieser kann im anschließenden Elongationsschritt nicht verlängert werden, so dass der vom Kompetitor gebundene Einzelstrang auch nach dem Elongationsschritt als überschüssiger Einzelstrang vorliegt. Im Gegensatz dazu wird der Gegenstrang vollständig umgeschrieben, da für diesen kein Kompetitor zugesetzt wird. Die in Bezug auf die Hybridisierungseigenschaften des Amplifkationsproduktes erwünschte Folge ist, dass über den gesamten Verlauf der Reaktion ein Einzelstrangüberschuss existiert, also eine asymmetrische Amplifikation erfolgt.

Bei der Quantifizierung der Amplifikationsprodukte über den gesamten Verlauf der Reaktionen war zwar festzustellen, dass die Amplifikationsrate durch Zugabe des Kompetitors verringert wurde. Die Amplifikationsrate wurde überraschenderweise jedoch deutlich weniger reduziert, als rechnerisch aus dem gewählten Primer-Kompetitor-Verhältnis zu erwarten gewesen wäre.

Zusätzlich wurde in Hybridisierungs-Experimenten, in denen gleiche Volumina aus den verschiedenen PCR-Ansätzen zur Hybridisierung verwendet wurden, festgestellt, dass die Hybridisierungs-Signale bei asymmetrischer Amplifikation über den gesamten detektierbaren Verlauf der Reaktion, insbesondere bei einem Kompetitor-Anteil von 50-90%, d.h. bis zu einem 9-fachen molaren Überschuss, gegenüber der symmetrischen Amplifikation um den Faktor 2 bis 100 erhöht waren. Dies war überraschend, da bei einem solch hohen Kompetitor-Anteil erwartet wurde, dass die Amplifikation so stark verlangsamt wird, dass die erzeugte Menge an Amplifikationsprodukt unter der Nachweisgrenze der Hybridisierung liegt.

Die nach dem oben beschriebenen Prinzip der asymmetrischen Amplifikation erzeugten PCR-Produkte weisen somit über den gesamten Verlauf der Reaktion einen Einzelstrangüberschuss auf. Sie können daher in Hybridisierungs-Assays wesentlich sensitiver nachgewiesen werden, als durch symmetrische Amplifikation erzeugte, komplett doppelsträngige DNA-Moleküle.

Im weiteren wurde festgestellt, dass die durch die erfindungsgemäße asymmetrische PCR erzeugten PCR-Produkte nicht nur in klassischen Hybridisierungspuffem effizient mit Sonden auf Sondenarrays hybridisieren, sondern dass eine effiziente und spezifische Hybridisierung auch in Puffern stattfinden kann, die als PCR-Reaktions-puffer geeignet sind. Dies eröffnet die Möglichkeit, Nachweisreaktionen, in denen eine PCR-Amplifikation von einer Hybridisierungsreaktion gefolgt wird, in ein und demselben Puffer in einem kontinuierlichen Prozess durchzuführen. Bei einem solchen erfindungsgemäß bevorzugten Verfahren werden am Anfang der Nachweisreaktion alle für die PCR-Amplifikation notwendigen Komponenten vereinigt, das Reaktionsgemisch mit einem Array versehen und die Reaktion anschließend einem für die Amplifikation des jeweiligen Templates geeigneten PCR-Temperatur-Regime sowie einem anschließenden Hybridisierungsschritt unterworfen.

Die PCR-Amplifikation kann auch als Multiplex-Reaktion durchgeführt werden. Dabei wird für jedes in der Multiplex-Reaktion zu amplifizierende Template vorzugsweise ein Primer-Paar mit entsprechendem Kompetitor zugegeben. Dies ist besonders bevorzugt, wenn die Multiplex-Reaktion in geschlossenen Reaktionskammern ausgeführt werden soll, bei denen während der Reaktion eine Zugabe weiterer Komponenten nicht möglich ist.

Andernfalls kann die Multiplex-Reaktion auch in einem zweistufigen Prozess durchgeführt werden, wie er in Favis et al. (Nat. Biotechnol. (2000) 18(5):561-4) beschrieben ist. In diesem Fall wird die Multiplex-Reaktion durchgeführt, indem zunächst für jedes zu amplifizierende Fragment eine limitierende Menge eines Primerpaars zugegeben wird, dessen 3'-Ende Homologie zum jeweiligen Template aufweist, dessen 5'-Enden jedoch für alle forward- und alle reverse-Primer identisch sind (universelle Region). Die zweite Phase der Reaktion wird mit einem Primerpaar durchgeführt, das zu den universellen Regionen komplementär ist. Wenn ein solches Multiplex-Verfahren angewendet wird, ist die Verwendung nur eines Kompetitors besonders bevorzugt. Dieser Kompetitor ist so beschaffen, dass er mit einem der universellen Primer um die Bindungsstelle auf den Templates konkurriert. Besonders bevorzugt ist ebenfalls, dass einer der universellen Primer als Primer-Kompetitor ausgelegt ist und die Reaktion in der zweiten Phase wie oben für die Verwendung eines Primer-Kompetitors beschrieben durchgeführt wird.

Eine spezielle Ausführungsform für asymmetrische Multiplex-Reaktionen besteht darin, dass die in der ersten Phase verwendeten spezifischen Primer eine höhere Schmelztemperatur als die in der zweiten Phase der Reaktion verwendeten universellen Primer und der Kompetitor aufweisen. Unter dieser Voraussetzung können alle für die zweistufige Multiplex-PCR und die asymmetrische Amplifikation notwendigen Komponenten am Anfang der Reaktion zugegeben werden. Die Zweistufigkeit der Reaktion wird in diesem Fall durch ein entsprechendes Temperaturregime erreicht. Hohe Annealingtemperaturen in der ersten Phase gewährleisten eine symmetrische Amplifikation unter Verwendung der spezifischen Primer, verringerte Annealingtemperaturen in der zweiten Phase führen zur Verwendung der universellen Primer und zur asymmetrischen Amplifikation. Diese Ausführungsform ist besonders bevorzugt, wenn die zweistufige Form der Multiplex-PCR in einer geschlossenen Reaktionskammer durchgeführt werden soll, so dass während der Reaktion eine Zugabe weiterer Komponenten nicht möglich ist.

In einer weiteren Ausführungsform wird die asymmetrische Multiplex-PCR in einem kontinuierlichen, bezüglich der Amplifikationsreaktion dreiphasigen Prozess ausgeführt. In diesem Fall weisen spezifischer Primer, universeller Primer und Kompetitor eine in der genannten Reihenfolge sinkende Schmelztemperatur auf. Der Übergang von spezifischer zu symmetrischer universeller und zu asymmetrischer Amplifikation wird jeweils durch eine Erniedrigung der Annealing-Temperatur im PCR-Programm erreicht.

Für die Detektion der Hybridisierungssignale auf dem Sondenarray ist es in den meisten Fällen ein Markierung des Targets notwendig, d.h. das Target muss mit einem Farbstoff, einer Ankergruppe oder einer anderweitig detektierbaren Einheit versehen werden.

Im Rahmen der vorliegenden Erfindung erfolgt die Markierung des Targets (der PCR-Produkte) vorzugsweise durch den Einbau markierter Primer. Alternativ können jedoch auch markierte Monomere Verwendung finden.

Besonders bevorzugt erfolgt die Markierung dadurch, dass der nicht mit dem Kompetitor konkurrierende Primer der Reaktion als markierte Komponente zugesetzt wird.

Die Markierung basiert vorzugsweise auf der Verwendung von Fluorophormarkierten Primern bzw. Monomeren. In Verbindung mit Fluoreszenzdetektion erlaubt das Verfahren die Markierung mit beliebigen an Nukleotidtriphosphate oder an Oligonukleotide während oder nach deren Synthese koppelbaren Farbstoffen, beispielsweise Cy-Farbstoffe wie Cy3 oder Cy5 (Amersham Pharmacia Biotech, Uppsala, Schweden), Alexa-Farbstoffe wie Texas-Rot, Fluorescein, Rhodamin (Molecular Probes, Eugene, Oregon, USA) oder Lanthanide wie Samarium, Ytterbium und Europium (EG&G Wallac, Freiburg, Deutschland). Bevorzugt werden Fluoreszenz-markierte Primer verwendet. Die Markierung erfolgt besonders bevorzugt mit Cy-Farbstoffen.

Bei einer alternativen Ausführungsform des erfindungsgemäßen Verfahrens basiert die Detektion auf der Verwendung von Primern bzw. Monomeren, die mit Ankergruppen gekoppelt sind. Bevorzugt verwendete Ankergruppen sind Biotin, Digoxigenin und dergleichen. Die Ankergruppen werden in einer anschließenden Reaktion mit spezifisch bindenden Komponenten, beispielsweise Streptavidin-Konjugaten oder Antikörper-Konjugaten umgesetzt, die selbst detektierbar sind, oder eine detektierbare Reaktion auslösen. Bei Einsatz von Ankergruppen erfolgt die Umsetzung der Ankergruppen in detektierbare Einheiten im Anschluss an die Hybridisierung.

Bei einer weiteren alternativen Ausführungsform des erfindungsgemäßen Verfahrens werden Nachweisverfahren verwendet, die im Ergebnis ein Addukt mit bestimmtem Löslichkeitsprodukt, das eine Präzipitation zur Folge hat, liefern. D.h. zur Markierung werden Substrate eingesetzt, die in ein schwerlösliches, üblicherweise gefärbtes Produkt umgesetzt werden können. Beispielsweise können bei dieser Markierungsreaktion Enzyme verwendet werden, die den Umsatz eines Substrats in ein schwerlösliches Produkt katalysieren. Beispiele für solche Reaktionen sind der Umsatz von chromogenen Substraten wie Tetramethylbenzidin (TMB)-haltigen, präzipitierenden Reagenzien durch Peroxidasen (z.B. Meerettichperoxidase, HRP) bzw. der Umsatz von BCIP/NBT- Gemischen durch Phosphatasen. Beispiele für sehr sensitive Nachweisverfahren, bei denen ein schwerlösliches Produkt gebildet wird, sind das Tyramid Signal Amplifikations-System (TSA) und die Enzyme-Labeled Fluorescence (ELF)-Signal-Amplifikation (siehe R. Haugland, Molecular Probes, Handbook of Fluorescent Probes and Research Products, 9. Auflage, 152-166).

Bei einer weiteren alternativen Ausführungsform des erfindungsgemäßen Verfahrens werden als Marker Detektionskügelchen, insbesondere aus Gold, eingesetzt. Das Signal bei der Detektion kann vorzugsweise durch Silberabscheidung an den Detektionskügelchen verstärkt werden. So kann das vorstehend genannte Nachweisverfahren mittels schwerlöslicher Produkte basierend auf Metallmarkierungen durchgeführt werden. Dabei werden beispielsweise kolloidales Gold oder definierte Goldcluster direkt an den nicht mit dem Kompetitor konkurrierenden Primer gekoppelt oder über bestimmte Vermittlermoleküle wie Streptavidin oder Antikörper an den Primer gebunden, der dann über eine entsprechende Ankergruppe für diese Vermittlermoleküle verfügen muss. Ebenfalls möglich ist das Einführen der Goldmarkierung durch Sandwich-Hybridisierung mit dem Target. In allen Fällen findet anschließend eine Verstärkung der Markierung durch Reaktion mit unedleren Metallen, wie z.B. Silber statt. Der Nachweis der durch die spezifische Reaktion verstärkten Bereiche erfolgt mittels eines sehr einfachen optischen Aufbaus im Durchlicht (Kontrast durch Abschattung) bzw. Auflicht (Kontrast durch Reflexion).

Eine weitere alternative Ausführungsform bei der Detektion basiert auf der Verwendung von radioaktiv markierten Strukturen oder Bausteinen wie ³²P oder ³H.

In bestimmten Ausführungsformen kann auf eine Markierung des Targets vollständig verzichtet werden, wenn das Auslesen der Hybridisierungssignale auf einem markierungsfreien Detektionsverfahren wie beispielweise Massenspektroskopie oder Oberflächen-Plasmon-Resonanz beruht.

In einer weiteren Ausführungsform wird das Verfahren mit einem in der Deutschen Patentanmeldung 101 42 643.7-44 beschriebenen Sondenarray durchgeführt. Auch in diesem Fall kann die Markierung des Targets entfallen.

Die Durchführung des bevorzugten erfindungsgemäßen Verfahrens, bei dem die nachzuweisende Nukleinsäure zunächst durch eine PCR amplifiziert wird, wobei durch anfängliche Zugabe eines Kompetitors in windestens äquimolen meyen zu einem in der PCR verwendetel Primes die Amplifikation eines der beiden Template-Stränge inhibiert wird, und der Nachweis der amplifizierten einzelsträngigen Nukleinsäure durch Hybridisierung gegen eine Sonde mit entsprechend komplementärer Sequenz erfolgt, bringt viele Vorteile mit sich. Da in der PCR-Reaktion der nachzuweisende Strang im Überschuss produziert wird, liegt der Strang im zur Hybridisierung verwendeten Reaktionsgemisch automatisch bevorzugt in einzelsträngiger Form vor. Dadurch wird die Konkurrenz zwischen der Hybridisierung mit dem komplementären Strang in der Lösung und der Hybridisierung mit der komplementären Sonde auf dem Microarray im wesentlichen beseitigt, die bei Verwendung doppelsträngiger DNA, als thyldiscungstarget wie sie durch eine PCR unter üblichen nicht-erfindungsgemäßen Bedingungen produziert wird, auftritt. Die Effizienz der Hybridisierung mit der Sonde auf dem Microarray wird bei dem erfindungsgemäßen Verfahren erhöht.

Ein weiterer Vorteil bei dem erfindungsgemäßen Verfahren liegt darin, dass der Einzelstrangüberschuss im gesamten Verlauf der Amplifikation vorliegt, ohne dass der Experimentator durch Eingreifen in das Reaktionssystem wie durch Zugabe von Komponenten in einer fortgeschrittenen Phase der Amplifikation diesen Einzelstrangüberschuss erst erzeugen muss. Dieses Eingreifen des Experimentators ist, wie oben erwähnt, charakteristisch für die klassische lineare oder asymmetrische PCR. Da ein solches Eingreifen beim erfindungsgemäßen Verfahren nicht notwendig ist, hat dieses den Vorteil, sehr einfach und auch in geschlossenen Reaktionsgefäßen durchführbar zu sein, die nach dem Start der Reaktion nicht mehr von Außen zugänglich sind.

Ein weiterer Vorteil des erfindungsgemäßen Verfahrens liegt darin, dass die Amplifikation der Targetsequenzen und die Hybridisierung der Amplifikationsprodukte in ein und demselben Puffersystem ohne Zugabe weiterer Komponenten oder Wechsel der Wasch- bzw. Hybridisierungspuffer durchgeführt werden kann. Dadurch kann z.B. bei Microarray-basierten Nachweisverfahren der gesamte Assay ohne die Notwendigkeit eines zwischenzeitlichen Eingreifens des Experimentators durchgeführt werden.

Noch ein Vorteil des erfindungsgemäßen Verfahrens ist, dass die einzelsträngigen Target-Moleküle trotz Kompetitor in für die Hybridisierung ausreichenden Mengen amplifiziert werden.

Gegenstand der vorliegenden Erfindung ist ebenfalls ein Verfahren zum effizienten Nachweis von Nukleinsäuren durch Hybridisierung, wobei die Hybridisierung der Sonde und des Targets, die jeweils über komplementäre Sequenzbereiche verfügen, in Gegenwart eines Moleküls durchgeführt wird, das in der Nähe des Sequenzbereichs des Targets, der mit der Sonde hybridisiert, an das Target binden kann, wobei eine Prä-Inkubation des Targets mit dem Molekül nicht notwendig ist.

Es ist jetzt überraschend festgestellt worden, dass durch anfängliche Zugabe von Molekülen zu Hybridisieruags-Assays, bei denen eine einzelsträngige NukleinsäureSonde an ein einzelsträngiges Nukleinsäure-Target bindet, wobei sowohl die Sonde als auch das Target über komplementäre Sequenzbereiche verfügen und das Molekül in der Nähe des Sequenzbereichs des Targets, der mit der Sonde hybridisiert, an das Target bindet, die Effizienz der Hybridisierung zwischen Sonde und Target wesentlich erhöht und der Nachweis somit verbessert werden kann.

Überraschenderweise ist dabei eine Vorinkubation des Targets mit dem genannten Molekül vor der eigentlichen Hybridisierung nicht nötig. Damit ist ein Eingreifen des Experimentators während des Hybridisierungsverfahrens nicht nötig.

Ohne an eine wissenschaftliche Theorie gebunden sein zu wollen, wird vermutet, dass diese Erhöhung der Effizienz der Hybridisierung darauf zurückzuführen ist, dass die Bindung des Moleküls an einen Bereich des Targets, der in Nähe des Sequenzbereichs liegt, der durch die Hybridisierung mit der Sonde nachgewiesen wird, die Ausbildung von Sekundärstrukturen in der Target-Sequenz verhindert.

Erfindungsgemäß kann es sich bei solchen Molekülen um Proteine, um Peptide, um Nukleinsäuren oder deren Analoga, um Interkalatoren und andere chemische Verbindungen handeln, die spezifisch in der Nähe des Bereichs des Targets binden, der durch die Hybridisierung mit der Sonde nachgewiesen wird. Im Folgenden werden die Moleküle, die die erfindungsgemäße Wirkung haben, auch als Sekundärstrukturbrecher bezeichnet.

Als Sekundärstrukturbrecher werden erfindungsgemäß bevorzugt Proteine bzw. Peptide eingesetzt, die in der Lage sind, einzelsträngige Nukleinsäuren mit Sequenz-Spezifität zu binden und über die oben definierten Eigenschaften verfügen. Besonders bevorzugt werden erfindungsgemäß als Sekundärstrukturbrecher Nukleinsäuremoleküle und Nukleinsäure-Analoga-Moleküle eingesetzt.

Bei den Nukleinsäure-Analoga kann es sich z.B. um die bereits erwähnten PNAs, um Phosphothioat-modifizierte Nukleinsäuren oder anderweitig hinsichtlich ihrer Basen oder ihres Zuckerrückgrats modifizierte Nukleinsäuren handeln. Diese sind bereits oben im Zusammenhang mit den PCR-Kompetitoren aufgeführt worden. Generell müssen die genannten Nukleinsäure-Analoga sich dadurch auszeichnen, dass sie spezifisch in der Nähe des Bereichs des Targets, der durch Hybridisierung mit der Sonde nachgewiesen wird, an das Target binden können.

In einer besonders bevorzugten Ausführung der Erfindung werden als Sekundärstrukturbrecher Nukleinsäuremoleküle eingesetzt, bei denen es sich um DNA- oder RNA-Moleküle handelt. Erfindungsgemäß müssen solche Nukleinsäure-Sekundärstrukturbrecher über Sequenzen verfügen, die es ihnen erlauben, in Nähe des Targetbereichs, der durch Hybridisierung mit der Sonde nachgewiesen wird, spezifisch zu hybridisieren.

Wenn es sich bei den erfindungsgemäßen Sekundärstrukturbrechern um Nukleinsäuren oder Nukleinsäure-Analoga handelt, umfassen diese erfindungsgemäß zwischen 5 und 100 Basen, bevorzugt zwischen 10 und 50 Basen, besonders bevorzugt zwischen 10 und 30 Basen und am meisten bevorzugt zwischen 15 und 25 Basen.

Überraschenderweise ist im Rahmen der vorliegenden Erfindung festgestellt worden, dass, wenn es sich bei den erfindungsgemäßen Sekundärstrukturbrechern um Nukleinsäuren bzw. Nukleinsäure-Analoga handelt, diese nicht in unmittelbarer Nachbarschaft zu der Sonde mit dem Target hybridisieren müssen, d.h. kein Abstand aber auch keine Überlappung zwischen dem hybridisierten Sekundärstrukturbrecher und der Sonde vorhanden sein darf, wie es im Stand der Technik gefordert wird, sondern sie lediglich in der Nähe des Bereichs der Targetsequenz, der durch die Hybridisierung mit der Sonde nachgewiesen wird, an das Target binden müssen.

Erfindungsgemäß bedeutet "in der Nähe", dass, wenn als Sekundärstrukturbrecher Nukleinsäuren bzw. deren Analoga verwendet werden, zwischen dem mit dem Target hybridisierten Sekundärstrukturbrecher und der mit dem Target hybridisierten Sonde mindestens 20, bevorzugt mindestens 15, ebenfalls bevorzugt mindestens 10, besonders bevorzugt mindestens 8, ebenfalls besonders bevorzugt mindestens 5, insbesondere bevorzugt mindestens 4, ebenfalls insbesondere bevorzugt mindestens 3 und am meisten bevorzugt mindestens 2 Nukleotide oder 1 Nukleotid Abstand vorhanden sein können.

Werden als Sekundärstrukturbrecher Proteine, Peptide, Aptamere etc. oder Nukleinsluremoleküle bzw. Moleküle von Nukleinsäure-Analoga, die enzymatisch nicht verlängerbar sind, eingesetzt, können diese auch in unmittelbarer Nachbarschaft der Target-Sequenz, die durch die Sonde nachgewiesen wird, d.h. ohne Abstand davon, aber auch ohne Überlappung damit, an das Target-Molekül binden.

Wenn als erfindungsgemäße Sekundärstrukturbrecher Nukleinsäuren bzw. deren Analoga eingesetzt werden, können diese eine Sequenz aufweisen oder chemisch derart modifiziert sein, dass sie enzymatisch z.B. in einer PCR-Reaktion nicht verlängerbar sind, müssen es aber nicht

Die Frage, ob sie eine entsprechende enzymatisch nicht verlängerbare Sequenz aufweisen oder entsprechend chemisch modifiziert sind, richtet sich danach, ob sie erst zur Hybridisierung eingesetzt werden, oder wie bei dem oben dargestellten Verfahren, bereits zu Anfang der Amplifikation des Targets einer PCR-Reaktion zugesetzt werden.

Wenn sie bereits zu Anfang der PCR-Reaktion zugesetzt werden, müssen die Sekundärstrukturbrecher eine Sequenz aufweisen oder derart chemisch modifiziert sein, dass sie enzymatisch nicht durch eine DNA- oder RNA-Polymerase verlängert werden können. Die Anforderungen hinsichtlich der Sequenz bzw. der chemischen Modifikation der Sekundärstrukturbrecher-Nukleinsäuremoleküle ist dabei bereits oben im Zusammenhang mit den Nukleinsäure-Kompetitoren dargestellt worden und kann auf die Sekundärstrukturbrecher übertragen werden, wenn es sich um Nukleinsäuren oder deren Analoga handelt. Allerdings ist sich der Fachmann bewusst, dass Sekundärstrukturbrecher hinsichtlich ihrer Länge größer sein können als die zur erfindungsgemäßen asymmetrischen PCR verwendeten Kompetitoren. Wenn enzymatisch nicht-verlängerbare Sekundärstrukturbrecher bereits zu Beginn einer PCR zugesetzt werden, muss der PCR-Reaktion nicht zusätzlich ein erfindungsgemäßer Kompetitor zugesetzt werden, der die Amplifizierung eines der beiden Template-Stränge inhibiert. In diesem Fall wird bereits durch die Zugabe des Sekundärstrukturbrechers zur PCR-Reaktion eine Steigerung der Hybridisierungs-signale erreicht, d.h. in einer bevorzugten Ausführungsform der Erfindung kann der Sekundärstrukturbrecher zur Steigerung von Hybridisienmgssignalen auch einer symmetrichen PCR zugesetzt werden.

Erfindungsgemäß können die Nukleinsäure-Sekundästrukturbrecher zusätzlich zu den Nukleinsäure-Kompetitoren, die z.B., wie oben erwähnt, mit einem der Primer um die Bindung an das Template konkurrieren können, der PCR-Reaktion zugesetzt werden. Diese Ausführungsform ist erfindungsgemäß besonders bevorzugt, da auf diese Weise in der PCR ein Einzelstrangüberschuss erzielt wird und in der sich anschließenden Hybridisierung die Effizienz der Hybridisierung zwischen Sonde und Target durch das Vorhandensein des Sekundärstrukturbrechers erhöht wird, ohne dass der Experimentator im Laufe des gesamten Verfahrens eingreifen muss. Auf diese Ausführungsform des Verfahrens treffen die bereits oben dargestellten Vorteile gegenüber dem Stand der Technik in vollem Umfang zu.

Die Sekundärstrukturbrecher-Nukleinsäuren bzw. deren Analoga können aber auch alleine der PCR-Reaktion zugesetzt werden. Sie fungieren dann zum einen während der PCR-Amplifikation als Nukleinsäure-Kompetitoren und bewirken eine Amplifikation nur eines Template-Strangs im Überschuss, auf der anderen Seite wirken sie während der Hybridisierung als Sekundärstrukturbrecher und erhöhen entsprechend die Effizienz der Hybridisierung. Bei dieser ebenfalls bevorzugten Ausführungsform der Erfindung muss der Experimentator wiederum nicht eingreifen. Da PCR-Kompetitor und Hybridisierungs-Sekundärstrukturbrecher in diesem Fall identisch sind, kann das Verfahren sehr kosteneffizient durchgeführt werden.

Wie erwähnt, wirkt bei bevorzugten Ausführungsformen der Erfindung ein und dasselbe Molekül als Kompetitor und Strukturbrecher. Dies ist insbesondere dann der Fall, wenn das Template so kurz ist, dass die Target-Sequenz und die durch den Primer adressierte Sequenz in mittelbarer oder unmittelbarer Nachbarschaft liegen oder wenn die asymmetrische Amplifikation durch Bindung des Kompetitors an einen von der Primer-Bindungsstelle verschiedenen Bereich des Templates erreicht wird.

Handelt es sich bei den Sekundärstrukturbrechern z.B. um hitzestabile Proteine, Peptide, Aptamere oder sonstigen Liganden mit der oben erwähnten Spezifität bezüglich der Bindung an das Target, können diese selbstverständlich auch zu Anfang der PCR-Amplifikation im Nachweisverfahren eingesetzt werden. Diese Ausführung des erfindungsgemäßen Verfahrens weist dann wiederum die genannten Vorteile auf.

Wenn als Sekundärstrukturbrecher Nukleinsäuren bzw. Nukleinsäure-Analoga nach der PCR-Amplifikation, d.h. erst zur Hybridisierung dem Reaktionsgemisch beigefügt werden, müssen diese nicht eine Sequenz aufweisen bzw. derart chemisch modifiziert sein, dass sie enzymatisch nicht verlängerbar sind. Bei dieser erfindungsgemäßen Ausführungsform ist zwar ein weiterer Arbeitsschritt, nämlich das Hinzufügen der Sekundärstrukturbrecher zur Reaktionslösung notwendig, dennoch ist diese Ausführungsform der Erfindung vorteilhaft, da hiermit die Effizienz die Hybridisierung des in der PCR-produzierten einzelsträngigen Targets an die Sonde wesentlich gesteigert werden kann, da sich keine Sekundärstrukturen im Target ausbilden.

Den erfindungsgemäßen Sekundärstrukturbrechern ist gemeinsam, dass sie zu Anfang der Hybridisierung zusammen mit Targets und Sonden zugesetzt werden können. Eine generelle Prä-Inkubation der Sekundärstrukturbrecher mit dem Target, also z.B. ein prä-annealing der entsprechenden Sekundärstrukturbrecher-DNA-Oligonukleotide mit den Target-DNA-Molekülen ist erfindungsgemäß nicht notwendig.

In Experimenten wurde nämlich überraschend festgestellt, dass Sekundärstrukturbrecher, bei denen es sich um Oligonukleotide bzw. Nukleinsäuren handelt, auch in einem wie oben beschriebenen kontinuierlichen PCR-Hybridisierungs-Assay, bei dem zusätzlich als Kompetitoren Nukleinsäuren eingesetzt wurden, die mit einem der Primer um die Bindung an das Template kompetieren, eine zusätzliche Verstärkung des Hybridisierungs-Signals problematischer Targets bewirken, wenn sie am Anfang der Reaktion dem Reaktionsgemisch zugesetzt werden. In diesem Fall dürfen die Sekundärstrukturbrecher nicht enzymatisch verlängerbar sein.

Bei den Strukturbrechern handelt es sich bevorzugt um Nukleinsäuremoleküle oder Nukleinsäure-Analoga, besonders bevorzugt um DNA- oder PNA-Moleküle.

In einer besonders bevorzugten Ausführungsform werden die Strukturbrecher vor der PCR-Reaktion dem Reaktionsgemisch zugesetzt. Handelt es sich bei den Shukturbrechern um RNA- oder DNA-Moleküle, wird deren Struktur oder chemische Zusammensetzung so ausgelegt, dass diese nicht von der Polymerase durch DNA-Synthese verlängert werden können. Bevorzugt ist das 3'-Ende eines Nukleinsäure Strukturbrechers so ausgelegt, dass es keine Komplementarität zum Template aufweist und/oder anstelle der 3-OH-Gruppe am 3'- Ende einen anderen Substituenten trägt. Bei diesem anderen Substituenten handelt es sich bevorzugt um eine Phosphat-Gruppe, ein Wasserstoff-Atom (so dass ein Dideoxynukleotid entsteht), Biotin oder eine Aminogruppe, letztere gegebenenfalls verknüpft mit einem in der chemischen DNA-Synthese üblichen Linkerbaustein. Bezüglich der Komplementarität des 3'-Endes wird auf die Nukleinsäure-Kompetitoren verwiesen, da die Erfordernisse identisch sind. In einer bevorzugten Ausführungsform sind Sekundärstrukturbrecher enzymatisch nicht verlängerbar und können eine 100%-ige Komplementarität zu ihrer Zielsequenz aufweisen.

Ebenfalls bevorzugt ist, wenn die Strukturbrecher dem Reaktionsgemisch erst nach erfolgter Amplifikation, direkt vor dem Hybridisierungsschritt zugesetzt werden. Diese Ausführungsform ist besonders bevorzugt, wenn die Reaktion nicht in einer abgeschlossenen Reaktionskammer durchgeführt wird, bei der eine Zugabe weiterer Komponenten im Reaktionsverlauf nicht möglich wäre.

Die optimalen molaren Verhältnisse von Strukturbrecher zu Target am Ende der Reaktion liegen üblicherweise zwischen 1 bis 100.000, bevorzugt zwischen 2 bis 1.000, besonders bevorzugt zwischen 5 bis 500 und am meisten bevorzugt zwischen 5 bis 100.

Dem Fachmann ist bewusst, dass die verschiedenen Aspekte der Erfindung auch miteinander kombiniert werden können, um eine effiziente Amplifikation von einzelsträngigen Nukleinsäuremolekülen zu erzielen und diese effizient durch Hybridisierung in einem kontinuierlichen Prozess nachzuweisen.

In einer weiteren Ausführungsform des erfindungsgemäßen Verfahrens kann eine parallele Hybridisierungs-basierte Echtzeit-Quantifizierung von PCR-Targets erreicht werden. In diesem Fall wird eine asymmetrische PCR nach einer der oben beschriebenen Ausführungsformen durchgeführt. Im Unterschied zu den bisher beschriebenen Ausführungsformen wird der kontinuierliche Amplifikations- und Hybridisierungsprozess jedoch zyklisch durchgeführt. D.h. nach jedem PCR-Zyklus oder jeweils einer bestimmten Anzahl von PCR-Zyklen wird eine Hybridisierung durchgeführt. Die an den einzelnen Sonden gemessenen Hybridisierungssignale werden nach jedem Hybridisierungsschritt quantifiziert. Diese Signale stellen ein Maß für die Konzentration des Targets in der Reaktionslösung zum Zeitpunkt der Hybridisierungsreaktion dar.

Durch die Vielzahl der Sonden auf dem Sondenarray kann eine Vielzahl von Targets gleichzeitig quantifiziert werden. Die Amplifikationskinetik der einzelnen Targets kann verfolgt werden, indem die Zunahme der an jedem Spot gemessenen Hybridisierungssignale mit steigender Zyklenzahl verfolgt wird.

Die zyklische kontinuierliche PCR und Hybridisierungsreaktion kann naturgemäß unter Verwendung von Strukturbrechermolekülen nach einem der vorangegangenen Ausführungsbeispiele oder ohne deren Zugabe durchgeführt werden.

Zusammengenommen eröffnen diese Verfahren die Möglichkeit Microarray-basierte Analysen schneller durchzuführen. Analysen werden auf wenige Schritte reduziert, wodurch die Fehleranfälligkeit sinkt. Analysen, die eine Amplifikation und Hybridisierung von Targets umfassen, können nun vollständig in geschlossenen Systemen wie beispielsweise dem u.a. in der internationalen Patentanmeldung WO 01/02094 beschriebenen Array-Prozessor von Clondiag durchgeführt werden. Dadurch wird eine wesentliche Voraussetzung geschaffen, Array-basierte Analysen von Nukleinsäuren mit kleinen, handlichen, gegebenenfalls mobilen Systemen durchzuführen, so dass erstmals eine Basis für Chip-basierte "point of care"-Diagnostik geschaffen wird. Die Arbeit in geschlossenen Systemen vermindert außerdem die Kontaminationsgefahr mit gegebenenfalls infektiösen Probenmaterial, sowie die Übertragung von Amplifikationsprodukten in andere Reaktionen.

Im Folgenden wird die Erfindung an konkreten Beispielen erläutert. Diese sind nicht einschränkend zu deuten.

### Ausführungsbeispiele:

### Ausführungsbeispiel 1:

Vergleichende Hybridisierung von durch symmetrische PCR und durch erfindungsgemäße asymmtrische PCR mit abgestuften Kompetitor-Anteilen erzeugten PCR-Produkten gegen immobilisierte Sonden.

### Ampifikationskinetik der asymmetrischen PCR:

In einem initialen Experiment wurde zunächst untersucht, in welchem Ausmaß die Produktbildung bei der PCR vom Kompetitor-Anteil abhängt. Dazu wurden zunächst PCR-Reaktionen mit identischen Primer-Konzentrationen, aber unterschiedlichem Kompetitor-Anteil durchgeführt.

Bei dem Kompetitor handelte es sich um ein DNA-Oligonukleotid, das die gleiche Sequenz wie der reverse-Primer der PCR aufwies, an der 3'-OH-Gruppe am 3'-Ende jedoch mit einer NH₂-Gruppe modifiziert war. Die Amino-Modifaktion wurde während der chemischen Synthese des Oligonukleotides in das Molekül integriert (3'-Amino-Modifier C7, Glen Research Corp., Sterling, VA, USA).

### PCR-Reaktionen:

Der forward-Primer 16sfD1Cy3 wies folgende Sequenz auf und war am 5'-Ende mit Cy3 markiert.
5'-AGAGTTTGATCCTGGCTCAG-3'

Der reverse-Primer wies folgende Sequenz auf:
5'-TACCGTCACCATAAGGCTTCGTCCCTA-3'

Es wurden PCR-Ansätze mit unterschiedlichen Kompetitor-Anteilen hergestellt. Dabei wies jeder PCR-Ansatz folgende Zusammensetzung und Endkonzentrationen auf.

| | |
|---|---|
| 1x | PCR-Reaktionspuffer (Eppendorf Hamburg, Deutschland) |
| 200 nM | forward-Primer 16sfD1Cy3, am 5'-Ende mit dem Fluoreszenzfarbstoff Cy3 markiert (Amersham-Pharmacia, Freiburg, Deutschland) |
| 200 µM | dNTPs |
| 0,05 U/µl | Taq-Polymerase (Eppendorf Hamburg, Deutschland) |
| 2 ng/µl | chromosomale DNA *Corynebacterium glutamicum* |

zusätzlich enthielten:

| | | |
|---|---|---|
| Reaktion 1: | 200 nM | reverse Primer 16s Ra (entsprechend 0 % Kompetitor, symmetrische PCR) |
| Reaktion 2: | 0 nM | reverse Primer 16s Ra |
| | 200 nM | Kompetitor 16s Ra 3' NH2 (entsprechend 100 % Kompetitor, asymmetrische PCR) |
| | | |
| Reaktion 3: | 10 nM | reverse Primer 16s Ra |
| | 190 nM | Kompetitor 16s Ra 3' NH2 (entsprechend 95 % Kompetitor, asymmetrische PCR) |
| | | |
| Reaktion 4: | 20 nM | reverse Primer 16s Ra |
| | 180 nM | Kompetitor 16s Ra 3' NH2 (entsprechend 90 % Kompetitor, asymmetrische PCR) |
| | | |
| Reaktion 5: | 40 nM | reverse Primer 16s Ra |
| | 160 nM | Kompetitor 16s Ra 3' NH2 (entsprechend 80 % Kompetitor, asymmetrische PCR) |
| | | |
| Reaktion 6: | 60 nM | reverse Primer 16s Ra |
| | 140 nM | Kompetitor 16s Ra 3' NH2 (entsprechend 70 % Kompetitor, asymmetrische PCR) |
| | | |
| Reaktion 7: | 100 nM | reverse Primer 16s Ra |
| | 100 nM | Kompetitor 16s Ra 3' NH2 (entsprechend 50 % Kompetitor, asymmetrische PCR) |
| | | |
| Reaktion 8: | 140 nM | reverse Primer 16s Ra |
| | 60 nM | Kompetitor 16s Ra 3' NH2 (entsprechend 30 % Kompetitor, asymmetrische PCR) |

Das Gesamtvolumen jedes PCR-Ansatzes betrug 25 µl. Die Reaktionen wurden nach folgendem Temperaturregime durchgeführt:

| | | |
|---|---|---|
| Initiale Denaturierung: | 2 min | 95° C |
| | | |
| 25 Zyklen: | 30 sec | 95° C |
| | 30 sec | 60° C |
| | 30 sec | 72° C |
| | | |
| Terminale Elongation: | 7 min | 72° C |

Die Reaktionsansätze wurden in LightCycler-Küvetten (Roche Diagnostics, Mannheim, Deutschland) gefüllt und die PCR-Reaktion im Light Cycler (Roche Diagnostics, Mannheim, Deutschland) durchgeführt. Die Produktbildungskinetik wurde nach den Herstellerangaben aufgenommen.

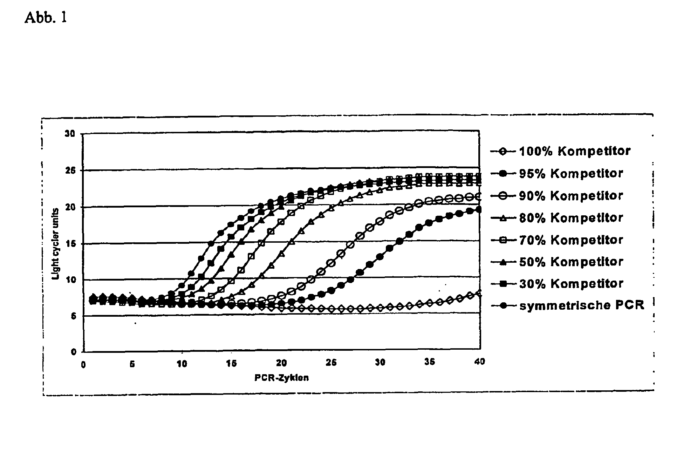

Die Ergebnisse sind in Abbildung 1 (Abb. 1) dargestellt. Die Menge an gebildetem PCR-Produkt ist dabei als Light cycler units in Abhängigkeit von der Zyklenzahl angegeben.

Es ist deutlich zu erkennen, dass mit steigendem Kompetitor-Anteil die Effizienz der PCR und entsprechend die Produkt-Ausbeute sinkt.

### Vergleich der Hybridisierungssignale in Abhängigkeit vom Kompetitor-Anteil:

Im Folgenden wurde die Produktbildung und das Hybridisierungssignal bei symmetrischer und asymmetrischer PCR-Amplifikation mit unterschiedlichen Kompetitor-Anteilen zu einem bestimmten Zeitpunkt (Abschluss der Reaktion nach 25 Zyklen) verglichen.

Es wurden die gleichen Primer bzw. Kompetitoren wie oben angegeben eingesetzt.

### PCR-Reaktionen:

Es wurden PCR-Ansätze mit unterschiedlichen Kompetitor-Anteilen hergestellt Dabei wies jeder PCR-Ansatz folgende Zusammensetzung und Endkonzentrationen auf.

| | |
|---|---|
| 1x | PCR-Reaktionspuffer (Eppendorf Hamburg, Deutschland) |
| 200 nM | forward-Primer 16sfD1Cy3, am 5'-Ende mit dem Fluoreszenzfarbstoff Cy3 markiert (Amersham-Pharmacia, Freiburg, Deutschland) 16sfD1Cy3 |
| 200 µM | dNTPs |
| 0,05 U/µl | Taq-Polymerase (Eppendorf Hamburg, Deutschland) |
| 2 ng/µl | chromosomale DNA *Corynebacterium glutamicum* |

zusätzlich enthielten:

| | | |
|---|---|---|
| Reaktion 1: | 200 nM | reverse Primer 16s Ra (entsprechend 0% Kompetitor, symmetrische PCR) |
| | | |
| Reaktion 2: | 80 nM | reverse Primer 16s Ra |
| | 120 nM | Kompetitor 16s Ra 3' NH2 (entsprechend 60% Kompetitor, asymmetrische PCR) |
| | | |
| Reaktion 3: | 40 nM | reverse Primer 16s Ra |
| | 160 nM | Kompetitor 16s Ra 3' NH2 (entsprechend 80% Kompetitor, asymmetrische PCR) |
| | | |
| Reaktion 4: | 20 nM | reverse Primer 16s Ra |
| | 180 nM | Kompetitor 16s Ra 3' NH2 (entsprechend 90% Kompetitor, asymmetrische PCR) |

Die Reaktionen wurden nach dem gleichen Temperaturprotokoll wie oben durchgeführt:

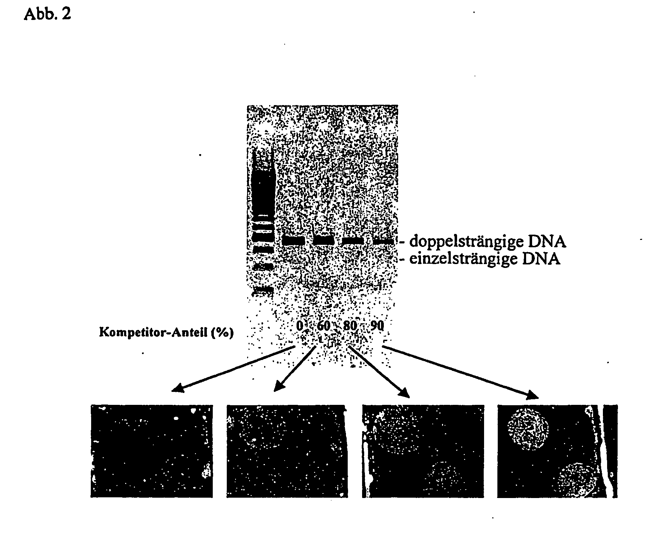

Das Gesamtvolumen jeder PCR betrug 25 µl. Jeweils 5 µl der PCR-Ansätze wurden auf einem 2% Agarosegel analysiert (Abb. 2). Man erkennt, dass mit steigendem Kompetitor-Anteil die Menge an doppelsträngiger DNA ab- und an einzelsträngiger DNA zunimmt.

Anschließend wurden die restlichen 20 µl der PCR-Reaktionen mittels Quiaquick PCR-Purification-Kit (Qiagen, Hilden, Deutschland) nach Herstellerangaben gereinigt. Die Elution der PCR-Fragmente erfolgte jeweils in 50 µl Wasser. Das Eluat wurde im Vakuum auf 10 µl eingeengt.

### Immobilisierung der Hybridisierungssonden:

Auf mit einem Epoxid beschichteten Glasflächen der Größe 3x3 mm ("Chip") wurde an zwei definierten Stellen ("Spot") ein Amino-modifiziertes Oligonukleotid mit einer Länge von 18 Nukleotiden der Sequenz 5'-NH2-GTTTCCCAGGCTTATCCC-3') kovalent immobilisiert.

Dazu wurden 0,1 µl einer 5 µM Lösung des Oligonukleotides in 0,5 M Phosphatpuffer auf der Glasfläche abgelegt und schließlich bei 37° C eingetrocknet. Die kovalente Verknüpfung der abgelegten Oligonukleotide mit den Epoxid-Gruppen auf der Glasoberfläche erfolgte durch 30-minütiges Backen der Chips bei 60° C. Anschließend wurden die Chips kräftig mit destilliertem Wasser gespült und danach 30 min in 100 mM KCl gewaschen. Nach weiterem kurzen Spülen in 100 mM KCl und anschließend destilliertem Wasser wurden die Chips 10 min bei 37° C getrocknet.

### Hybridisierung:

2 µl der gereinigten PCR-Aliquots wurden in 50 µl 6x SSPE, 0,1% SDS aufgenommen (Sambrook et al., *vide supra*). Zu jeder Hybridisierungslösung wurde ein Chip gegeben. Die Reaktion wurde 5 min bei 95° C denaturiert und anschließend für 1 h bei 50° C inkubiert. Im Anschluß erfolgten 3 aufeinander folgende 5-minütige Waschschritte bei 30° C in 2x SSC, 0,1% SDS, bei 30° C in 2x SSC und bei 20° C in 0,2x SSC (Sambrook et al. *vide supra*). Die Chips wurden anschließend im Vakuum getrocknet.

### Detektion der Hybridisierungssignale:

Die Detektion der Hybridisierungssignale erfolgte unter einem Zeiss Fluoreszenzmikroskop (Zeiss, Jena, Deutschland). Die Anregung erfolgte im Auflicht mit einer Weißlichtquelle und einem für Cyanine 3 geeigneten Filtersatz. Die Signale wurden mit einer CCD-Kamera (PCO-Sensicam, Kehlheim, Deutschland) aufgezeichnet. Die Belichtungszeit betrug 2000 ms. (Abb. 2)

### Ergebnisse:

Es zeigte sich, dass die nach dem erfindungsgemäßen Verfahren erzeugten PCR-Produkte trotz deutlich geringerer Produktmenge deutlich erhöhte Hybridisierungssignale zeigen.

Bei Zunahme der Kompetitorkonzentration im Bereich von 0-90% Kompetitoranteil ist eine Zunahme des Hybridisierungssignales zu beobachten, obwohl die Gesamtmenge des Produktes abnimmt. Überraschenderweise wurden die besten Signale beim höchsten Kompetitor-Anteil (90%) und damit der geringsten Menge an einzelsträngiger DNA erzielt.

### Ausführungsbeispiel 2:

Es wurde eine quantitative Analyse des Hybridisierungssignals in Abhängigkeit vom Kompetitor-Anteil durchgeführt. Für die PCR wurden die gleichen forward- und reverse-Primer und der gleiche Kompetitor wie in Beispiel 1 verwendet.

Es wurden PCR-Ansätze mit folgender Zusammensetzung und Endkonzentrationen hergestellt:
alle Reaktionen enthielten:

| | |
|---|---|
| 1 x | PCR-Reaktionspuffer (Eppendorf Hamburg, Deutschland) |
| 200 nM | forward-Primer 16sfD1Cy3, am 5'-Ende mit dem Fluoreszenzfarbstoff Cy3 markiert (Amersham-Pharmacia, Freiburg, Deutschland) |
| 200 µM | dNTPs |
| 0,05 U/µl | Taq-Polymerase (Eppendorf Hamburg, Deutschland) |
| 2 ng/µl | chromosomale DNA *Corynebacterium glutamicum* |

zusätzlich enthielten:

| | | |
|---|---|---|
| Reaktion 1: | 266 nM | reverse-Primer 16s Ra, kein Kompetitor (symmetrische PCR) |
| | | |
| Reaktion 2: | 133 nM | reverse-Primer 16s Ra |
| | 133 nM | Kompetitor 16s Ra 3' NH2 (entsprechend 50% Kompetitor, asymmetrische PCR) |
| | | |
| Reaktion 3: | 40 nM | reverse-Primer 16s Ra |
| | 160nM | Kompetitor 16s Ra 3' NH2 (entsprechend 75% Kompetitor, asymmetrische PCR) |
| | | |
| Reaktion 4: | 33 nM | reverse Primer 16s Ra |
| | 233 nM | Kompetitor 16s Ra 3' NH2 (entsprechend 87,5% Kompetitor, asymmetrische PCR) |

Die Reaktionen wurden jeweils in Aliquots von 25 µl aufgeteilt. Das Temperaturprotokoll war identisch zu Beispiel 1. Jeweils ein Aliquot von Reaktion 1-4 wurde nach 15, 20, 25, 30 bzw. 35 PCR-Zyklen entnommen.

Jeweils 5 µl jedes Aliquots wurden auf einem 2% Agarosegel analysiert. Die Gelanalyse zeigte, dass bei geringer Zyklenzahl bei symmetrischer PCR deutlich mehr PCR-Produkt gebildet wurde als bei der asymmetrischen PCR-Reaktion.

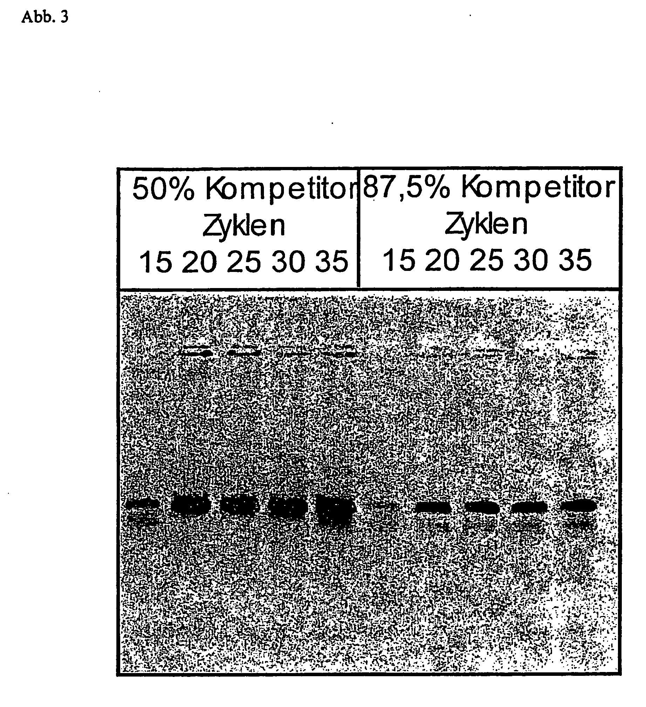

Abbildung 3 (Abb. 3) zeigt ein Agarosegel, auf dem parallel 5 µl-Proben von Reaktionen mit einem Kompetitor-Anteil von 50% bzw. 87,5% analysiert wurden. Die Reaktionen waren jeweils nach der angegebenen Zyklenzahl gestoppt worden. Man erkennt, dass bei 87,5% Kompetitor-Anteil weniger Produkt erzeugt wird.

### Immobilisierung der Hybridisierungssonden:

Auf mit einem Epoxid beschichteten Glasfläche der Größe 3x3 mm ("Chip") wurde an zwei definierten Stellen ("Spot") ein aminomodifiziertes Oligonukleotid mit einer Länge von 18 Nukleotiden der Sequenz 5'-NH₂- GTTTCCCAGGCTTATCCC-3') kovalent immobilisiert.

Dazu wurden 0,1 µl einer 5 µM Lösung des Oligonukleotides in 0,5 M Phosphatpuffer auf der Glasfläche abgelegt und schließlich bei 37° C eingetrocknet. Die kovalente Verknüpfung der abgelegten Oligonukleotide mit den Epoxid-Gruppen auf der Glasoberfläche erfolgte durch 30-minütiges Backen der Chips bei 60° C. Anschließend wurden die Chips kräftig mit destilliertem Wasser gespült und danach 30 min in 100 mM KCl gewaschen. Nach weiterem kurzen Spülen in 100 mM KCl und anschließend destilliertem Wasser wurden die Chips 10 min bei 37° C getrocknet.

### Hybridisierung:

Weitere 5 µl der Reaktion wurden mit 50 µl 6x SSPE, 0,1% SDS gemischt und in Hybridisierungsexperimenten eingesetzt (Sambrook et al. *vide supra*). Zu jeder Hybridisierungslösung wurde ein Chip gegeben. Die Reaktion wurde 5 min bei 95° C denaturiert und anschließend für 1 h bei 50° C inkubiert. Im Anschluß erfolgten 3 aufeinander folgende 5 minütige Waschschritte bei 30° C in 2x SSC, 0,1% SDS, bei 30° C in 2x SSC und bei 20° C in 0,2x SSC. Die Chips wurden anschließend im Vakuum getrocknet.

### Detektion der Hybridisierungssignale:

Die Detektion der Hybridisierungssignale erfolgte unter einem Zeiss Fluoreszenzmikroskop (Zeiss, Jena, Deutschland). Die Anregung erfolgte im Auflicht mit einer Weißlichtquelle und einem für Cyanine 3 geeigneten Filtersatz. Die Signale wurden mit einer CCD-Kamera (PCO-Sensicam, Kehlheim, Deutschland) aufgezeichnet. Die Belichtungszeit betrug 2000 ms.

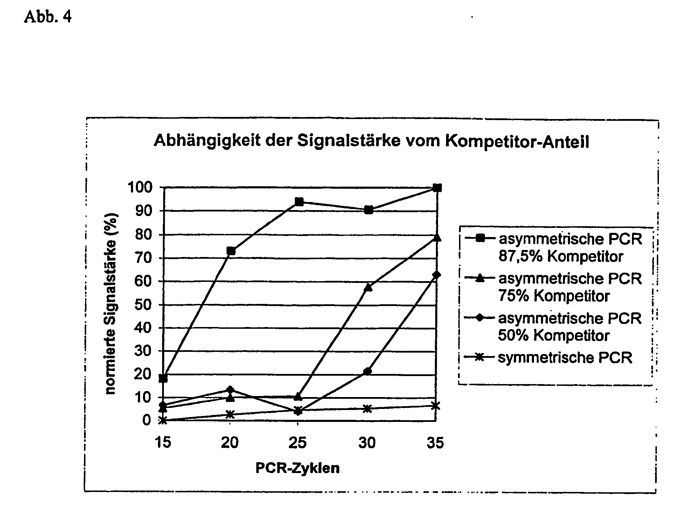

Die Intensität der Hybridisierungssignale wurde gemessen, indem die Signalstärke (gemessene Graustufe) über den gesamten Bereich der Spots gemittelt wurde. Von diesem Wert wurde der über den Spot-freien Bereich (Hintergrund) gemittelte Grauwert abgezogen. Die so errechneten Signalintensitäten wurden normiert (höchster Wert = 100%) und gegen die Zyklenzahl aufgetragen (Abb. 4).

### Ergebnisse:

Abb. 4 zeigt, dass mit PCR-Produkten aus asymmetrischen PCR-Reaktionen über den gesamten untersuchten Bereich von 15 bis 35 Zyklen ein stärkeres Hybridisierungssignal erreicht wird, obwohl die Gelanalyse (Abb. 3) für diese Reaktionen eine geringere Produktmenge insbesondere nach 15 und 20 Zyklen zeigte.

Das stärkste Signal wurde überraschenderweise mit dem höchsten Kompetitor-Anteil erreicht, obwohl hier die geringste Menge an einzelsträngigen Target-Molekülen vorhanden war. Die Hybridisierung von PCR-Produkten, die durch asymmetrische PCR mit unterschiedlichem Kompetitor-Anteil hergestellt wurden, zeigte somit ein bis zu 20-fach höheres Hybridisierungssignal über den gesamten messbaren Bereich der Amplifikation.

### Ausführungsbeispiel 3:

Nachweis der Bildung eines Einzelstrangüberschusses bei asymmetrischer PCR

Es wurden zwei PCR-Ansätze (je 150 µl) folgender Zusammensetzung und Endkonzentrationen hergestellt.

Der forward- und reverse-Primer sowie der Kompetitor hatten die gleichen Sequenzen wie in Beispiel 1. Allerdings war der forward-Primer am 5'-Ende mit dem Fluoreszenzfarbstoff IRD 800 markiert.

Beide Reaktionen enthielten:

| | |
|---|---|
| 1 x | PCR-Reaktionspuffer (Eppendorf Hamburg, Deutschland) |
| 200 nM | forward-Primer 16sfD 1 IRD, am 5' -Ende mit dem Fluoreszenzfarbstoff IRD 800 markiert (MWG-Biotech, Ebersberg, Deutschland) |
| 160 µM | dNTPs |
| 0,1 U/µl | Taq-Polymerase (Eppendorf Hamburg, Deutschland) |
| 2 ng/µl | chromosomale DNA *Corynebacterium glutamicum* ATCC 13032 |

zusätzlich enthielten:

| | | |
|---|---|---|
| Reaktion 1: | 200 nM | reverse Primer 16s Ra, kein Kompetitor (symmetrische PCR) |
| | | |
| Reaktion 2: | 20 nM | reverse Primer 16s Ra |
| | 180 nM | Kompetitor 16s Ra 3' NH2 (entsprechend 90% Kompetitor, asymmetrische PCR) |

Die Reaktionen wurden in 6 Aliquots von 25µl aufgeteilt (1/a 1/f bzw. 2/a - 2/f) und nach folgendem Temperaturprotokoll inkubiert:

| | | |
|---|---|---|
| Initiale Denaturierung | 2 min | 95° C |
| 35 Zyklen | 30 sec | 95° C |
| | 30 sec | 50-70° C |
| | | entspricht für: |
| | | Reaktion a: 50° C |
| | | Reaktion b: 54° C |
| | | Reaktion c: 60 °C |
| | | Reaktion d: 63 °C |
| | | Reaktion e: 67° C |
| | | Reaktion f: 70° C |
| | 30 sec | 72° C |
| | | |
| Terminale Extension | 7 min | 72° C |

Dabei waren die annealing-Temperaturen für die Reaktionen e und f so gewählt, dass ein Annealing des Kompetitors bzw. des reverse-Primers nicht stattfinden sollte, da sie über der Schmelztemperatur dieser Oligonukleotide lager.

Jeweils 2 µl einer 1:10-Verdünnung jedes Aliquots wurden auf einem 3,5%-igen nativen Polyacrylamidgel in einem LICOR-*Sequencer (Typ, Firma)* analysiert. Das Gel hatte eine Stärke von 1 mm. Die Elektrophorese erfolgte bei Begrenzung der Spannung auf 200 V, und der Leistung auf 5 W. Es erfolgte weder eine aktive Erwärmung noch eine aktive Kühlung des Gels (Die Regelgröße für die Temperatur wurde auf 15° C gesetzt).

Die Datenaufnahme erfolgte mit einer Tiefe von 16 Bit, Scan Speed 1 und Signal Filter 3.

Während der PCR-Amplifikation wird nur einer der beiden Stränge mit dem IRD-Farbstoff markiert. Nur dieser ist bei der Gelanalyse im LICOR-Sequenzer sichtbar. Da ein natives Gel verwendet wurde, werden die PCR-Produkte nicht nur nach Länge, sondern auch nach strukturellen Eigenschaften getrennt.

### Ergebnisse:

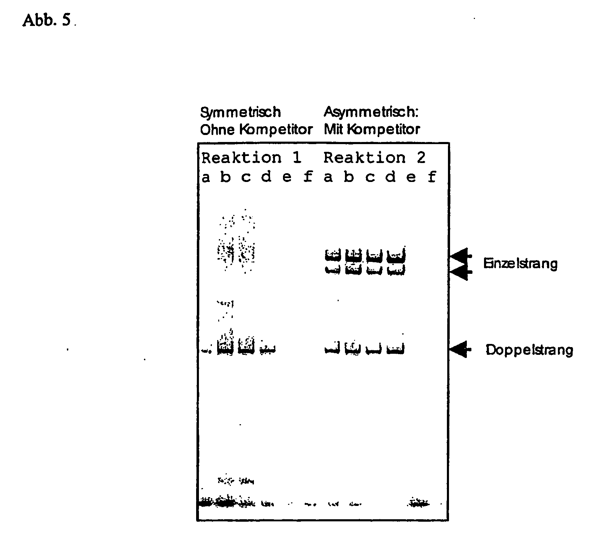

Die Ergebnisse sind in Abb. 5 gezeigt. Bei der Gelanalyse der symmetrischen Reaktionen wird erwartungsgemäß für alle Reaktionen, in denen eine Amplifikation erfolgte, nur ein dominierendes Produkt nachgewiesen. Dabei handelt es sich um das doppelsträngige PCR-Produkt. Im Gegensatz dazu werden bei allen detektierbaren asymmetrischen Reaktionen 3 dominierende Produkte nachgewiesen. Neben dem doppelsträngigen Template werden zwei weitere Banden erhalten, die verschiedenen Strukturen des markierten Einzelstranges entsprechen. Die Gelanalyse zeigt damit, dass die asymmetrische Amplifikation zu einem Einzelstrang-Überschuss führt.

### Ausführungsbeispiel 4:

### Erhöhung des Hybridisierungssignals durch Zugabe von Sekundärstrukturbrechern

Es wurde eine asymmetrische Multiplex-PCR-Reaktion durchgeführt, die folgende Zusammensetzung und Endkonzentrationen aufwies. Als Template wurde genomische DNA verwendet, die das humane cyp2D6 Gen enthielt und deren Genotyp bekannt war:
1 µl cyp2D6-Fusionsprimer Mix bestehend aus:
   0,5 µM uniA cyp2D6 1/2f 
(5'GGAGCACGCTATCCCGTTAGACCAGAGGAGCCCATTTGGTAGTGAGG
CAGGT3')
   1 µM uniA_cyp2D6_1/2f_F2
      (5'GGAGCACGCTATCCCGTTAGACTGGACGCCGGTGGTCGTGCTCAA3')
   1,5 µM uniA_cyp2D6_3/4f
      (5'GGAGCACGCTATCCCGTTAGACCACGCGCACGTGCCCGTCCCA3')
   1 µM uniA_cyp2D6_5/6f_kF
      (5'GGAGCACGCTATCCCGTTAGACCGCTGGCTGGCAAGGTCCTACGC3')
   0,8 µM uniA_cyp2D6_8/9f_kF
      (5'GGAGCACGCTATCCCGTTAGACGCCCCGGCCCAGCCACCAT3')
   1 µM uniB_cyp2D6_1/2r
      (5'CGCTGCCAACTACCGCACATGGGTCCCACGGAAATCTGTCTCTGT3')
   0,5 µM uniB_cyp2D6_1/2r_F1
      (5'CGCTGCCAACTACCGCACATGCCTCTGCCGCCCTCCAGGACCTC3')
   1,5 µM uniB_cyp2D6_3/4r
      (5'CGCTGCCAACTACCGCACATGCTCTCGCTCCGCACCTCGCGCAGA3')
   1 µM uniB_cyp2D6_5/6r 
(5'CGCTGCCAACTACCGCACATGCCCTCGGCCCCTGCACTGTTTCCCAGA
3')
   0,8 µM uniB_cyp2D6_8/9r_kF
      (5'CGCTGCCAACTACCGCACATGTGGCTAGGGAGCAGGCTGGGGACT3')

| | |
|---|---|
| 200 µM | dNTPs |
| 1x | Advantage PCR Reaction buffer (Clontech, Palo Alto, USA) |
| 0,1 U/µl | Advantage DNA Polymerase (Clontech, Palo Alto, USA) |
| 1 µl | humane DNA-Probe KDL 36 (0,71µg/µl) (Urs Meyer, Biozentrum Basel) Genotyp: homozygot cyp2D6 C2938T |

Das Gesamtvolumen der PCR betrug 25 µl. In der nun folgenden ersten Phase der zweiphasigen Amplifikationsreaktion erfolgte die Inkubation unter folgenden Bedingungen:

| | | |
|---|---|---|
| Initiale Denaturierung: | 95° C | 10 min |
| 25 Zyklen: | 95° C | 35 sec |
| | 65° C | 50 sec |
| | 72° C | 70 sec |
| | | |
| Kühlen auf 4° C | | |

Im Verlauf der Reaktion wurden die Primer aufgebraucht und alle Fragmente der Multiplex-PCR auf ein einheitliches molares Niveau amplifiziert. In dieser ersten Phase erfolgte die Amplifikation mit Primern, deren 3'-Ende Spezifität für die Targetfragmente aufwiesen, deren 5'-Enden hingegen für alle forward-Primer und für alle reverse-Primern eine einheitliche Sequenz besaßen.

In der nun folgenden zweiten Phase der Reaktion wurden Primer zugegeben, die komplementär zu den einheitlichen Sequenzen waren. Gleichzeitig wurde ein Kompetitor zugegeben, der die gleiche Sequenz wie der jetzt eingesetzte reverse-Primer besaß, aber eine NH₂-Modifikation an der 3'-OH-Gruppe am 3'-Ende aufwies. Dadurch erfolgte die Reaktion in der zweiten Phase asymmetrisch.

Der universelle forward-Primer uniB hatte folgende Sequenz und war mit einer Cy3-Gruppe am 5'-Ende markiert:
5'-GGAGCACGCTATCCCGTTAGAC-3'

Der universelle reverse-Primer uniA hatte folgende Sequenz:
5'-CGCTGCCAACTACCGCACATG-3'

Die zweite Phase wurde mit der Zugabe von 25 µl folgenden Reaktionsmixes gestartet:

| | |
|---|---|
| 240 nM | Primer uniA |
| 560 nM | Kompetitor uniA 3 'NH2 |
| 800 nM | Primer uniB 5'Cy3 (Amersham-Pharmacia, Freiburg, Deutschland) |
| 1x | Advantage PCR Reaction buffer (Clontech, Palo Alto, USA) |
| 0,4 U/µl | Advantage DNA Polymerase (Clontech, Palo Alto, USA) |
| 400 µM | dNTPs |

Das Gesamtvolumen der PCR-Reaktion betrug 25 µl. Die Reaktion wurde in der zweiten Phase folgendermaßen inkubiert:

| | | |
|---|---|---|
| Initiale Denaturierung: | 95° C | 30 sec |
| 20 Zyklen: | 95° C | 35 sec |
| | 65° C | 50 sec |
| | 72° C | 90 sec |
| terminale Elongation : | 72° C | 7 min |

### Array-Belegung

Es wurde ein Sondenarray durch in situ-Synthese hergestellt. Das Array bestand aus 1024 Sonden-Elementen von 64 x 64 µm Größe. Diese Spots repräsentierten zwei unterschiedliche Oligonukleotide:

| | |
|---|---|
| C2938T | 5' AATGATGAGAACCTGTGCATAGTGGTG 3' |
| C293 WT | 5' AATGATGAGAACCTGCGCATAGTGGTG 3' |

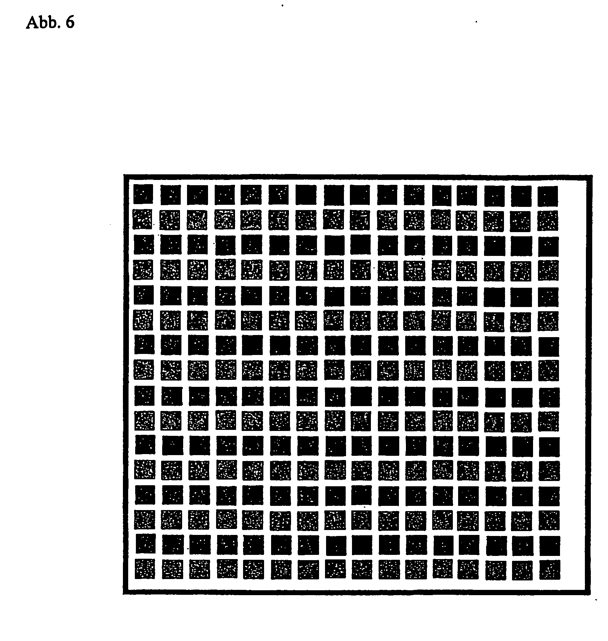

Die Sonden wurden nach dem in Abbildung 6 dargestellten Muster angeordnet. Schwarze Felder entsprechen der Sonde C2938T, graue Felder entsprechen der Sonde C2938 WT.

### Hybridisierung:

Es wurden 5 Hybridisierungsansätze angefertigt, indem jeweils 5 µl der Multiplex-PCR-Reaktionen in 60 µl SSPE, 0,1% SDS aufgenommen wurden. Es wurden die Strukturbrecher

| | |
|---|---|
| C2938TBL3 | (5'-GGCTGACCTGTTCTCTGCCG-3') und |
| C2938TBL5 | (5'-GGAACCCTGAGAGCAGCTTC-3') |

in verschiedenen molaren Konzentrationen zugegeben:

| | |
|---|---|
| Reaktion 1: | keine Strukturbrecher |
| Reaktion 2: | je 1,5 nM Strukturbrecher |
| Reaktion 3: | je 15 nM Strukturbrecher |
| Reaktion 4: | je 150 nM Strukturbrecher |
| Reaktion 5: | je 1,5 µM Strukturbrecher |

Zu jeder Reaktion wurde ein Array mit dem oben beschriebenen Layout gegeben. Die Hybridisierungsreaktionen wurden für 5 min bei 95° C denaturiert und anschließend für 1 h bei 50° C inkubiert. Im Anschluss erfolgten drei aufeinander folgende 5-minütige Waschschritte bei 30° C in 2x SSC, 0,1% SDS, bei 30° C in 2x SSC und bei 20° C in 0,2x SSC. Die Chips wurden entnommen und anschließend im Vakuum getrocknet.

### Detektion der Hybridisierungssignale:

Die Detektion der Hybridisierungssignale erfolgte unter einem Zeiss Fluoreszenzmikroskop (Zeiss, Jena, Deutschland). Die Anregung erfolgte im Auflicht mit einer Weißlichtquelle und einem für Cyanine 3 geeigneten Filtersatz. Die Signale wurden mit einer CCD-Kamera (PCO-Sensicam, Kehlheim, Deutschland) aufgezeichnet. Die Belichtungszeit betrug 5000 ms.
(Abb. 7)

### Ergebnisse:

Die Hybridisierungsergebnisse zeigen folgendes Muster: Die Sonden für die Mutation C2938T zeigen starke Signale, während die Wildtyp-Sonden sowie die Deletionsvarianten deutlich geringere Signale zeigen. Dies entspricht den Erwartungen. Auffällig ist, dass die Signale durch Zugabe der Strukturbrecher weiter verstärkt werden. Damit ist eine Kombination von Kompetitor und Sekundärstrukturbrecher besonders nützlich, um gute Signalstärken zu erreichen.

### Ausführungsbeispiel 5:

Zugabe eines Sekundärstrukturbrechers, der in Nachbarschaft mit zu den Sonden komplementären Sequenzabschnitten des Targets hybridisiert.

Es wurde eine asymmetrische Multiplex-PCR-Reaktion durchgeführt, die identisch zu der des Beispiels 4 war. Es fanden Arrays mit dem gleichen Layout wie im vorangehenden Beispiel 4 beschrieben Verwendung.

### Hybridisierung:

Es wurden 4 Hybridisierungsansätze angefertigt, indem jeweils 5 µl der Multiplex-PCR-Reaktionen in 60 µl SSPE, 0,1% SDS aufgenommen wurden. Die Hybridisierungsansätze unterschieden sich hinsichtlich der Zugabe der Strukturbrecher

| | |
|---|---|
| C2938TBL3 | (5'-GGCTGACCTGTTCTCTGCCG-3') bzw. |
| C2938TBL5 | (5'-GGAACCCTGAGAGCAGCTTC-3'). |

| | |
|---|---|
| Reaktion 1: | keine Strukturbrecher |
| Reaktion 2: | 1,5 µM Strukturbrecher C2938TBL3 |
| Reaktion 3: | 1,5 µM Strukturbrecher C2938BL5 |
| Reaktion 4: | je 1,5 µM Strukturbrecher C2938TBL3 und C2938TBL5 |

Zu jeder Reaktion wurde ein Array mit dem oben beschriebenen Layout gegeben. Die Hybridisierungsreaktionen wurden für 5 min bei 95° C denaturiert und anschließend für 1 h bei 50° C inkubiert. Im Anschluss erfolgten drei aufeinander folgende 5-minütige Waschschritte bei 30° C in 2x SSC, 0,1% SDS, bei 30° C in 2x SSC und bei 20° C in 0,2x SSC. Die Chips wurden entnommen und anschließend im Vakuum getrocknet.

### Detektion der Hybridisierungssignale:

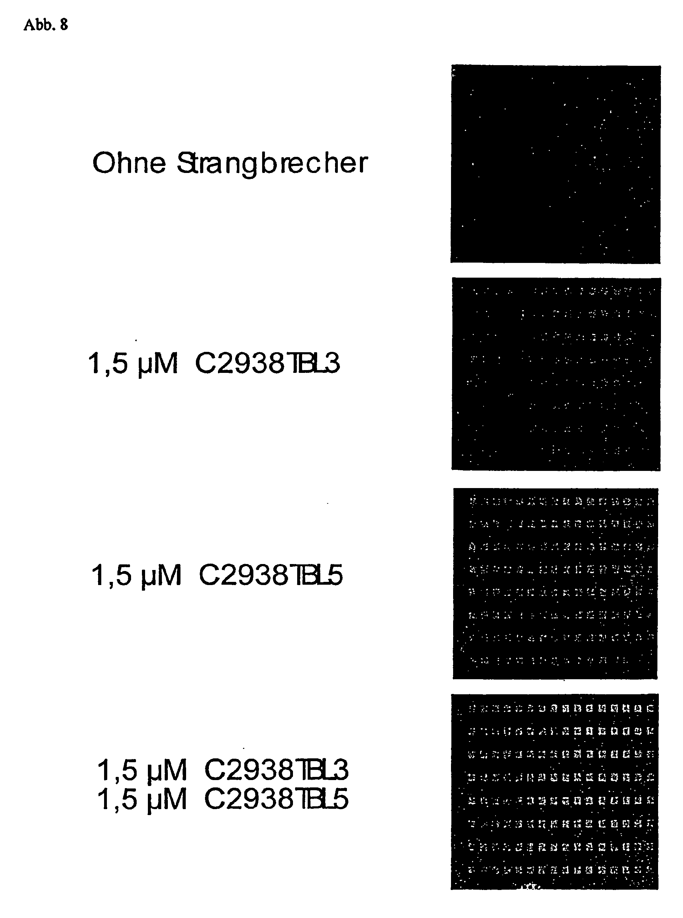

Die Detektion der Hybridisierungssignale erfolgte unter einem Zeiss Fluoreszenzmikroskop (Zeiss, Jena, Deutschland). Die Anregung erfolgte im Auflicht mit einer Weißlichtquelle und einem für Cyanine 3 geeigneten Filtersatz. Die Signale wurden mit einer CCD-Kamera (PCO-Sensicam, Kehlheim, Deutschland) aufgezeichnet. Die Belichtungszeit betrug 10000 ms (Abb. 8).

### Ergebnisse:

Die Hybridisierungsergebnisse zeigen folgendes Muster: Die Sonden für die Mutation C2938T zeigen starke Signale, während die Wildtyp-Sonden sowie die Deletionsvarianten deutlich geringere Signale zeigen. Dies entspricht den Erwartungen. Auffällig ist, dass bereits bei Zugabe eines der beiden Strukturbrecher deutlich erhöhte Signalen erzielt werden.

### Ausführungsbeispiel 6:

### Einfluss von Strukhubrechern auf die Match-Mismatch-Diskriminierung

Sekundärstrukturbrecher sind im vorliegenden Beispiel Oligonukleotide, welche bei der Hybridisierung einer doppelsträngigen Nukleinsäure in der Nähe zur eigentlichen Hybridisierungssonde binden. Vermutlich wird der Doppelstrang der Nukleinsäure an der betreffenden Stelle aufgelöst und die Hybridisierungssonde kann besser binden. Wie im nachfolgenden Anwendungsbeispiel gezeigt wird, kommt es dadurch zu einer Verstärkung des Hybridisierungssignals um den Faktor 3-5 ohne Beeinflussung der Spezifität der Reaktion.

### Vorbereitung des Arrays:

Auf einem epoxidierten Glaswafer der Firma Schott wurden 397 DNA-Sondenarrays mit je 15 Oligonukleotidsonden unterschiedlicher Sequenz und Länge synthetisch erzeugt. Alle Sonden waren komplementär zu einer Teilsequenz des Exons 5/6 des humanen *cyp2D6*-Gens und unterschieden sich lediglich im mittleren Sondenbereich durch Einfügung einiger verschiedener Mutationen.

Nach der Synthese wurde der Wafer in 3,4 x 3,4 mm große Chips zersägt, wobei jeder dieser Chips ein Sondenarray mit allen 15 Oligonukleotiden in unterschiedlichen Redundanzen enthielt. Insgesamt bestand jedes Array aus 256 Spots. Diese waren in 16 identischen Feldern von 16 Spots angeordnet. In den einzelnen Spots wurden folgende Sequenzen aufgebaut.

| | |
|---|---|
| C2938T | AATGATGAGAACCTGTGCATAGTGGTG |
| C2938TC | AATGATGAGAACCTGTCGCATAGTGGTG |
| C2938GT | AATGATGAGAACCTGGTGCATAGTGGTG |
| C2938GTC | AATGATGAGAACCTGGTCGCATAGTGGTG |
| C2938G | AATGATGAGAACCTGGGCATAGTGGTG |
| C2938GC | AATGATGAGAACCTGGCGCATAGTGGTG |
| C2938d | AATGATGAGAACCTGGCATAGTGGTG |
| C2938CT | AATGATGAGAACCTGCTGCATAGTGGTG |
| C2938CTC | AATGATGAGAACCTGCTCGCATAGTGGTG |
| C2938 | AATGATGAGAACCTGCGCATAGTGGTG |
| C2938CC | AATGATGAGAACCTGCCGCATAGTGGTG |
| C2938AT | AATGATGAGAACCTGATGCATAGTGGTG |
| C2938ATC | AATGATGAGAACCTGATCGCATAGTGGTG |
| C2938A | AATGATGAGAACCTGAGCATAGTGGTG |
| C2938AC | AATGATGAGAACCTGACGCATAGTGGTG |

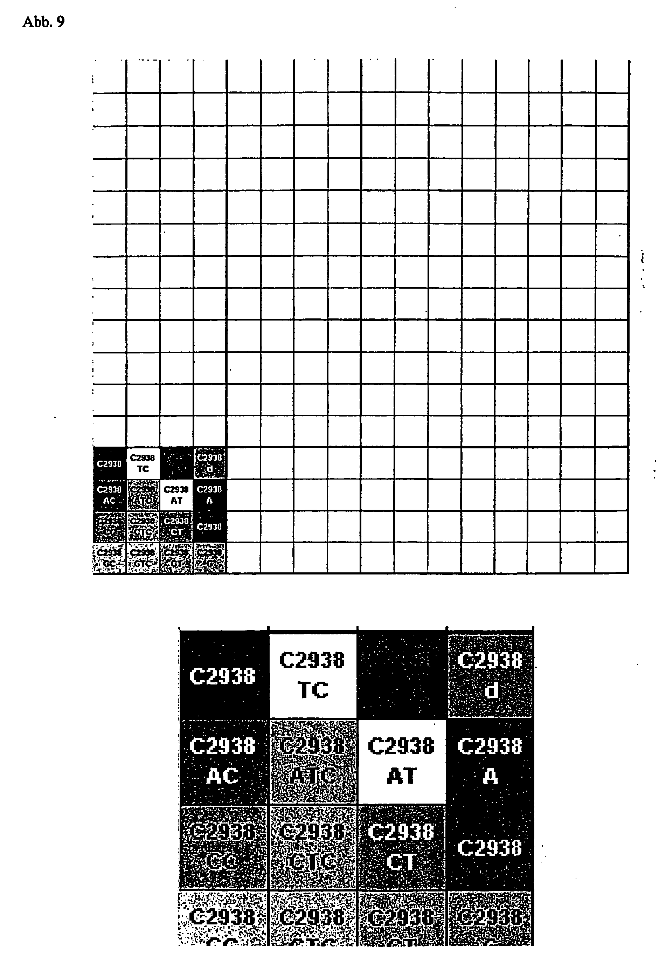

Die Anordnung der Sonden auf dem Array erfolgte wie in Abbildung 9 gezeigt. Jedes der 16 stark umrandeten Quadrate enthielt 16 Array-Elemente, die mit den für das Quadrat im unteren linken Bereich der Abbildung gezeigten Sonden bestückt waren.

### Vorbereitung der zu hybridisierenden DNA-Probe:

Zur Amplifikation des Targets für die Hybridisierung wurde eine asymmetrische PCR mit einer klinischen DNA-Probe als template (KDL 31, zur Verfügung gestellt durch Prof. U. Meyer, Biozentrum Basel) durchgeführt. Durch die PCR wurde eine Teilsequenz des Exons 5/6 des humanen cyp2D6-Gens amplifiziert. In der PCR wurde ein Kompetitor eingesetzt, dessen Sequenz zum forward-Primer identisch war, jedoch an der 3'-OH-Gruppe am 3'-Ende mit einer NH₂-Gruppe modifiziert war. Der reverse-Primer war am 5'-Ende mit Cy3 markiert.

Dazu wurde nach folgendem Schema ein PCR-Ansatz mit folgenden Endkonzentrationen angesetzt:

| | |
|---|---|
| 20 nM | forward-Primer: cyp2D6_5/6f |
| | (5'GGACTCTGTACCTCCTATCCACGTCA3') |
| 180 nM | Kompetitor: cyp2D6_5/6f_3NH2 |
| | (5'GGACTCTGTACCTCCTATCCACGTCA-NH₂-3') |
| 200 nM | reverse-Primer: cyp2D6_5/6r_5Cy3 |
| | (5'-Cy3-CCCTCGGCCCCTGCACTGTTTCCCAGA3') |
| 200 µM | dNTPs |
| 1x | 10x Taq Reaction Buffer (Eppendorf AG, Hamburg, Deutschland) |
| 5 U | MasterTaq DNA-Polymerase (Eppendorf AG, Hamburg, |
| | Deutschland), 1 ng/µl Template-DNA (KDL 31), |
| | Konzentration 50 ng/µl) |

Das Gesamtvolumen der PCR betrug 50 µl.

Dieser Ansatz wurde nach folgendem Temperaturprotokoll inkubiert:

| | | |
|---|---|---|
| Initiale Denaturierung: | 10 min | 95° C |
| 30 Zyklen: | 30 sec | 95° C |
| | 50 sec | 62° C |
| | 90 sec | 72° C |
| terminale Elongation | 7 min | 72° C |

Die Reaktionsprodukte wurden nach Herstellerprotokoll über Säulen (PCR Purification Kit, Qiagen, Hilden, Deutschland) gereinigt und anschließend quantifiziert.

### Hybridisierung:

Um den Einfluss der Sekundärstrukturbrecher auf die Hybridisierung zu untersuchen, wurden zwei verschiedene Hybridisierungsansätze durchgeführt: Zum ersten Ansatz wurden die entsprechenden Sekundärstrukurbrecher zugegeben, der zweite Ansatz diente als Kontrolle ohne Sekundärstrukurbrecher.

Dazu wurde die Cy3-markierte asymmetrische PCR in einer Endkonzentration von 25 nM in 50 µl 6x SSPE, 0,1% SDS aufgenommen. Zum Ansatz 1 wurden zusätzlich die Strukturbrecher

| | |
|---|---|
| 2938BL5 | (5'-GGAACCCTGAGAGCAGCTTC-3') und |
| 2938BL3 | (5'-GGCTGACCTGTTCTCTGCCG-3') |

zu einer Endkonzentration von je 1 µM zugegeben.

Nach Zugabe eines Chips in jeden Hybridisierungsansatz wurden diese für 5 min bei 95° C denaturiert und danach 1 h bei 45° C inkubiert. Anschließend wurden die Chips unter Schütteln je 10 min bei 30° C in 2x SSC, 0,2%SDS, bei 30° C in 2x SSC und bei 20° C in 0,2x SSC (Sambrook et al., *vide supra*) gewaschen und mit Druckluft trockengeblasen.

### Detektion der Hybridisierung:

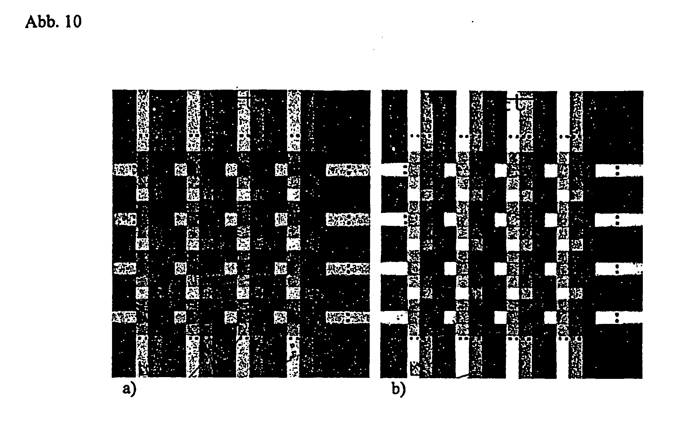

Die hybridisierten und gewaschenen Chips wurden in einem Slidescanner (Scanarray4000, GSI Lumonics) ausgelesen. Dabei wurden die Chips jeweils mit einer Scannereinstellung aufgenommen, die einen möglichst dynamischen Bereich bei der vorhandenen Signalintensität bot. Für die spätere Normierung der Daten wurde zusätzlich ein Fluoreszenzstandard (Fluoris^{®}I, Clondiag Chip Technologies) bei den gewählten Messeinstellungen aufgenommen (Abb. 10).

### Ergebnisse:

Die Ergebnisse der Hybridisierung sind in Abb. 10 dargestellt. Abb. 10a zeigt die Fluoreszenzsignale nach Hybridisierung einer PCR unter Zugabe von Strukturbrechern Die Aufnahme erfolgte in dem Slidescanner mit Laser-Power 70 und Photomultiplier 80. Abb. 10b zeigt dagegen die Negativkontrolle ohne Zugabe der entsprechenden Strukturbrecher. Die Scannereinstellungen waren Laser-Power 100 und Photomultiplier 75. Zu beachten ist, dass Abb. 10b somit bei deutlich höherer Laser- und Photomultiplierleistung des Scanners aufgenommen wurde. Daher sind die Signale auf diesem Chip nur scheinbar stärker.

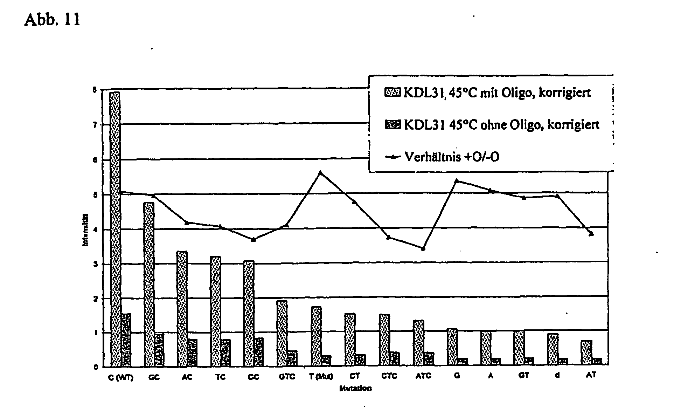

Die aufgenommenen Bilder wurden mit der Bildauswertungs-Software Iconoclust^{®} (Clondiag Chip Technologies) ausgewertet und die gewonnenen Daten mit Hilfe der Daten des Fluoreszenzstandards normalisiert. Die so erhaltenen Messdaten sind im Diagramm in Abb. 11 aufgetragen.

In Abb. 11 ist zu erkennen, dass bei Zugabe von Strukturbrechern eine Steigerung des Fluoreszenzsignals um den Faktor 3,5 bis 5,5 erfolgt. Wie aus dem Verhältnis der Hybridisierungssignale mit und ohne Sekundärstrukturbrecher-Oligonukleotid zu ersehen ist, wird die Spezifität der Hybridisierung unabhängig von der untersuchten Mutation im Rahmen der Fehlergrenzen nicht beeinflusst.

### Ausführungsbeispiel 7:

Durchführung einer kontinuierlichen asymmetrischen PCR-Amplifikations- und Hybridisierungsreaktion unter Zugabe von Strukturbrecher-Olgonukleotiden zu Beginn der PCR-Reaktion.

### PCR-Amplifikation und Hybridisierung

Als Target für die Hybridisierung diente eine asymmetrische Cy3-markierte PCR mit einer klinischen DNA-Probe als template (KDL31, U. Meyer, Biozentrum Basel). Es wurden die gleichen forward- und reverse-Primer sowie der gleiche Kompetitor wie im Beispiel 6 verwendet. Hinsichtlich der Sequenz wurden ebenfalls die gleichen Strukturbrecher eingesetzt. Allerdings trugen diese am 3'-Ende eine Amino-Modifikation (NH₂-Modifikation).

Der PCR-Reaktionsansatz hatte folgende Zusammensetzung und Endkonzentration:

| | |
|---|---|
| 70nM | forward-Primer: cyp2D6_5/6f |
| | (5'-GGACTCTGTACCTCCTATCCACGTCA-3') |
| 130 nM | Kompetitor: cyp2D6_5/6f_3NH2 |
| | (5'-GGACTCTGTACCTCCTATCCACGTCA-NH₂-3') |
| 200 nM | reverse Primer: cyp2D6_5/6r_5Cy3 |
| | (5'-Cy3-CCCTCGGCCCCTGCACTGTTTCCCAGA-3') |
| 20 nM | Strukturbrecher 2938BL5 |
| | (5'GGAACCCTGAGAGCAGCTTC- NH₂-3') |
| 20 nM | Strukturbrecher 2938BL3 |
| | (5'GGCTGACCTGTTCTCTGCCG-NH₂-3') |
| 200 µM | dNTPs |
| 1x Puffer | 10x cDNA-Reaction Buffer (Clontech, Palo Alto, USA), |
| 70 mM | K-Acetat |
| 15 U | MasterTaq DNA-Polymerase (Eppendorf AG, Hamburg, Deutschland), |
| 1 ng/µl | Template-DNA (humane genomische DNA KDL 31 (Urs Meyer, Biozentrum Basel) |

Das Volumen der PCR betrug 50 µl. Ein Parallelansatz hatte die identische Zusammensetzung für alle Komponenten mit Ausnahme der Strukturbrecher-Oligonukleotide. Letztere wurden nicht zugegeben.

Beide Ansätze wurden mit je einem Array desselben Layouts wie in Beispiel 6 versehen und nach folgendem Temperaturprotokoll inkubiert:

| | | |
|---|---|---|
| Initiale Denaturierung: | 10min | 95° C |
| 30 Zyklen: | 30s | 95° C |
| | 50s | 62° C |
| | 90s | 72° C |
| Terminale Elongation: | 7min | 72° C |

Die Hybridisierung erfolgte folgendermaßen:

| | | |
|---|---|---|
| Denaturierung: | 5min | 95° C |
| Hybridisierung | 60min | 40° C |

Im Anschluss an die Hybridisierung wurden die Chips unter Schütteln je 10min bei 30° C in 2x SSC, 0,2% SDS, bei 30° C in 2x SSC und bei 20° C in 0,2xSSC (Sambrook et al., *vide supra*) gewaschen und mit Druckluft getrocknet.

### Nachweis der Hybridisierung und Auswertung

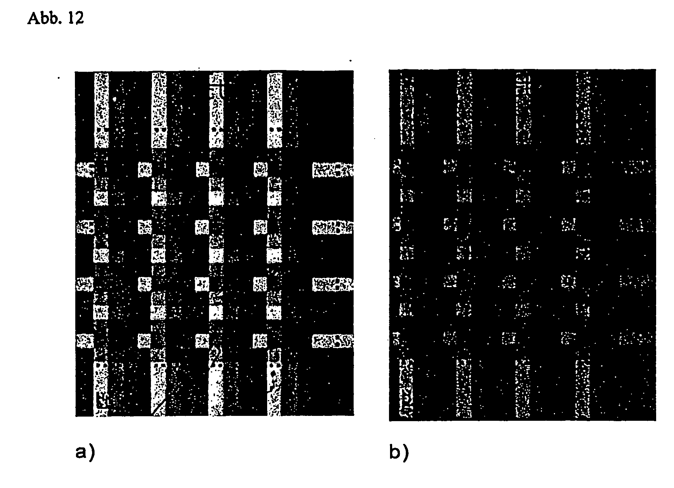

Die hybridisierten und gewaschenen Chips wurden in einem Slidescanner (Scanarray4000, GSI Lumonics) ausgelesen. Beide Arrays wurden mit den identischen Scannereinstellungen (Laser-Power 70 und Photomultiplier 80) aufgenommen. Abbildung 12 (Abb.12) zeigt die entsprechenden Scanneraufuahmen. Abb. 12a zeigt die Fluoreszenzsignale nach Hybridisierung einer PCR unter Zugabe von Strukturbrechern, Abb. 12b dagegen die Reaktion ohne Zugabe der entsprechenden Strukturbrecher.

### Ergebnisse

Es wird deutlich, dass die Zugabe der Strukturbrecher am Beginn der PCR-Reaktion keinen negativen Einfluß auf das Ergebnis der Reaktion hatte. Vielmehr war eine sensitivere Detektion der Hybridisierungssignale bei Zugabe der Strukturbrecher-Oligonukleotide möglich.

### Ausführungsbeispiel 8:

Vergleich von asymmetrischer und symmetrischer Amplifikation in einer kontinuierlichen PCR-Amplifkations- und Hybridisierungsreaktion.

Es wurde eine Target-Sequenz aus *Corynebacterium glutamicum* durch Hybridisierung gegen einen Sondenarray auf Vorhandensein von Insertionen oder Deletionen untersucht.

### Array

Es wurde ein DNA-Array durch ortsspezifische in situ-Synthese von Oligonukleotiden hergestellt. Das Array enthielt insgesamt 64 Array-Elemente mit einer Größe von 256 x 256 µm. Jedes Array-Element enthielt eine der folgenden Sonden.

| | |
|---|---|
| Insertion: | 5'-GTTTCCCAGGGCTTATCCC-3' |
| Deletion: | 5'-GTTTCCCAGCTTATCCC-3' |
| Match: | 5'-GTTTCCCAGGCTTATCCC-3' |

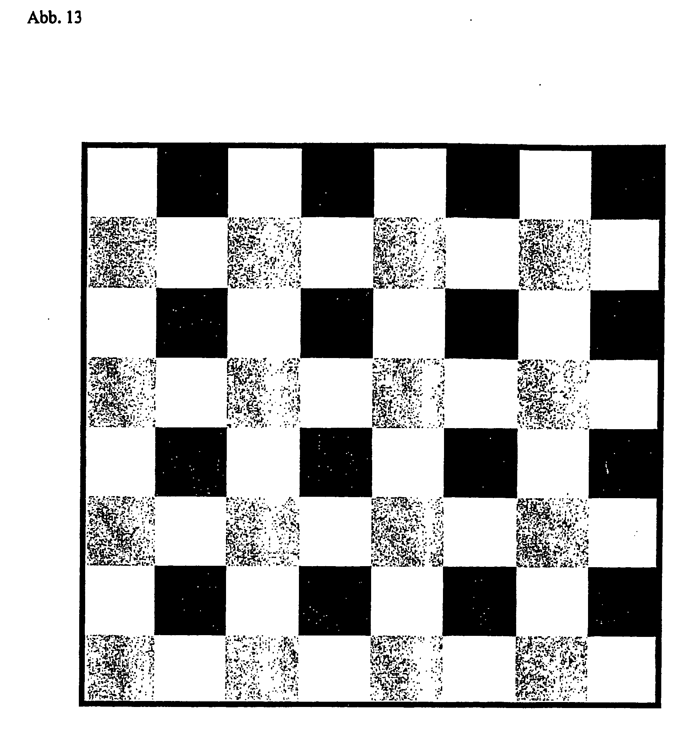

Die Zuordnung der einzelnen Sonden auf die Array-Elemente ist in Abbildung 13 wiedergegeben. Mit der Match-Sonde belegte Array-Elemente sind weiß, mit der Deletions-Sonde belegte Sonden-Elemente sind schwarz und mit der Insertions-Sonde belegte Sondenelemente sind grau dargestellt.

### Kontinuierliche PCR und Hybrldisierungsreaktion

Der forward-Primer 16sfD15'Cy3 hatte folgende Sequenz und war am 5'-Ende mit dem Fluoreszenzfarbstoff Cy3 (Amersham-Pharmacia, Freiburg, Deutschland) markiert:
5'-AGAGTTTGATCCTGGCTCAG-3'

Der reverse-Primer 16sRa und der Kompetitor 16sRa3'NH₂ hatte folgende Sequenz:
5'-TACCGTCACCATAAGGCTTCGTCCCTA-3'

Der Kompetitor trug am 3'-Ende eine Amino-Modifikation (NH₂), die während der chemischen Synthese des Oligonukleotids in das Molekül integriert wurde. Es wurden jeweils 5 symmetrische und 5 asymmetrische Reaktionsansätze mit folgender Zusammensetzung hergestellt. In den Klammern sind jeweils die Konzentrationen der Stock-Lösungen angegeben.

Asymmetrische Reaktionen:

| | |
|---|---|
| 0,5 µl | reverse-Primer 16sRa (10 µM) |
| 1 µl | Kompetitor 16SRa3'NH₂ (10 µM) |
| 1,5 µl | 16SfD15'Cy3 (10 µM) |
| 0,75 µl | dNTP Mix (20 mM) |
| 0,75 µl | chromosomale DNA *Corynebakterium glutamicum* (10 ng/µl) |
| 0,75 µl | 10 x Clontech cDNA Puffer |
| 54,5 µl | PCR-Grade Water |
| 3 µl | Taq-Polymerase (Eppendorf, Hamburg, Deutschland) (5 U/µl) |
| 5 µl | 1M Kaliumacetat |

Symmetrische Reaktionen:

| | |
|---|---|
| 1,5 µl | reverse-Primer 16Ra (10 µM) |
| 1,5 µl | 16SfD15'Cy3 (10 µM) |
| 0,75 µl | dNTP Mix (20 mM) |
| 0,75 µl | chromosomale DNA *Corynebacterium glutamicum* (10ng/µl) |
| 0,75 µl | 10 x Clontech cDNA Puffer |
| 54,5 µl | PCR-Grade Water |
| 3 µl | Taq-Polymerase (Eppendorf, Hamburg, Deutschland) (5 U/µl) |
| 5 µl | 1M Kaliumacetat |

Zu den Reaktionsansätzen in 0,2 ml PCR-Tubes wurde jeweils ein Array gegeben. Die Reaktionsgemische einschließlich Array wurden folgendem Temperturprotokoll zur PCR-Amplifkation und Hybridisierung unterworfen:

### PCR:

| | | |
|---|---|---|
| Initiale Denaturierung | 2 min | 95° C |
| 10-30 PCR-Zyklen | 30 sec | 95° C |
| | 30 sec | 62° C |
| Extension | 30 sec | 72° C |

Die PCR-Amplitkation wurde für jeweils einen symmetrischen und einen asymmetrischen Reaktionsansatz nach 10, 15, 20, 25 bzw. 30 Zyklen beendet. Danach folgte sofort die Hybridisierung.

### Hybridisierung:

| | | |
|---|---|---|
| Denaturieren | 30 sec | 95° C |
| Hybridisieren | 60 min | 42° C |

### Auswertung der Hybridisierung

Nach Abschluss der Hybridisierung wurden die Arrays ein Mal kurz in 0,2x SSC bei Raumtemperatur gewaschen. Nach Entfernen der Flüssigkeit wurden die Arrays getrocknet und in einem Fluoreszenzscanner (Scanarray4000, GSI Lumonics) ausgelesen. Die Einstellungen von Laser und PMT variierten in Abhängigkeit von der Intensität der Hybridisierungssignale.

### Ergebnisse

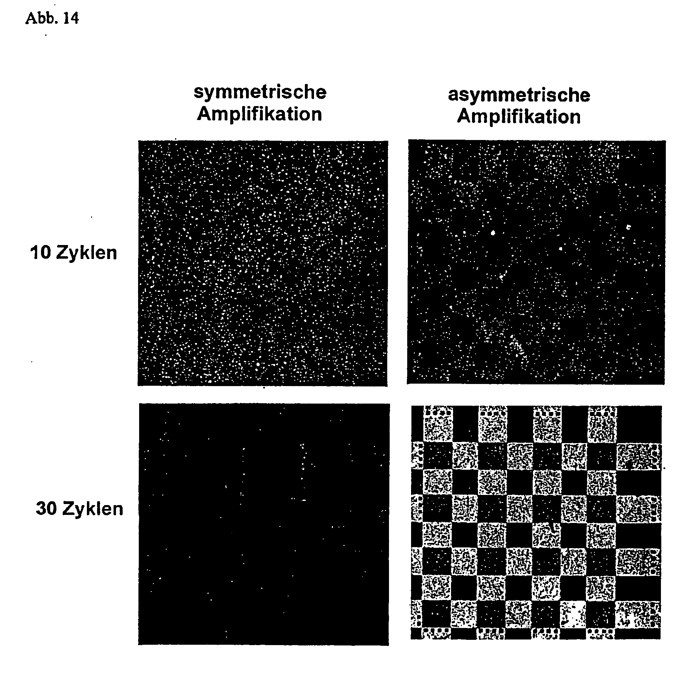

Es zeigte sich, dass die asymmetrische Reaktion gegenüber der symmetrischen Reaktion stets zu einem stärkeren Hybridisierungssignal führte, unabhängig davon, über wie viele Zyklen die Amplifikation erfolgte. Als Beispiel sind in Abbildung 14 die Hybridisierungssignale der nach 10 bzw. 30 Zyklen abgestoppten Reaktion zu sehen.

### Ausführungsbeispiel 9:

Zugabe eines Moleküls, das gleichzeitig als Kompetitor und Sekundärstrukturbrecher wirkt, zum Beginn einer kontinuierlichen PCR- und Hybridisierungsreaktion

Dieses Ausführungsbeispiel zeigt, dass die Zugabe eines Moleküls, das gleichzeitig als Kompetitor und Sekundärstrukturbrecher wirkt, zu Beginn einer kontinuierlichen PCR- und Hybridisierungsreaktion, zu höheren Hybridisierungssignalen führt.

Sekundärstrukturbrecher sind Oligonukleotide, welche bei der Hybridisierung einer doppelsträngigen Nukleinsäure in Nachbarschaft zur eigentlichen Hybridisierungssonde binden. Dadurch kann die Hybridisierungssonde besser binden. Bei Kompetitoren handelt es sich um Moleküle, die während der PCR-Amplifikation durch Bindung an einen der komplementären Stränge die Bildung des Gegenstranges hemmer. Das folgende Ausführungsbeispiel zeigt, daß ein Molekültyp die Funktionalität sowohl des Kompetitors als auch des Strukturbrechers aufweisen kann.

### Herstellung des Arrays

Auf einem in einem Standardprozeß bei Clondiag epoxidierten Glaswafer der Firma Schott wurden 397 DNA-Sonden-Arrays je mit 15 Oligonukleotiden unterschiedlicher Sequenz und Länge mittels micro wet printing (Clondiag) synthetisch erzeugt. Alle Oligos waren komplementär zu einer Teilsequenz des Exons 5/6 des humanen *cyp2D6*-Gens und unterschieden sich lediglich im mittleren Sondenbereich durch Einfügung einiger verschiedener Mutationen. Nach der Synthese wurde der Wafer in 3,4 x 3,4mm große Chips zersägt, wobei jeder dieser Chips ein Sondenarray mit allen 15 Oligonukleotiden in unterschiedlichen Redundanzen enthielt. Insgesamt bestand jedes Array aus 256 Spots. Diese waren in 16 identischen Feldern von 16 Spots angeordnet. In den einzelnen Spots wurden folgende Sequenzen aufgebaut.

| | |
|---|---|
| C2938T | AATGATGAGAACCTGTGCATAGTGGTG |
| C2938TC | AATGATGAGAACCTGTCGCATAGTGGTG |
| C2938GT | AATGATGAGAACCTGGTGCATAGTGGTG |
| C2938GTC | AATGATGAGAACCTGGTCGCATAGTGGTG |
| C2938G | AATGATGAGAACCTGGGCATAGTGGTG |
| C2938GC | AATGATGAGAACCTGGCGCATAGTGGTG |
| C2938d | AATGATGAGAACCTGGCATAGTGGTG |
| C2938CT | AATGATGAGAACCTGCTGCATAGTGGTG |
| C2938CTC | AATGATGAGAACCTGCTCGCATAGTGGTG |
| C2938 | AATGATGAGAACCTGCGCATAGTGGTG |
| C2938CC | AATGATGAGAACCTGCCGCATAGTGGTG |
| C2938AT | AATGATGAGAACCTGATGCATAGTGGTG |
| C2938ATC | AATGATGAGAACCTGATCGCATAGTGGTG |
| C2938A | AATGATGAGAACCTGAGCATAGTGGTG |
| C2938AC | AATGATGAGAACCTGACGCATAGTGGTG |

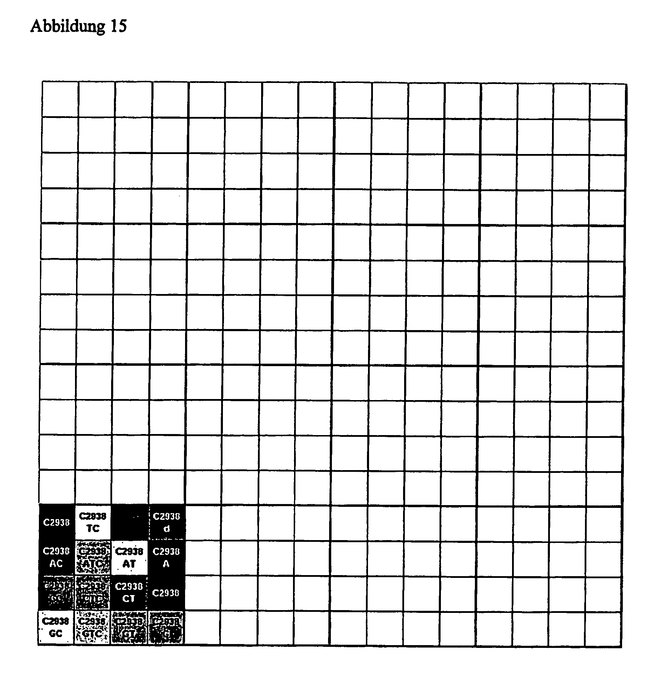

Der Aufbau des Arrays ist in Abbildung 15 gezeigt.

### PCR- und Hybridisierungsreaktion

Es wurde ein Fragment des humanen cyp2D6-genes in Anwesenheit eines DNA-Arrays amplifiziert und anschließend direkt mit dem Array hybridisiert. Die Reaktionsansätze (jeweils 50µl) hatten folgende Zusammensetzung:

Alle Reaktionen enthielten
1 x Advantage PCR Reaction buffer (Clontech, Palo Alto, USA)
2 x Advantage cDNA Polymerase-Mix (BD Bioscience, Palo Alto, USA) chromosomale DNA KDL 31 (0,1 ng/µl Endkonzentration)
200 µM dNTPs (je 200 µM dATP, dTTP, dCTP, dGTP) zusätzlich 35 mM (Endkonzentration) Kaliumacetat (Fluka)
Primer cyp2D6_5/6r_5Cy3, Endkonzentration 200 nM

Zusätzlich enthielten die Reaktionen folgende Komponenten:
Ansatz 1:
   Primer cyp2D6_5/6f, Endkonzentration 66 nM
   Kompetitor cyp2D6_5/6f_3NH2, Endkonzentration 132 nM
Ansatz 2:
   Primer cyp2D6_5/6f, Endkonzentration 66 nM
   Kompetitor cyp2D6_5/6f_3NH2, Endkonzentration 132 nM
   Strukturbrecher/Kompetitor C2938T BL5_3'NH2, Endkonzentration 2 µM
Ansatz 3:
   Primer cyp2D6_5/6f, Endkonzentration 200 nM
   Strukturbrecher/Kompetitor C2938T BL5_3'NH2, Endkonzentration 2 µM
Ansatz 4:
   Primer cyp2D6_5/6f, Endkonzentration 200 nM
Ansatz 5:
   Primer cyp2D6_5/6f, Endkonzentration 66 nM
   Kompetitor cyp2D6_5/6f_3NH2, Endkonzentration 132 nM
   Strukturbrecher/Kompetitor C2938T BL5_3'NH2, Endkonzentration 200 nM

Die Oligonukleotide hatten folgende Sequenzen:
Primer cyp2D6_5/6r_5Cy3, Sequenz 5'-Cy3-CCCTCGGCCCCTGCACTGTTTCCCAGA-3',
Primer cyp2D6_5/6f, Sequenz 5'-GGACTCTGTACCTCCTATCCACGTCA-3'
Kompetitor cyp2D6_5/6f_3NH2. Sequenz 5'-GGACTCTGTACCTCCTATCCACGTCA- NH₂-3'
Strukturbrecher/Kompetitor C2938T BL5_3'NH2, Sequenz 5'-GGAACCCTGAGAGCAGCTTC-NH₂-3'

Die humane chromosomale DNA-Probe KDL 31 wurde zur Verfügung gestellt durch Prof. U. Meyer, Biozentrum Basel.

### PCR-Amplifkation:

Die Reaktionsansätze (in 0,2 ml PCR-Tubes) wurden jeweils mit einem Array versehen und folgendem Temepraturregime unterworfen:
Initiale Denaturierung (2 min, 95°C)
30 Zyklen(30 s, 95°C; 30 s, 62 °C; 150 s, 72 °C)

### Hybridisierung:

Die Hybridisierung erfolgte direkt im Anschluß an die Amplifkation. Dazu wurde das Reaktionsgemisch mit dem Array für 3 min bei 95 °C und anschließend für 60 min bei 42 °C inkubiert.

Im Anschluß an die Hybridisierung wurden die Arrays dem Reaktionsgemisch entnommen, kurz in 500 µl 0, 2x SSC (Sambrook et al, 1989) gespült und anschließend im Vakuum getrocknet.

### Gelanalyse:

Im Anschluß an die Hybridisierung wurden je 5 µl des Reaktionsgemisches entnommen und elektrophoretisch auf einem 2% Agarosegel aufgetrennt (Sambrook et al, 1989). Das Gel wurde mit Ethidiumbromid gefärbt und in einem Geldokumentationssystem (Geldoc 2000, BioRad, München, Deutschland) fotografiert.

### Nachweis der Hybridisierung und Auswertung

Die hybridisierten und gewaschenen Chips wurden in einer Vorrichtung, welche das Auslesen von kleinen Chips in einem Slidescanner erlaubt, in einem Slidescanner (Scanarray4000, GSI Lumonics) ausgelesen. Es wurde mit 85% Laser- und 80%PMT-Leistung gearbeitet.

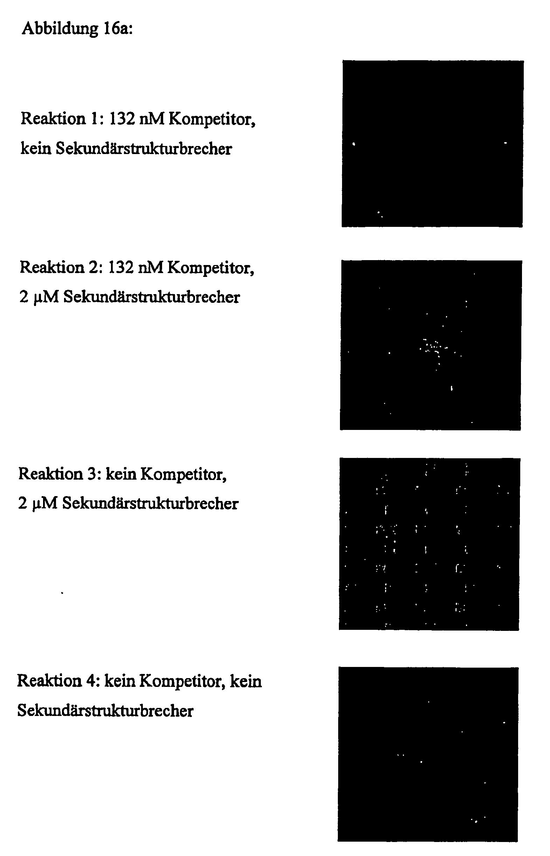

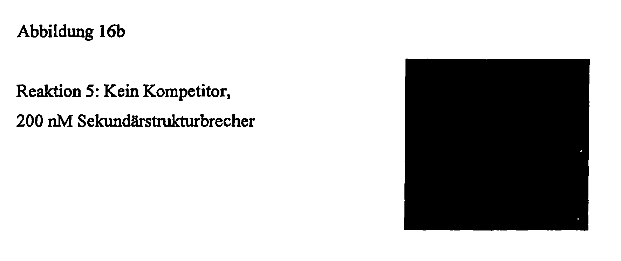

Die Abbildung 16 zeigt die Hybridisierungsergebnisse.

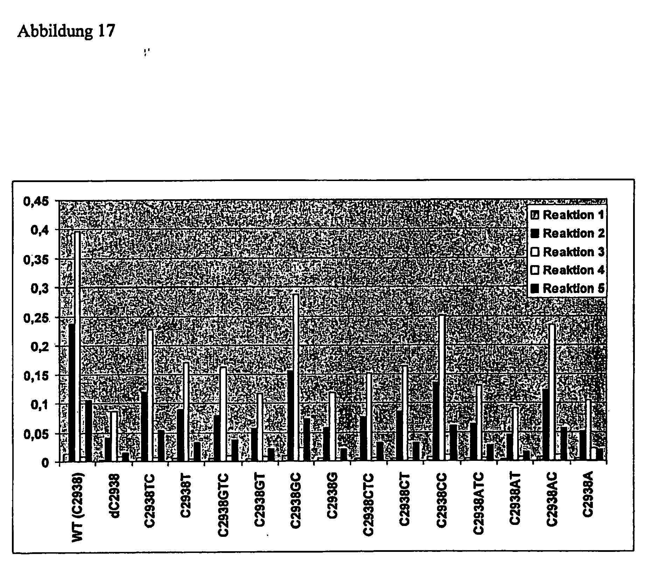

Die aufgenommenen Bilder wurden mit der Bildauswertungs-Software Iconoclust^{®} (Clondiag Chip Technologies) ausgewertet. Die Ergebnisse sind in Abbildung 17 dargestellt

Das Ausführungsbeispiel zeigt, daß sowohl bei symmetrischer, als auch bei asymmetrischer Amplifkation ohne Zugabe von Strukturbrechern im kontinuierlichen PCR- und Hybridisierungsverfahren nur sehr geringe Hybridisierungssignale, erhalten werden, die vom Hintergrundsignal nicht zu diskriminieren sind. Die Zugabe des Sekundärstrukturbrechers führt zu einem starken Ansteigen des Hybridisierungssignales. Die Gelanalyse zeigt, daß der Anstieg der Hybridisierungssignale nicht mit der Menge an PCR-Produkt, das in der Reaktion entstanden ist, korreliert. Die Zugabe des Sekundärstrukturbrecher hemmt offenbar die Produktbildung in der PCR, wie dies für einen Kompetitor zu erwarten ist und fördert die Hybridisierungsfähigkeit der entstandenen Produkte, wie dies für einen Sekundärstrukturbrecher erwartet wird.

Zur Durchführung der entsprechenden Reaktionen siehe auch Sambrook, J., Fritsch, E.F., Maniatis, T. (1989): Molecular Cloning: a laboratory manual, 2nd Edition, Cold Spring Harbor Laboratory Press.

### Ausführungsbeispiel 10:

Identifikation von Pilzspezies durch asymmetrische Amplifkation eines Abschnittes der ribosomalen DNA und Hybridisierung gegen ein Micro-Array.

Der Nachweis der Hybridisierung erfolgt durch ein enzymatisches Verfahren, bei dem TMB durch Meerrettich-Peroxidase umgesetzt wird.

### PCR-Reaktionen:

Es wird ein PCR-Ansatz mit folgender Zusammensetzung hergestellt:

| | |
|---|---|
| 10 µM Primer IS3: | 0,66 µl |
| 10 µM Primer IS4_5'Bio | 2,0 µl |
| 10 µM Kompetitor IS3_3'H2 | 1,33 µl |
| 20 mM dNTP Mix (je 20 mM dATP, dCTP, dGTP und dTTP) | 1,0 µl |
| chromosomale DNA Aspergillus niger 5331 (10 ng/gl) | 2,0 µl |
| 10 x Advantage cDNA PCR-Reaktions-Puffer (Clontech, PaloAlto, U.S.A.) | 10,0 µl |
| PCR-Grade Wasser | 65 µl |
| 25 mM Mg-acetat (Eppendorf, Hamburg, Deutschland) | 6,0 µl |
| 5 U/µl Taq-Polymerase (Eppendorf, Hamburg, Deutschland) | 6,0 µl |
| 1 M Kaliumacetat | 6,0 µl |

Die Primer und der Kompetitor hatten folgende Sequenzen:

| | |
|---|---|
| IS3: | 5'-GCATCGATGAAGAACGCAGC-3' |
| IS4_5'Bio: | 5'-Biotin- TCCTCCGCTTATTGATATGC-3' |
| IS3_3'NH2: | 5'-GCATCGATGAAGAACGCAGC-Aminolink-3' |

Der Reaktionsansatz wurde in zwei 0,2 µl Reaktionsgefäße gegeben und in einem Thermocycler (Mastercycler Gradient, Eppendorf, Hamburg, Deutschland) nach folgendem Temperaturregime inkubiert:
1. Zyklus:

| | |
|---|---|
| 95°C | 300 sec |
| 56°C | 30 sec |
| 72°C | 60 sec |

30 Zyklen:

| | |
|---|---|
| 95°C | 30 sec |
| 56°C | 30 sec |
| 72°C | 60 sec |

Die Reaktionsgemische wurden vereinigt und gegen ein Array-Tube der Firma Clondiag hybridisiert.

### Array-Tube:

Es fand ein Array-Tube (Clondiag Chip Technologies, Jena, Deutschland) Verwendung, das mit folgenden Sonden in 3-facher Redundanz bestückt wurde:

| | |
|---|---|
| A_Pen_Gen_24 | 5'-CGGTCCTCGAGCGTATGGGGCTTT-Aminolink-3' |
| A.ter_26C | 5'-CCGACGCATTTGTGCAACTTGTT-Aminolink-3' |
| A.ter_26A2 | 5'-CCGAACGCATTTATTTGCAACTTGTT-Aminolink-3' |
| A.ter_25B | 5'-CCGACGCATTTGTTTGCAACTTGTT-Aminolink-3' |
| A.ter_25A1 | 5'-CCGACGCATTTATTTGCAACTTGTT-Aminolink-3' |
| A.nig_24B | 5'-GCCGACGTTTTCCAACCATTCTTT-Aminolink-3' |
| A.nig_23A | 5'-GCCGACGTTATCCAACCATTTTT-Aminolink-3' |
| A.fum_24C | 5'-CAGCCGACACCCAACTTTATTTTT-Aminolink-3' |
| A.fum_24B | 5'-CCAGCCGACACCCAACTTTATTTT-Aminolink-3' |
| A.fum_24A | 5'-GCCAGCCGACACCCAACTTTATTT-Aminolink-3' |
| A.fla_25C | 5'-CCGAACGCAAATCAATCTTTTTCCA-Aminolink-3' |
| A.fla_25A | 5'-CTTGCCGAACGCAAATCAATCTTTT-Aminollink-3' |
| A.fla_24B | 5'-GCCGAACGCAAATCAATCTTTTTC-Aminolink-3' |

### Hybridisierung und Prozessierung des Array-Tube:

Das Array-Tube wurde 2-fach mit 200 µl entionisiertem Wasser gewaschen und anschließend für 15 min mit 2% Milchpulver in 6 x SSPE 0,005% Triton bei 30°C inkubiert. Die vereinigten PCR-Reaktionen wurden 5 min bei 95°C denaturiert. Das
Array-Tube wurde geleert, auf 40°C vorgewärmt und anschließend mit der PCR befüllt. Das Array-Tube wurde für eine Stunde bei 40°C und 550rpm in einem Thermoschüttler (Eppendorf, Hamburg, Deutschland) inkubiert. Anschließend wurde das Array-Tube jeweils für 5 min bei 20°C und 550 rpm mit folgenden Lösungen nacheinander gewaschen:
2 x SSC/0,01% Triton,
2 x SSC,
0,2 x SSC.

Das Array-Tube wurde anschließend für 30 min bei 30°C und 550 rpm mit einer 1:10000-Verdünnung Poly-HRP-Streptavidin (Pierce, Rockford, USA) in 6 x SSPE, 0,005% Triton inkubiert.

Danach erfolgten drei Schritte jeweils für 5 min bei 20°C und 550 rpm mit:
2 x SSC/0,01% Triton,
2 x SSC,
0,2 x SSC

### Detektion und Auswertung der Hybridisierungssignale:

Das Array-Tube wurde geleert und anschließend mit 100 µl True Blue TMB Peroxidase-Substrat (Medac, Wedel, Deutschland) gefüllt. Das Array-Tube wurde sofort in einem AT-Reader(Clondiag Chip Technologies, Jena, Deutschland) ausgelesen. Die Temperatur betrug dabei 25°C. Es wurde eine Bildreihe von 70 Bildern im Abstand von 10s aufgenommen.

Die Bildreihe wurde anschließend mit der Software Iconoclust (Clondiag Chip Technologies, Jena, Deutschland) ausgewertet. Dabei wurde mit automatischer Spoterkennung gearbeitet. Das Signal jedes Spots wurde gegen den lokalen Hintergrund korrigiert.

Signale wurden lediglich an den für *Aspergillus niger* spezifischen Sonden A.nig_24B und A.nig_23A sowie an der Sonde mit Array-Tube -Spezifität A_Pen_Gen_24 beobachtet. Die Signale der restlichen Sonden mit Spezifität für andere Array-Tube -Stämme lagen im Bereich des Hintergrundsignales.

### Ausführungsbeispiel 11:

Die identische Reaktion wie in Ausführungsbeispiel 10 wurde in einer geschlossenen Reaktionskammer, dem Clondiag Assay-Prozessor (AP) (Clondiag Chip Technologies GmbH, Jena Deutschland) durchgeführt.

Der Reaktionsansatz und das Array-Layout entsprachen dem vorangegangenen Ausführungsbeispiel.

### Prozessierung des Assay-Prozessors:

Der Assay-Prozessor wurde mittels der AP-Befüllstation (Clondiag Chip Technologies GmbH, Jena Deutschland) mit der PCR-Reaktionslösung befüllt. Anschließend erfolgte im AP eine PCR-Reaktion nach den gleichen Parametern wie im vorhergehenden Ausführungsbeispiel. Es folgte ein Denaturierungsschritt für 5 min bei 95°C und die anschließende Hybridisierung für 1 Stunde bei 40°C. Der Assay-Prozessor wurde zunächst mit 500 µl 0,2 x SSC und dann mit 500 µl einer Blockierungslösung (5% fetales Kälberserum in 6xSSPE, 0,005% Triton gespült (Flußrate jeweils 450 µl/Minute, Temperatur 20°C) und zum Ende der Spülung wurde der Assay-Prozessor für 10 min mit der Blockierunglösung inkubiert. Im Anschluß wurde die Blockierungslösung entfernt und der Assay-Prozessor mit 250 µl einer 1:100-Verdünnung eines HRP-Streptavidinkomplexes (Sigma, Deisenhofen, Deutschland) in 6 x SSPE, 0,005% Triton X-100 gespült (Flußrate 450 µl/min) und 30 min bei 20°C mit dieser Lösung inkubiert. Der Assay-Prozessor wurde mit 1 ml 2xSSC, 0,01%Triton und anschließend mit 500 µl 2x SSC gespült (Flußrate jeweils 450 µl/Minute, Temperatur 20°C). Anschließend wurde die Reaktionskammer geleert und mit 250 µl True Blue TMB Peroxidase-Substrat (Medac, Wedel, Deutschland) gespült und anschließend mit diesem Substrat inkubiert.

Die Detektion der Hybridisierungssignale (TMB-Fällung) erfolgte im Auflicht (Rotlicht-LED) mit einer CCD-Kamera. Es wurde eine Bildreihe von 70 Bildern im Abstand von 10 s aufgenommen.

Die Auswertung der Hybridisierungsergebnisse und die Ergebnisse entsprechen dem vorhergehenden Ausführungsbeispiel.

### Ausführungsbeispiel 12:

Durchführung einer asymmetrischen RT-PCR und Hybridisierung im Clondiag Assay-Prozessor in einem kontinuierlichen Prozeß

### Assay-Prozessor:

Es wurde ein Assay-Prozessor (Clondiag Chip Technologies) mit einem Array verwendet, das folgende Sonden in 4-facher Redundanz enthielt (Darstellung der Sonden in 5'-3'-Richtung, alle Sonden besaßen am 3'-Ende einen C7-Aminolink):

| | |
|---|---|
| Y_bla-47-24 | TTCGGACTCTTTGATGATTCATAA |
| Y_bla-45-23 | TCTTCGGACTCTTTGATGATTCA |
| SalR3b_22 | GGAAGGTGTTGTGGTTAATACC |
| SalR3a_22 | GGAAGGTGTTGTGGTTAATAAC |
| PA-39-20 | CAAATGTTGGGTGAACGGCT |
| PA-35-22 | AAAGCAAATGTTGGGTGAACGG |
| Ms2_2824 | TTCTCGATGGTCCATACCTTAGAT |
| Ms2_2724 | TAGATGCGTTAGCATTAATCAGGC |
| Ms2_2624 | TTAGATGCGTTAGCATTAATCAGG |
| Ms2_2525 | CTTAGATGCGTTAGCATTAATCAGG |
| Ms2_1423 | ATGGTCCATACCTTAGATGCGTT |
| GneR1_22 | AGGCCTTCGGGTTGTAAAGTAC |
| EcoR3_22 | GGAAGGGAGTAAAGTTAATACC |
| BsuR3b_22 | GAACAAGTGCCGTTCGAATAGG |
| BsuR3a_22 | GAACAAGTACCGTTCGAATAGG |
| BsuR1_22 | AGGTTTTCGGATCGTAAAGCTC |
| bla-3-20 | AACGATCGGAGGACCGAAGG |
| bla-1-21 | ACAACGATCGGAGGACCGAAG |
| BanR3_22 | GAACAAGTGCTAGTTGAATAAG |
| BanR1_22 | AGGCTTTCGGGTCGTAAAACTC |

### RT-PCR:

Es wurde eine RT-PCR mit folgender Zusammensetzung hergestellt:

| | |
|---|---|
| 10 µM Primer MS2_r_5'Cy3 (5`-Cyanine3_TCCGGTTGAGGGCTCTATCT-3') | 1,3 µl |
| 10 µM Primer MS2_f (5'-CAACTGGCGCGTACGTAAA-3') | 0,44 µl |
| 10 µM Kompetitor MS2_f_3'NH2 | |
| (5'-CAACTGGCGCGTACGTAAA-Aminolink-3') | 0,86 µl |
| 20 mM dNTP Mix (je 20 mM dATP, dCTP, dGTP,dTTP) | 0,65 µl |
| 100 mM DTT | 3.25 µl |
| 25 mM MgCl₂ | 6.5µl |
| PCR-Grade Wasser | 35 µl |
| 1 M Kaliumacetat | 3,9 µl |
| 5 x Titan one Tube PCR-Puffer (Roche, Mannheim, Deutschland) | 13 µl |
| Titan one Tube RT-PCR Enzyme Mix (Roche, Mannheim, Deutschland) | 1,3 µl |
| Template: MS2-RNA (10 ng/µl) | 1 µl |

Der Array-Prozessor wurde mittels einer Befüllstation mit dem RT-PCR-Reaktionsmix befüllt. Anschließend wurde eine RT-PCR-Reaktion im AP folgendermaßen durchgeführt:
*RT-Reaktion:*
10 min bei 50°C

*PCR-Amplifkation:*
1. Zyklus:
5 min 95°C
30 sec 60°C
40 sec 72°C

39 Zyklen:
30 sec 95°C
30 sec 60°C
40 sec 72°C

### Hybridisierung:

Das Reaktionsgemisch wurde im AP für 3 min bei 95°C denaturiert und anschließend für 1h bei 40°C hybridisiert. Es folgte ein Spülschritt mit 500 µl 2 x SSC bei 20°C und einer Flussgeschwindigkeit von 450 µl/min. Der Assay-Prozessor blieb nach Abschluß der Spülung mit 2 x SSC gefüllt.

### Auslesen der Hybridisierungssignale:

Die Detektion der Hybridisierungssignale erfolgte unter einem Zeiss Fluoreszenzmikroskop (Zeiss, Jena, Deutschland). Die Anregung erfolgte im Auflicht mit einer Weißlichtquelle und einem für Cyanine 3 geeigneten Filtersatz. Die Signale wurden mit einer CCD-Kamera (PCO-Sensicam, Kehlheim, Deutschland) aufgezeichnet. Die Belichtungszeit betrug 10 sec.

Die Auswertung der Hybridisierungssignale erfolgte mit der Software Iconoclust (Clondiag Chip Technologies GmbH, Jena, Deutschland). Hybridisierungssignale über dem experimentellen Hintergrund wurden an den MS2-spezifischen Sonden Ms2_2824, Ms2_2724, Ms2_2624, Ms2_2525, Ms2_1423 gemessen, während an den anderen Sonden kein Signal detektierbar war.

### Abbildungen:

Abb. 1: Menge an gebildetem PCR-Produkt gemäß Ausführungsbeispiel 1. Die Menge ist dabei als Light cycler units in Abhängigkeit von der Zyklenzahl angegeben.
Abb. 2: Analyse der PCR-Ansätze von Ausführungsbeispiel 1 auf einem 2% Agarosegel analysiert. Man erkennt, dass mit steigendem Kompetitor-Anteil die Menge an doppelsträngiger DNA ab- und an einzelsträngiger DNA zunimmt.
Abb. 3: Agarosegel, auf dem parallel 5 µl-Proben von Reaktionen gemäß Ausführungsbeispiel 2 mit einem Kompetitor-Anteil von 50% bzw. 87,5% analysiert wurden. Die Reaktionen waren jeweils nach der angegebenen Zyklenzahl gestoppt worden. Man erkennt, dass bei 87,5% Kompetitor-Anteil weniger Produkt erzeugt wird.
Abb. 4: Messung der Intensität der Hybridisierungssignale gemäß Ausführungsbeispiel 2. Die Intensität wurde gemessen, indem die Signalstärke (gemessene Graustufe) über den gesamten Bereich der Spots gemittelt wurde. Von diesem Wert wurde der über den Spot-freien Bereich (Hintergrund) gemittelte Grauwert abgezogen. Die so errechneten Signalintensitäten wurden normiert (höchster Wert = 100%) und gegen die Zyklenzahl aufgetragen.
Abb. 5: Ergebnisse gemäß Ausführungsbeispiel 3. Bei der Gelanalyse der symmetrischen Reaktionen wird erwartungsgemäß für alle Reaktionen, in denen eine Amplifikation erfolgte, nur ein dominierendes Produkt nachgewiesen. Dabei handelt es sich um das doppelsträngige PCR-Produkt. Im Gegensatz dazu werden bei allen detektierbaren asymmetrischen Reaktionen drei dominierende Produkte nachgewiesen. Neben der dem doppelsträngigen Template werden zwei weitere Banden erhalten, die verschiedenen Strukturen des markierten Einzelstranges entsprechen. Die Gelanalyse zeigt damit, dass die asymmetrische Amplifikation zu einem Einzelstrang-Überschuss führt.
Abb. 6: Array-Belegung gemäß Ausführungsbeispiel 4. Schwarze Felder entsprechen der Sonde C2938T, graue Felder entsprechen der Sonde C2938 WT.

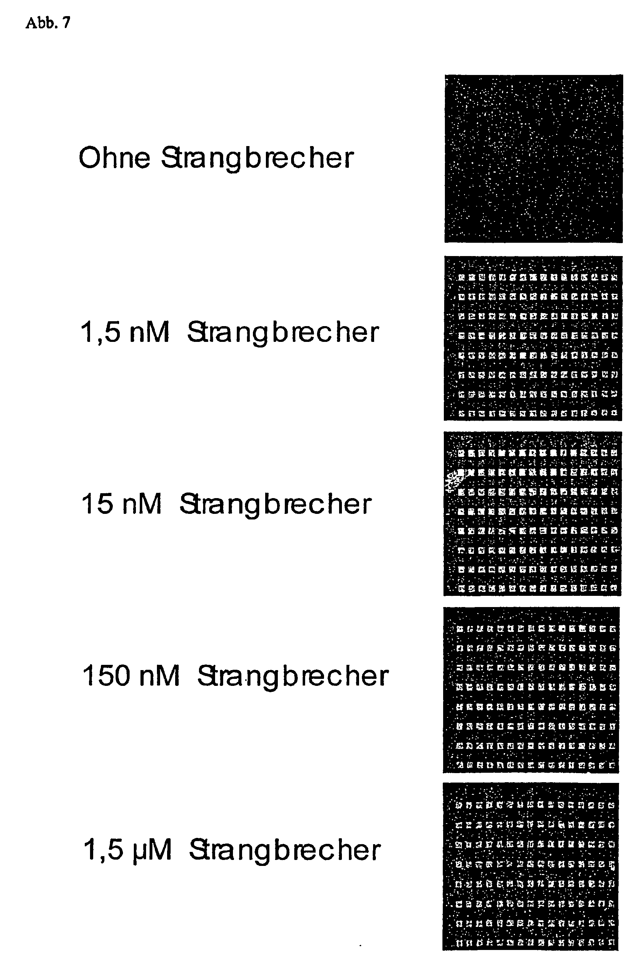

Abb. 7: Detektion der Hybridisierungssignale gemäß Ausführungsbeispiel 4. Die Hybridisierungsergebnisse zeigen folgendes Muster: Die Sonden für die Mutation C2938T zeigen starke Signale, während die Wildtyp-Sonden sowie die Deletionsvarianten deutlich geringere Signale zeigen. Dies entspricht den Erwartungen. Auffällig ist, dass die Signale durch Zugabe der Strukturbrecher weiter verstärkt werden. Damit ist eine Kombination von Kompetitor und Sekundärstrukturbrecher besonders nützlich, um gute Signalstärken zu erreichen.
Abb. 8: Detektion der Hybridisierungssignale gemäß Ausführungsbeispiel 5. Die Hybridisierungsergebnisse zeigen folgendes Muster: Die Sonden für die Mutation C2938T zeigen starke Signale, während die Wildtyp-Sonden sowie die Deletionsvarianten deutlich geringere Signale zeigen. Dies entspricht den Erwartungen. Auffällig ist, dass bereits bei Zugabe eines der beiden Strukturbrecher deutlich erhöhte Signalen erzielt werden.
Abb. 9: Anordnung der Sonden gemäß Ausführungsbeispiel 6. Jedes der 16 stark umrandeten Quadrate enthielt 16 Array-Elemente, die mit den für das Quadrat im unteren linken Bereich der Abbildung gezeigten Sonden bestückt waren.
Abb. 10. Detektion der Hybridisierungssignale gemäß Ausführungsbeispiel 6. Abb. 10a zeigt die Fluoreszenzsignale nach Hybridisierung einer PCR unter Zugabe von Strukturbrechern. Die Aufnahme erfolgte in dem Slidescanner mit Laser-Power 70 und Photomultiplier 80. Abb. 10b zeigt dagegen die Negativkontrolle ohne Zugabe der entsprechenden Strukturbrecher. Die Scannereinstellungen waren Laser-Power 100 und Photomultiplier 75. Zu beachten ist, dass Abb. 10b somit bei deutlich höherer Laser- und Photomultiplierleistung des Scanners aufgenommen wurde. Daher sind die Signale auf diesem Chip nur scheinbar stärker.
Abb. 11. Darstellung der normalisierten Messergebnisse aus der Hybridisierung einer PCR unter Zugabe und bei Fehlen von Strukturbrechern gemäß Ausführungsbeispiel 6. Aufgetragen ist zusätzlich das Verhältnis Hybridisierungssignal mit/ohne Strukturbrecher (+O/-O).
Abb. 12: Scanneraufnahmen der Hybridisierungsergebnisse gemäß Ausführungsbeispiel 7. Die hybridisierten und gewaschenen Chips wurden in einem Slidescanner (Scanarray4000, GSI Lumonics) ausgelesen. Beide Arrays wurden mit den identischen Scannereinstellungen (Laser-Power 70 und Photomultiplier 80) aufgenommen. Abb. 12a zeigt die Fluoreszenzsignale nach Hybridisierung einer PCR unter Zugabe von Strukturbrechern, Abb. 12b dagegen die Reaktion ohne Zugabe der entsprechenden Strukturbrecher.
Abb. 13: Zuordnung der einzelnen Sonden auf die Array-Elemente gemäß Ausführungsbeispiel 8. Mit der Match-Sonde belegte Array-Elemente sind weiß, mit der Deletions-Sonde belegte Sonden-Elemente sind schwarz und mit der Insertions-Sonde belegte Sondenelemente sind grau dargestellt.
Abb. 14: Hybridisierungssignale gemäß Ausführungsbeispiel 8 der nach 10 bzw. 30 Zyklen abgestoppten Reaktion.
Abb. 15: Aufbau des Arrays gemäß Ausführungsbeispiel 9. Jedes der 16 stark umrandeten Quadrate enthielt 16 Array-Elemente, die mit den für das Quadrat im unteren linken Bereich der Abbildung gezeigten Sonden bestückt waren.
Abb. 16a und 16b: Hybridisierungsergebnisse gemäß Ausführungsbeispiel 9.
Abb. 17: Auswertung der aufgenommenen Bilder gemäß Ausführungsbeispiel 9 mit der Bildauswertungs-Software Iconoclust^{®} (Clondiag Chip Technologies). Gezeigt ist die Hybridisierungsintensität der jeweiligen Sonden für die jeweiligen Reaktionen.

## Patentansprüche

1. Verfahren zur effizienten Amplifikation mindestens einer Template-Nukleinsäure durch eine PCR, wobei der PCR zu Anfang mindestens ein Kompetitor ausgewählt aus Nukleinsäuren und/oder Nukleinsäureanaloga in mindestens äquimolaren Mengen zu einem in der PCR verwendeten Primer zugesetzt wird, wobei der Kompetitor die Bildung eines der beiden durch die PCR amplifizierten Template-Stränge inhibiert.

2. Verfahren nach Anspruch 1,
**dadurch gekennzeichnet, dass** der Kompetitor ausgewählt wird aus DNA- und/oder RNA-Molekülen; PNA, LNA, TNA und/oder einem Nukleinsäureanalogon mit einer Phosphothioat-Bindung.

3. Verfahren nach Anspruch 2, wobei der Kompetitor enzymatisch nicht verlängerbar ist.

4. Verfahren nach Anspruch 3,
**dadurch gekennzeichnet, dass** das 3'-Ende der Kompetitor-Nukleinsäure und/oder des Kompetitor-Nukleinsäureanalogons enzymatisch nicht verlängerbar ist.

5. Verfahren nach Anspruch 4,
**dadurch gekennzeichnet, dass** das 3'-Ende der Kompetitor-Nukleinsäure und/oder des Kompetitor-Nukleinsäureanalogons keine freic 3'-OH-Gruppe aufweist.

6. Verfahren nach Anspruch 5,
**dadurch gekennzeichnet, dass** das 3'-Ende der Kompetitor-Nukleinsäure und/oder des Kompetitor-Nukleinsäureanalogons eine Amino-Gruppe, eine Phosphat-Gruppe, einen Biotin-Rest, ein Fluorophor oder ein H-Atom aufweist.

7. Verfahren nach einem der Ansprüche 4 bis 6,
**dadurch gekennzeichnet, dass** eine Kompetitor-Nukleinsäure und/oder ein Kompetitor-Nukleinsäureanalogon eingesetzt wird, die bzw. das an seinem 3'-Ende mindestens ein, zwei, drei, vier oder fünf Nukleotide aufweist, die nicht zu den entsprechenden Positionen in dem jeweiligen Template-Strang komplementär sind.

8. Verfahren nach einem der vorhergehenden Ansprüche,
**dadurch gekennzeichnet, dass** der Kompetitor an einen in der PCR verwendeten Primer bindet.

9. Verfahren nach Anspruch 8,
**dadurch gekennzeichnet, dass** der Kompetitor eine Nukleinsäure und/oder ein Nukleinsäureanalogon mit einer zumindest teilweise zu einem der in der PCR verwendeten Primer komplementären Sequenz ist.

10. Verfahren nach Anspruch 8 oder 9,
**dadurch gekennzeichnet, dass** der Kompetitor mit dem Primer kovalent verknüpft wird.

11. Verfahren nach Anspruch 10,
**dadurch gekennzeichnet, dass** die kovalente Verknüpfung zwischen Kompetitor und Primer nicht als Template-Strang für Polymerasen dienen kann.

12. Verfahren nach einem der Ansprüche 8 bis 11,
**dadurch gekennzeichnet, dass** die Stabilität des Komplexes von Primer mit Kompetitor geringer ist als die Stabilität des Komplexes des Primers mit seiner spezifischen Primer-Bindungsstelle auf dem Template-Strang.

13. Verfahren nach Anspruch 12,
**dadurch gekennzeichnet, dass** die Amplifikation bezüglich des Temperaturregimes als zweiphasiger Prozess durchgeführt wird, wobei in einer ersten Phase eine Annealing-Temperatur gewählt wird, bei der der Primer an seine spezifische Primer-Bindungsstelle auf dem Template-Strang, nicht aber an den Kompetitor bindet, und in einer zweiten Phase eine Annealing-Temperatur gewählt wird, bei der auch eine Bindung zwischen Primer und Kompetitor erfolgt.

14. Verfahren nach einem der Ansprüche 1 bis 7,
**dadurch gekennzeichnet, dass** der Kompetitor einen der Template-Stränge bindet.

15. Verfahren nach Anspruch 14,
**dadurch gekennzeichnet, dass** der Kompetitor mit einem der zur PCR verwendeten Primer um die Bindung an einen der Template-Stränge konkurriert.

16. Verfahren nach Anspruch 15,
**dadurch gekennzeichnet, dass** die Stabilität des Komplexes des Kompetitors mit der spezifischen Primer-Bindungsstelle des Primers auf dem Template geringer ist als die Stabilität des Komplexes des Primers mit seiner spezifischen Primer-Bindungsstelle auf dem Template-Strang.

17. Verfahren nach Anspruch 16,
**dadurch gekennzeichnet, dass** die Amplifikation bezüglich des Temperaturregimes als zweiphasiger Prozess durchgeführt wird, wobei in einer ersten Phase eine Annealing-Temperatur gewählt wird, bei der der Primer, nicht aber der Kompetitor an die spezifische Bindungsstelle des Primers auf dem Template-Strang bindet, und in einer zweiten Phase eine Annealing-Temperatur gewählt wird, bei der auch eine Bindung des Kompetitors an die spezifische Bindungsstelle des Primers auf dem Template erfolgt.

18. Verfahren nach einem der vorhergehenden Ansprüche,
**dadurch gekennzeichnet, dass** der Kompetitor gleichzeitig als Sekundärstrulcturbrecher wirkt.

19. Verfahren nach einem der vorhergehenden Ansprüche,
**dadurch gekennzeichnet, dass** mehrere Kompetitoren eingesetzt werden und mindestens einer der Kompetitoren ausschließlich als Sekundärstrukturbrecher wirkt.

20. Verfahren nach Anspruch 19,
**dadurch gekennzeichnet, dass** der Komplex des ausschließlich als Sekundärstrukturbrecher wirkenden Kompetitors mit dem Template-Strang weniger stabil ist als der Komplex mindestens eines inhibierend wirkenden Kompetitors mit dem Template-Strang.

21. Verfahren nach Anspruch 20,
**dadurch gekennzeichnet, dass** die Amplifikation bezüglich des Temperaturregimes als zweiphasiger Prozess durchgeführt wird, wobei in einer ersten Phase eine Annealing-Temperatur gewählt wird, bei der der inhibierend wirkende Kompetitor, nicht aber der ausschließlich als Sekundärstrukturbrecher wirkende Kompetitor an einen Template-Strang bindet, und in einer zweiten Phase eine Temperatur gewählt wird, bei der auch der
ausschließlich als Sekundärstrukturbrecher wirkende Kompetitor an den Template-Strang bindet.

22. Verfahren nach einem der vorhergehenden Ansprüche,
**dadurch gekennzeichnet, dass** der Kompetitor und/oder Strukturbrecher eine Nukleinsäure und/oder ein Nukleinsäureanalogon ist, das mindestens 10 Nukleotide umfasst.

23. Verfahren nach Anspruch 22,
**dadurch gekennzeichnet, dass** der Kompetitor und/oder Strukturbrecher eine Nukleinsäure und/oder ein Nukleinsäureanalogon ist, die bzw. das mindestens 15 Nukleotide umfasst.

24. Verfahren nach Anspruch 23,
**dadurch gekennzeichnet, dass** der Kompetitor und/oder Strukturbrecher eine Nukleinsäure und/oder ein Nukleinsäureanalogon ist, die bzw. das mindestens 17 Nukleotide umfasst.

25. Verfahren nach Anspruch 24,
**dadurch gekennzeichnet, dass** der Kompetitor und/oder Strukturbrecher eine Nukleinsäure und/oder ein Nukleinsäureanalogon ist, die bzw. das mindestens 18 Nukleotide umfasst.

26. Verfahren nach einem der vorhergehenden Ansprüche,
**dadurch gekennzeichnet, dass** das Verhältnis zwischen Primer und Kompetitor und/oder Strukturbrecher bezogen auf die molaren Mengen zu Beginn der Reaktion zwischen 0,01 und 0,99 liegt.

27. Verfahren nach Anspruch 26,
**dadurch gekennzeichnet, dass** das Verhältnis zwischen Primer und Kompetitor und/oder Strukturbrecher bezogen auf die molaren Mengen zu Beginn der Reaktion zwischen 0,05 und 0,8 liegt.

28. Verfahren nach Anspruch 27,
**dadurch gekennzeichnet, dass** das Verhältnis zwischen Primer und Kompetitor und/oder Strukturbrecher bezogen auf die molaren Mengen zu Beginn der Reaktion zwischen 0,1 und 0,5 liegt.

29. Verfahren zum Nachweis von mindestens einer Nukleinsäure,
**dadurch gekennzeichnet, dass** die mindestens eine Nukleinsäure durch ein Verfahren nach einem der Ansprüche 1 bis 28 amplifiziert und anschließend durch Hybridisierung mit einer komplementären Sonde nachgewiesen wird.

30. Verfahren nach Anspruch 29,
**dadurch gekennzeichnet, dass** die Amplifikation der Nukleinsäuren und deren Nachweis durch Hybridisierung gegen eine Sonde in einem kontinuierlichen Prozess erfolgen.

31. Verfahren nach Anspruch 29 oder 30,
**dadurch gekennzeichnet, dass** der Kompetitor den zur Sonde komplementären Template-Strang bindet.

32. Verfahren nach Anspruch 31,
**dadurch gekennzeichnet, dass** der Kompetitor den zur Sonde komplementären Template-Strang in einem Bereich bindet, der nicht durch die Sonde adressiert wird.

33. Verfahren nach Anspruch 32,
**dadurch gekennzeichnet, dass** der Kompetitor in unmittelbarer Nachbarschaft des Sequeazbereiches, der durch die Sonde adressiert wird, bindet.

34. Verfahren nach Anspruch 33,
**dadurch gekennzeichnet, dass** der Kompetitor ein DNA-Oligonukleotid ist, das mit dem Template-Strang, der durch die Hybridisierung nachgewiesen wird, in unmittelbarer Nachbarschaft des Sequenzbereichs, der durch die Sonde adressiert wird, hybridisiert.

35. Verfahren nach Anspruch 32,
**dadurch gekennzeichnet, dass** der Kompetitor in der Nähe des Sequenzbereiches, der durch die Sonde adressiert wird, bindet.

36. Verfahren nach Anspruch 35,
**dadurch gekennzeichnet, dass** der Kompetitor ein DNA-Oligonukleotid ist, das mit dem Template-Strang, der durch die Hybridisierung nachgewiesen wird, in der Nähe des Sequenzbereichs, der durch die Sonde adressiert wird, hybridisiert.

37. Verfahren nach einem der Ansprüche 29 bis 36,
**dadurch gekennzeichnet, dass** der PCR zu Beginn ein Kompetitor, der mit einem der PCR-Primer um die Bindung an das Template konkurriert, und/oder mindestens ein Kompetitor, der in Nähe oder unmittelbarer Nachbarschaft der Sequenz, die durch die Sonde nachgewiesen wird, an das Target bindet und somit als Sekundärstrukturbrecher wirkt, zugesetzt wird.

38. Verfahren nach einem der Ansprüche 29 bis 37,
**dadurch gekennzeichnet, dass** mindestens einer der ausschließlich als Sekundärstrukturbrecher wirkenden Kompetitoren dem Reaktionsgemisch erst nach Abschluss der PCR zugegeben wird.

39. Verfahren nach einem der Ansprüche 29 bis 38,
**dadurch gekennzeichnet, dass** eine Kompetitor-Nukleinsäure eingesetzt wird, die über einen Sequenzbereich verfügt, der zu einem Bereich des Template-Strangs, der nicht durch die Hybridisierung mit der Sonde adressiert wird, komplementär ist, und an seinem 3'-Ende mindestens ein, zwei, drei, vier oder fünf Nukleotide aufweist, die nicht zu den entsprechenden Positionen in dem jeweiligen Template-Strang komplementär sind.

40. Verfahren nach einem der Ansprüche 29 bis 39,
**dadurch gekennzeichnet, dass** PCR und Hybridisierung im gleichen Puffersystem durchgeführt werden können.

41. Verfahren nach einem der vorhergehenden Ansprüche,
**dadurch gekennzeichnet, dass** PCR und/oder Hybridisierung in einer geschlossenen Reaktionskammer durchgeführt werden.

42. Verwendung der Verfahren nach einem der Ansprüche 1 bis 41 für Microarray-basierte Nachweisverfahren.

43. Verwendung der Verfahren nach einem der Ansprüche 1 bis 41 für die EchtZeit-Quantifizierung von PCR-Reaktionen.

## Claims

1. Method for efficient amplification of at least one template nucleic acid by a PCR, wherein at least one competitor selected from nucleic acids and/or nucleic acid analogues is added to the PCR at the beginning in at least equimolar amounts to a primer used in the PCR, wherein the competitor inhibits the formation of one of the two template strands amplified by the PCR.

2. Method according to claim 1,
**characterized in that** the competitor is selected from DNA and/or RNA molecules; PNA, LNA, TNA and/or a nucleic acid analog having a phosphothioate bond.

3. Method according to claim 2, wherein the competitor is enzymatically not extendable.

4. Method according to claim 3,
**characterized in that** the 3'-end of the competitor nucleic acid and/or of the competitor nucleic acid analog is enzymatically not extendable.

5. Method according to claim 4,
**characterized in that** the 3'-end of the competitor nucleic acid and/or of the competitor nucleic acid analog has no free 3' OH-group.

6. Method according to claim 5,
**characterized in that** the 3'-end of the competitor nucleic acid and/or of the competitor nucleic acid analog has an amino group, a phosphate group, a biotin residue, fluorophore or a H-atom.

7. Method according any of claims 4 to 6,
**characterized in that** a competitor nucleic acid and/or a competitor nucleic acid analog is used having at its 3'-end at least one, two, three, four or five nucleotides which are not complementary to the corresponding positions in the respective template strand.

8. Method according to any of the preceding claims,
**characterized in that** the competitor binds to a primer used in the PCR.

9. Method according to claim 8,
**characterized in that** the competitor is a nucleic acid and/or a nucleic acid analog having a sequence complementary at least in part to one of the primers used in the PCR.

10. Method according to claim 8 or 9,
**characterized in that** the competitor is covalently linked to the primer.

11. Method according to claim 10,
**characterized in that** the covalent link between the competitor and the primer cannot serve as template strand for polymerases.

12. Method according to any of claims 8 to 11,
**characterized in that** the stability of the complex of the primer with the competitor is lower than the stability of the complex of the primer with its specific primer binding site on the template strand.

13. Method according to claim 12,
**characterized in that** the amplification is performed as a two-phase process with regard to the temperature regime, wherein in a first phase an annealing temperature is selected at which the primer binds to its specific primer binding site on the template strand but not to the competitor, and wherein in a second phase an annealing temperature is selected at which binding between primer and competitor also occurs.

14. Method according to any of claims 1 to 7,
**characterized in that** the competitor binds one of the template strands.

15. Method according to claim 14,
**characterized in that** the competitor competes against one of the primers used for the PCR for binding to one of the template strands.

16. Method according to claim 15,
**characterized in that** the stability of the complex of the competitor with the specific primer binding site of the primer on the template is lower than the stability of the complex of the primer with its specific primer binding site on the template strand.

17. Method according to claim 16,
**characterized in that** the amplification is performed as a two-phase process with regard to the temperature regime, wherein in a first phase an annealing temperature is selected at which the primer, but not the competitor, binds to the specific binding site of the primer on the template strand and wherein in a second phase an annealing temperature is selected at which binding of the competitor to the specific binding site of the primer on the template also occurs.

18. Method according to any of the preceding claims,
**characterized in that** the competitor simultaneously acts as a secondary structure breaker.

19. Method according to any of the preceding claims,
**characterized in that** several competitors are used and at least one of the competitors exclusively acts as a secondary structure breaker.

20. Method according to claim 19,
**characterized in that** the complex of the competitor acting exclusively as secondary structure breaker and the template strand is less stable than the complex of at least one competitor acting inhibitorily with the template strand.

21. Method according to claim 20,
**characterized in that** amplification is performed as a two-phase process with regard to the temperature regime, wherein in a first phase an annealing temperature is selected at which the competitor acting inhibitorily, but not the competitor acting exclusively as secondary structure breaker, binds to a template strand, and wherein in a second phase a temperature is selected at which also the competitor acting exclusively as secondary structure breaker binds to the template strand.

22. Method according to any of the preceding claims,
**characterized in that** the competitor and/or the structure breaker is a nucleic acid and/or a nucleic acid analog which comprises at least 10 nucleotides.

23. Method according to claim 22,
**characterized in that** the competitor and/or the structure breaker is a nucleic acid and/or a nucleic acid analog which comprises at least 15 nucleotides.

24. Method according to claim 23,
**characterized in that** the competitor and/or the structure breaker is a nucleic acid and/or a nucleic acid analog which comprises at least 17 nucleotides.

25. Method according to claim 24,
**characterized in that** the competitor and/or the structure breaker is a nucleic acid and/or a nucleic acid analog which comprises at least 18 nucleotides.

26. Method according to any of the preceding claims,
**characterized in that** the ratio between primer and competitor and/or structure breaker based on the molar amounts at the beginning of the reaction lies between 0.01 and 0.99.

27. Method according to claim 26,
**characterized in that** the ratio between primer and competitor and/or structure breaker based on the molar amounts at the beginning of the reaction lies between 0.05 and 0.8.

28. Method according to claim 27,
**characterized in that** the ratio between primer and competitor and/or structure breaker based on the molar amounts at the beginning of the reaction lies between 0.1 and 0.5.

29. Method for detection of at least one nucleic acid,
**characterized in that** the at least one nucleic acid is amplified a the method according to any of claims 1 to 28 and subsequently detected by hybridization with a complementary probe.

30. Method according to claim 29,
**characterized in that** the amplification of the nucleic acids and their detection by hybridization to a probe occurs in a continuous process.

31. Method according to claim 29 or 30,
**characterized in that** the competitor binds the template strand complementary to the probe.

32. Method according to claim 31,
**characterized in that** the competitor binds the template strand complementary to the probe in a region which is not addressed by the probe.

33. Method according to claim 32,
**characterized in that** the competitor binds in immediate proximity of the sequence region which is addressed by the probe.

34. Method according to claim 33,
**characterized in that** the competitor is a DNA oligonucleotide which hybridizes with the template strand detected by the hybridization in immediate proximity of the sequence region which is addressed by the probe.

35. Method according to claim 32,
**characterized in that** the competitor binds near the sequence region which is addressed by the probe.

36. Method according to claim 35,
**characterized in that** the competitor is a DNA oligonucleotide that hybridizes with the template strand detected by the hybridization near the sequence region which is addressed by the probe.

37. Method according to any of claims 29 to 36,
**characterized in that** a competitor which competes with the PCR primer for binding to the template, and/or at least one competitor which binds to the target in proximity or immediate proximity of the sequence detected by the probe, thus acting as a secondary structure breaker, is added to the PCR at the beginning.

38. Method according to any of claims 29 to 37,
**characterized in that** at least one of the competitors acting exclusively as secondary structure breaker is added to the reaction mixture only after completion of the PCR.

39. Method according to any of claims 29 to 38,
**characterized in that** a competitor-nucleic acid is used which has a sequence region complementary to a region of the template strand which is not addressed by the hybridization with the probe, and which has at its 3'-end at least one, two, three, four or five nucleotides which is/are not complementary to the corresponding positions in the respective template strand.

40. Method according to any of claims 29 to 39,
**characterized in that** the PCR and hybridization may be performed in the same buffer system.

41. Method according to any of the preceding claims,
**characterized in that** the PCR and/or hybridization are performed in a closed reaction chamber.

42. Use of the methods according to any of claims 1 to 41 for microarray-based detection methods.

43. Use of the methods according to any of claims 1 to 41 for the real-time quantification of PCR reactions.

## Revendications

1. Procédé pour l'amplification effective d'au moins un acide nucléique de template par une technique PCR, dans lequel, au début, au moins un compétiteur sélectionné parmi les acides nucléiques et/ou les analogues d'acide nucléique, est ajouté à la PCR en quantités au moins équimolaires en relation à une amorce utilisée dans la PCR, dans laquelle le compétiteur inhibe la formation de l'un des deux brins de template amplifiés par la PCR.

2. Procédé selon la revendication 1
**caractérisé par le fait que** le compétiteur est sélectionné parmi les molécules d'ADN et/ou ARN, APN, ALN, ATN et/ou parmi un analogue d'acide nucléique ayant une liaison phosophorotioate.

3. Procédé selon la revendication 2, dans lequel le compétiteur n'est pas prolongeable de façon enzymatique.

4. Procédé selon la revendication 3,
**caractérisé par le fait que** l'extrémité 3' de l'acide nucléique compétiteur et/ou de l'analogue de l'acide nucléique compétiteur n'est pas prolongeable de façon enzymatique.

5. Procédé selon la revendication 4,
**caractérisé par le fait que** l'extrémité 3' de l'acide nucléique compétiteur et/ou de l'analogue de l'acide nucléique compétiteur ne comprend aucun groupe 3'-OH libre.

6. Procédé selon la revendication 5,
**caractérisé par le fait que** l'extrémité 3' de l'acide nucléique compétiteur et/ou de l'analogue de l'acide nucléique compétiteur comprend un groupe amino, un groupe phosphate, un résidu de biotine, un fluorophore ou un atome H.

7. Procédé selon l'une des revendications 4 à 6,
**caractérisé par le fait qu**'un acide nucléique compétiteur et/ou un analogue de l'acide nucléique compétiteur est mis en oeuvre, ayant, à son extrémité 3', au moins un, deux, trois, quatre ou cinq nucléotides qui ne sont pas complémentaires aux positions correspondantes dans le brin de template respective.

8. Procédé selon l'une des revendications précédentes,
**caractérisé par le fait que** le compétiteur s'attache à une amorce utilisée dans la technique de PCR.

9. Procédé selon la revendication 8,
**caractérisé par le fait que** le compétiteur est un acide nucléique et/ou un analogue d'acide nucléique ayant une séquence qui est au moins partiellement complémentaire à l'une des amorces utilisées dans la technique de PCR.

10. Procédé selon la revendication 8 ou 9,
**caractérisé par le fait que** le compétiteur est lié à l'amorce de façon covalente.

11. Procédé selon la revendication 10,
**caractérisé par le fait que** la liaison covalente entre le compétiteur et l'amorce ne peut pas servir comme brin de template pour les polymérases.

12. Procédé selon l'une des revendications 8 à 11,
**caractérisé par le fait que** la stabilité du complexe amorce-compétiteur est plus faible que la stabilité du complexe de l'amorce avec son endroit de liaison d'amorce spécifique sur le brin de template.

13. Procédé selon la revendication 12,
**caractérisé par le fait que** l'amplification, relatif au régime de température, est réalisée comme processus en deux phases dans lequel, dans une première phase, une température de détrempage est sélectionnée, à laquelle l'amorce s'attache à son endroit de liaison d'amorce spécifique sur le brin de template mais ne s'attache pas au compétiteur, et, dans une deuxième phase, une température de détrempage est sélectionnée, à laquelle une liaison entre l'amorce et le compétiteur aura également lieu.

14. Procédé selon l'une des revendications 1 à 7,
**caractérisé par le fait que** le compétiteur s'attache à l'un des brins de template.

15. Procédé selon la revendication 14,
**caractérisé par le fait que** le compétiteur et l'une des amorces utilisées pour la technique de PCR font concurrence pour la liaison à l'un des brins de template.

16. Procédé selon la revendication 15,
**caractérisé par le fait que** la stabilité du complexe du compétiteur avec l'endroit de liaison d'amorce spécifique de l'amorce sur le template est plus faible que la stabilité du complexe de l'amorce avec son endroit de liaison d'amorce spécifique sur le brin de template.

17. Procédé selon la revendication 16,
**caractérisé par le fait que** l'amplification, relatif au régime de température, est réalisée comme processus en deux phases dans lequel, dans une première phase, une température de détrempage est sélectionnée, à laquelle l'amorce, mais pas le compétiteur, s'attache à son endroit de liaison spécifique de l'amorce sur le brin de template, et, dans une deuxième phase, une température de détrempage est sélectionnée, à laquelle une liaison du compétiteur à l'endroit de liaison spécifique de l'amorce sur le template aura également lieu.

18. Procédé selon l'une des revendications précédentes,
**caractérisé par le fait que**, en même temps, le compétiteur agit comme casseur de structure secondaire.

19. Procédé selon l'une des revendications précédentes,
**caractérisé par le fait que** plusieurs compétiteurs sont mis en oeuvre et au moins l'un des compétiteurs agit exclusivement comme casseur de structure secondaire.

20. Procédé selon la revendication 19,
**caractérisé par le fait que** le complexe du compétiteur agissant exclusivement comme casseur de structure secondaire, avec le brin de template est moins stable que le complexe d'au moins un compétiteur ayant un effet d'inhibition, avec le brin de template.

21. Procédé selon la revendication 20,
**caractérisé par le fait que** l'amplification, relatif au régime de température, est réalisée comme processus en deux phases dans lequel, dans une première phase, une température de détrempage est sélectionnée, à laquelle le compétiteur ayant un effet d'inhibition, mais pas le compétiteur agissant exclusivement comme casseur de structure secondaire, s'attache à un brin de template, et, dans une deuxième phase, une température est sélectionnée, à laquelle le compétiteur agissant exclusivement comme casseur de structure secondaire s'attache également au brin de template.

22. Procédé selon l'une des revendications précédentes,
**caractérisé par le fait que** le compétiteur et/ou le casseur de structure est un acide nucléique ou un analogue d'acide nucléique comprenant au moins 10 nucléotides.

23. Procédé selon la revendication 22,
**caractérisé par le fait que** le compétiteur et/ou le casseur de structure est un acide nucléique ou un analogue d'acide nucléique comprenant au moins 15 nucléotides.

24. Procédé selon la revendication 23,
**caractérisé par le fait que** le compétiteur et/ou le casseur de structure est un acide nucléique ou un analogue d'acide nucléique comprenant au moins 17 nucléotides.

25. Procédé selon la revendication 24,
**caractérisé par le fait que** le compétiteur et/ou le casseur de structure est un acide nucléique ou un analogue d'acide nucléique comprenant au moins 18 nucléotides.

26. Procédé selon l'une des revendications précédentes,
**caractérisé par le fait que** le rapport entre l'amorce et le compétiteur et/ou le casseur de structure, relatif aux quantités molaires au début de la réaction, est entre 0,01 et 0,99.

27. Procédé selon la revendication 26,
**caractérisé par le fait que** le rapport entre l'amorce et le compétiteur et/ou le casseur de structure, relatif aux quantités molaires au début de la réaction, est entre 0,05 et 0,8.

28. Procédé selon la revendication 27,
**caractérisé par le fait que** le rapport entre l'amorce et le compétiteur et/ou le casseur de structure, relatif aux quantités molaires au début de la réaction, est entre 0,1 et 0,5.

29. Procédé pour le décèlement d'au moins un acide nucléique,
**caractérisé par le fait que** le au moins un acide nucléique est amplifié par un procédé selon l'une des revendications 1 à 28 et ensuite est décelé par hybridation avec une sonde complémentaire.

30. Procédé selon la revendication 29,
**caractérisé par le fait que** l'amplification des acides nucléiques et le décèlement de ceux-ci sont réalisés par hybridation contre une sonde par un processus continu.

31. Procédé selon la revendication 29 ou 30,
**caractérisé par le fait que** le compétiteur établit une liaison avec le brin de template complémentaire à la sonde.

32. Procédé selon la revendication 31,
**caractérisé par le fait que** le compétiteur établit une liaison avec le brin de template complémentaire à la sonde dans un domaine qui n'est pas adressé par la sonde.

33. Procédé selon la revendication 32,
**caractérisé par le fait que** le compétiteur s'attache au voisinage immédiat du domaine de séquence qui est adressé par la sonde.

34. Procédé selon la revendication 33,
**caractérisé par le fait que** le compétiteur est un oligonucléotide d'ADN qui hybride avec le brin de template, qui est décelé par l'hybridation, au voisinage immédiat du domaine de séquence qui est adressé par la sonde.

35. Procédé selon la revendication 32,
**caractérisé par le fait que** le compétiteur s'attache au voisinage du domaine de séquence qui est adressé par la sonde.

36. Procédé selon la revendication 35,
**caractérisé par le fait que** le compétiteur est un oligonucléotide d'ADN qui hybride avec le brin de template, qui est décelé par l'hybridation, au voisinage du domaine de séquence qui est adressé par la sonde.

37. Procédé selon une des revendications 29 à 36,
**caractérisé par le fait que**, au début, un compétiteur est additionné qui fait concurrence avec l'une des amorces de PCR pour la liaison au template, et/ou au moins un compétiteur qui est décelé à proximité ou au voisinage immédiat de la séquence décelée par la sonde, établie une liaison à la cible et ainsi agit comme casseur de structure.

38. Procédé selon une des revendications 29 à 37,
**caractérisé par le fait qu**'au moins l'un des compétiteurs agissant exclusivement comme des casseurs de structure n'est additionné au mélange de réaction qu'après l'achèvement de la PCR.

39. Procédé selon une des revendications 29 à 38,
**caractérisé par le fait qu**'un acide nucléique compétiteur est mis en oeuvre ayant un domaine de séquence qui est complémentaire à un domaine du brin de template qui n'est pas adressé par l'hybridation avec la sonde, et qui comprend, à son extrémité 3', au moins un, deux, trois, quatre ou cinq nucléotides qui ne sont pas complémentaires aux positions correspondantes dans le brin de template respectif.

40. Procédé selon l'une des revendications 29 à 39,
**caractérisé par le fait que** la PCR et l'hybridation sont réalisées dans le même système de tampon.

41. Procédé selon l'une des revendications précédentes,
**caractérisé par le fait que** la PCR et/ou l'hybridation sont réalisées dans une chambre de réaction fermée.

42. Utilisation des procédés selon l'une des revendications 29 à 41 pour des procédés de décèlement à base de biopuce.

43. Utilisation des procédés selon l'une des revendications 1 à 41 pour la quantification en temps réel des réactions de PCR.
